# EUROPEAN PATENT SPECIFICATION

(11) **EP 1 341 546 B1**
(45) Date of publication and mention of the grant of the patent: **21.09.2011**
(21) Application number: 01986266.3
(22) Date of filing: 05.10.2001
(51) Int. Cl.: A61K 35/74, A61K 31/715, A61K 39/39, A61P 37/00

(54) **KYBERDRUG AS AUTOVACCINES WITH IMMUNE-REGULATING EFFECTS**
KYBERDRUG ALS AUTOVAKZINE MIT IMMUNREGULIERENDEN WIRKUNGEN
KYBERMEDICAMENT UTILISE COMME AUTOVACCINS AVEC EFFETS D'IMMUNOREGULATION

(30) Priority: 06.10.2000 US 238656 P; 23.01.2001 US 263494 P
(43) Date of publication of application: 10.09.2003
(73) Proprietor: The Symbio Herborn Group GmbH u.Co, 35745 Herborn-Hörbach (DE)
(72) Inventor: Paradies, Henrich H., 58636 Iserlohn (DE); Rusch, Volker, 35745 Herborn (DE); Zimmermann, Kurt, 35745 Herborn-Seelbach (DE)
(74) Representative: Reinhard - Skuhra - Weise & Partner GbR
(86) International application number: PCT/IB2001/002284
(87) International publication number: WO 2002/028424

(56) References cited:
- WO-A-00/26384
- WO-A-97/19688
- WO-A-97/25061
- US-A- 4 912 094

## Description

### FIELD OF THE INVENTION

The present invention relates to a biologically active material isolated from non-pathogenic bacteria, pharmaceutical compositions containing same and methods for treating viral and bacterial infections and maladies caused by viruses, including retroviruses and bacteria.

### BACKGROUND OF THE INVENTION

The gastrointestinal tract of humans and animals contains a large number of pathogenic bacteria that are prevented from reaching the systemic circulation by the presence of an effective mucosal barrier. In healthy humans and animals, toxins and a small number of microorganisms continuously break the epithelial lining of the gut; nevertheless, further migration of the bacteria is prevented by the action of gut-associated lymphoid tissues or by certain specific cells like leukocytes and their subgroups or, if present, impaired lymphocytes. However, many host responses to traumas, burns, chemotherapy, inflammatory diseases and secondary infections have been shown to cause an increase in the permeability of the intestinal tissue and the bowel to microorganisms and viruses as well as toxins. Such changes in intestinal permeability can originate from translocation of bacteria, i.e., the process by which the endogenous gut flora penetrate the intestinal barrier and invade sterile tissue. As a result, T-cell immunity is impaired, and endotoxins are formed and released, causing damage to the intestinal lumen and more importantly to the intestinal barrier. This action allows the intestinal bacteria and endotoxins to invade more deeply into the host, thereby amplifying the host's response to the infection and causing a prolonged or life-threatening course of the disease. The enhancement of intestinal permeability due to the release of bacterial endotoxins has deleterious effects not only on T-cell lymphocytes and enterocytes, but more importantly on the cell-cell mediators, e.g. cytokines, including growth factors and interferons. Moreover, the enhancement of intestinal permeability impairs the immune system of the patient, creates inflammation in the patient and causes tissue remodeling.

A type of bacteria endogenous to the gut which is translocated and causes the deleterious effects described hereinabove is the enterobacteriaceae. These are a family of gram negative, facultatively anaerobic bacteria that are widespread as parasites and pathogens of animals, including humans and plants. Examples include the intestinal bacteria E. coli and Proteus vulgaris. Another bacteria that causes these deleterious effects is the Salmonella. The enterobacteriaceae are found in feces, pus, sputum, urine, and skin of mammals as well as in infected areas in the mammals, especially those caused by bacteria and viruses, including those of inflammatory diseases.

Bacterial translocation includes the migration of microbial organisms to various tissues, such as lymph nodes, spleen, liver, blood and the lungs. The translation of these bacteria may have deleterious effects. For example, the presence of endotoxins within the distal airspace of the lung induces an inflammatory response that results in the accumulation of neutrophils and the formation of edema within 24 hours. The response is attributed to the effect of endotoxin on epithelial cells, alveolar macrophages, and endothelial cells inducing the production of cytokines. These cytokines subsequently induce upregulation of adhesion molecules and acute migration of neutrophils and, at later times, mononuclear cells.

It is believed that the deleterious effects are attributable to an endotoxin produced by the enterobacteriaceae and injected into the cell. This endotoxin is or contains lipid A. Gram-negative bacteria contain lipid A. Lipid A is the major element in the lipopolysaccharide molecules that coat the surface of all Gram-negative bacteria. The general structure of the lipopolysaccharide is as follows:
(oligosaccharide repeating unit)ₙ-core oligosaccharide-(ketodeoxyoctanate)₃-lipid A.
Lipid A is a diphosphorooligosaccharide which contains a glucosamine backbone to which generally are linked long chain fatty acids. More specifically, it consists of a backbone of (β, 1-6) linked D-glucosamine dissaccharide which carries phosphate residues in positions 1 and 4' on the sugar ring. Amidated or esterified long chain fatty acids (generally D-3-hydroxy and/or acyloxy fatty acids) are present in each of the possible sites in the glucosamine moieties.

The lipid A moiety is the portion of the endotoxin which is responsible for the deleterious and lethal effects. Moreover, the chemical structure of lipid A is the most constant among the different genera of gram-negative bacteria.

The Lipopolysaccharides are anchored to the outer surface of the outer membrane of the enterobacteriaceae via covalently linked lipid A, and they are released into the tissue when the enterobacteriaceae invade the host.

The unwanted toxic effects are associated with the pathophysiological activities of free lipid A, e.g. the induction of endotoxic shock, pyrogenicity, macrophage activation, β-lymphocyte mitogenicity, induction of unspecific interferon production, complement activation and human tumor regression (see e.g., C. Galanos et al., Int. Rev. Biochem. 14, 280-288, (1977); C. Galanos et al., Eur. J. Biochem. 31, 230-233, (1972); C. Galanos, et al. Eur. J.Biochem., 9, 245 - 249, (1969); Weinberg et al., J.Immunol., 121, 72-80, (1978); E.E. Ribi et al., J.Natl Cancer Inst., 55, 1253-1257 (1982)).

However, the endotoxins also contain a portion which has beneficial effects to the animal, i.e., the oligosaccharide repeating unit (O-chain). It plays a role in protecting the cell from cell death and phagocytosis, even though the presence of this O-specific antigen is not necessary for the survival of the bacteria *in vitro.*

The lipopolysaccharides also carry immunodominant structures (O-factors) against which the host immune system produces antibodies. Therefore, the O-specific chain is responsible for the O-antigenic properties of the lipopolysaccharides. The O-factor has also been found to promote such beneficial effects as suppressing tumor growth by stimulating certain specific cytokines and interferons, inhibiting foreign bacterial invasion, inhibiting viral adhesion to cells and/or tissue of the animal, stimulating individual defense mechanisms against invading bacteria through immune-modulating mediators and specific humor factors of the cellular immune response, suppressing cellular stimuli responsible for inflammation, suppressing-bacteria and virus infiltration, enhancing macrophage activation and complement (MHC) activation, reducing lymphocyte mitogenicity and minimizing endotoxic shock and pyrogenicity. In addition, other attributes possessed by this biological material include the potential to inhibit the expression of target molecules in vivo, i.e., ICAM-1 or laminan like adhesion molecules, and to re-regulate the message to specific cytokines and/or interleukines, adhesion molecules (ICAM) and viruses by preventing upregulation of expression of endothelial cells and inhibiting colitis (M. Crohn) and caragellnan-induced neutrophil migration into the subcutaneous or intestinal epithelial space.

The endotoxin produced by the enterobacteriaceae has both beneficial and detrimental effects. The beneficial effects lie in the O-polysaccharide portion of the molecule, whereas the endotoxic properties reside entirely on the lipid A moiety. However, in the intact bacteria, lipopolysaccharide and lipid A are in a complex with phospholipid and protein, respectively.

Thus, these bacteria exhibit both beneficial and adverse effects. These bacteria, particularly the enterobacteriaceae (e.g., E. Coli), produce compounds which provide immunostimulating effects, but, at the same time, also provide the deleterious and lethal side effects. Thus, the objective was to find a way to minimize the deleterious effects and to maximize the beneficial effects.

The detoxification of deleterious compounds and the use thereof has been described in the art.

U.S. Patent No. 4,436,727 describes the production of a refined detoxified endotoxin which, when combined with cell wall skeleton, resulted in a therapeutically effective composition for the treatment of cancerous tumors without the deleterious side effects which are normally associated with endotoxins. Furthermore, the detoxified endotoxin described therein is prepared from batch cultures of microorganisms after methanol-chloroform precipitation with subsequent acidic hydrolysis in order to obtain a crude lipid A fraction. The purified detoxified endotoxin was combined with the cell wall skeleton for imparting immunotherapy.

U.S. Patent No. 4,912,094 discloses the production of modified lipopolysaccharides, particularly de-3-O-acylated monophosphoryl lipid A and 3-O-acylated diphosphorphoryl lipid A under strict controlled alkaline hydrolysis which removes only the β-hydroxymyrystic acyl residue that is esterfied to the reducing end of the glucosamine at position 3. This material is being used against type I hypersensitivity in warm blooded animals sensitive to allergens, e.g. pollen allergen, mold allergen, insect salina allergen, insect part allergen, drug and food allergen.

U.S. Patent No. 5,762,943 describes methods and compositions for treating type I immunoglobulin E (IgE)-dependent hypersensitivity by administration of monophosophoryl lipid A of 3-deacylated monophosphoryl lipid A. The biologically active material can be administered as part of a desensitization regimen or as a Type I of a prophylactic vaccine to prevent a Type I hypersensitivity reaction.

U.S. Patent No. 5,888,519 discloses high encapsulated high-concentration lipid A as an immunogenic agent. This disclosure aims to provide human antibodies in the form of hyperimmune polyclonal antiserum, or a human monoclonal antibody reactive with Gram-negative bacteria including providing effective passive prophylaxis against or therapeutic treatment of sepsis.

U.S. Patent No. 5,776,468 discloses a novel vaccine composition comprising small particles of 3-O-deacylated monophosphoryl lipid A. In particular, it describes how to prepare a certain particle size of less than 120nm, which can applied to induce protective immunity, even with very low doses of antigen. The specification provides evidence that these compounds protect against primary and current infections, and stimulate advantageously both specific humoral by neutralizing antibodies, and also effector cell mediated immune response. Moreover, it is alleged that vaccine compositions comprising small particles of the 3-O-deacylated monophosphoryl lipid A molecules, especially those below 120nm, as measured by photon correlation spectroscopy, are useful in providing protection against hepatitis infections (Hepatitis A,B,C,D, and E), and herpes (HSV-1 or HSV-2).

However, none of the aforementioned prior art describes the isolation of biologically active microorganisms from individual patients, suffering from acute or chronic infections of bacterial or viral origin, wherefrom a specifically active material or analog thereto can be obtained in a form of a viable non-replicating bacteria.

The present inventors, through a series of controlled isolation steps, have isolated a biologically active material which contains the immunostimulating effects without the toxic side effects.
More specifically, they found a biological material which minimizes or completely eliminates the toxic effects of the endotoxin induced translocation of bacteria in the gut and simultaneously promotes the beneficial effects of a lipid A-like material.

The present inventors found that this biological material is non-toxic and yet maintains the ability to inhibit the growth of other bacteria. It is known that bacteria can produce proteinaceous compounds that are lethal against other bacteria; at the same time the bacterial cell producing these proteinaceous compounds (called bacteriocins) are immune to its antagonist action. Thus, the present inventors found and isolated a biological material which maintained this property and yet is non-toxic.

The present inventors found and isolated such biological material. They found that by utilizing a certain isolated strain of bacteria of enterobacteriaceae, they were able to isolate a compound and prepare a drug therefrom which makes use of the immunostimulating effects attributable to this class of bacteria and simultaneously substantially eliminate the deleterious lethal side effects due to endotoxin induced translation of bacteria in the gut and in the gastrointestinal lumen. Moreover, they found that this isolated material exhibited the attributes identified hereinabove.

For example, they found that this isolated material exhibited immuno-stimulating effects. It is capable of stimulating the appropriate cytokines and interferons for suppressing tumor growth and inhibiting foreign bacterial invasion and adhesion of virus to cells. In addition, it promotes the stimulation of the defense mechanisms of the animal when invaded by viruses or bacteria. The isolated material also suppresses cellular stimuli responsible for inflammation, and suppresses bacteria and virus infiltration, enhances macrophage activation, reduces lymphocytic mitogenicity and minimizes endotoxic shock and pyrogenicity. In addition, this isolated material from these strain of bacteria re-regulates soluble and cell associated molecules involved in the process relating to the recruitment and retention of leucocytes at local sites of inflammation.

This material which they ultimately isolated from patients suffering from acute or chronic diseases is called Kyberdrug.

### SUMMARY OF THE PRESENT INVENTION

Thus, the present invention is directed to this isolated substantially pure bacterial material (hereinafter known as "Kyberdrug") which has the characteristics as described in claim 1, the material isolated from viable nonreplicating bacteria described herein.

The material called Kyberdrug was isolated from individual patients suffering from acute or chronic diseases and can be successfully administered to mammals in a dose dependent manner for successfully treating certain diseases, e. g. viral and bacterial infections. In 0.9% sodium chloride solution, they form colloidal crystals. In addition these colloidal crystals of the Kyberdrug as well as in aqueous solutions exhibit low polydispersity, having a narrow range of size of diameter of 0.65um, and are arranged in a face or body centered cubic bicontinuous phase or lattice. Upon heating, the colloidal crystals melt (Tm-47 C) forming a hexagonal lamella phase. Upon cooling the lamella phase reverses to the body or face centered lattice.

In addition, it was found that 10 uM aqueous suspension of Kyberdrug contains predominantly the aggregated form as colloid crystals of a monomeric unit of weight average molecular weight of about 1,900-2,100 daltons (99,95% w/w), and only a trace amount of the monomeric form (0.05% w/w) at equilibrium. Moreover, the aggregated form of the Kyberdrug dissociates into the monomeric form at a very slow rate at pH 7.8 (20 C), which is only influenced by the presence of Ca2+ or Mg2+ ions in the concentration ranges of 1-25 uM in case of Ca2+, and between 20-30 uM in the presence of Mg2+, respectively.

More specifically, as described hereinbelow, these Kyberdrugs are isolated from individual non-pathogenic strains of enterobacteriaceae (e. g., E. coli) found in mammals, including humans, using careful and specific selection process. Starting from selected individual bacterial cultures of mammals, the enterobacteriaceae are obtained from mammals, e. g., humans, which have been afflicted with infectious material, and can be found in feces, pus, sputum, urine, skin, and the areas of infection. Thereafter, the enterobacteriaceae are isolated, purified, and freed from contamination like enterotoxins, adhesions, and other unwanted toxic enterohemolysines including other proven pathogenic factors. The enterobacteriaceae are validated through specific and biochemical tests including a DNA amplification analysis of the product by, e. g., the polymerase chain reaction.

This strain of bacteria so isolated does not possess any endotoxins or any forms of verotoxins or hemolysins. It does not possess genes responsible for producing verotoxins, and does not contain activation factors for AMP adenylate cyclase, or c-GMP Guanylatecyclase. It, in addition, does not contain the eae gene sequence characteristic for EHEC or EPEC. It does not possess the lipid A molecules described hereinabove, in its toxic form. The bacteria producing the Kyberdrug are unable to replicate, but contain, among other biochemical active components, specific lipopolysaccharide-like structures, which are responsible for the specific immunemodulating effects in humans and animals.

The present invention relates to the production and therapeutic application of the biological material isolated from these non-pathogenic bacteriae (Kyberdrug), mainly enterobacteriaceae. The method of preparing a non-toxic biological material according to the invention is described in claim 2. The Kyberdrugs of the present invention are useful in treating infectious and chronic diseases as well as allergic reactions specifically and individually in mammals. More specifically, the Kyberdrug inhibits bacterial and viral activities. The Kyberdrug increases the immune response and defenses. The Kyberdrug of the present invention is useful in treating allergic diseases, such as asthma and is effective against both DNA and RNA viruses, including HIV-viruses, and coated and uncoated viruses. More specifically, this biological material isolated in accordance with the present invention prevents adhesion of rhinoviruses to hemagglutinin ii.) inhibits the viral neuraminidase, iii.) inhibits other bacterial and fungal infections, and iv.) inhibits the rate of transcription of the reverse transcriptase of retroviruses.

The present invention is also directed to a pharmaceutically effective amount of the biological material of claim 1 for use in a method of treating bacterial and viral infections in humans by administering effective amounts of the Kyberdrug thereto. It has been observed that patients treated with the Kyberdrug exhibit a decrease in the magnitude of the allergy or asthma attacks as well as the frequency of the symptoms, including the frequency of the seizures. This effect has also been observed in patients suffering from rheumatic diseases which also have been treated with the Kyberdrug of the present invention.

The Kyberdrug of the present invention thus inhibits bacterial and viral activities and reduces the observed clinical symptoms. Although it possesses the properties of the anchored lipid A properties, it does not possess the lethal effects of free lipid A, and is devoid of lipopolysaccharide (LPS) toxicity.

The present invention is also directed to pharmaceutical formulations containing an effective amount of the Kyberdrug in association with the pharmaceutical carrier for administration to mammals in a dose dependent manner for successfully treating certain diseases, e.g. viral and bacterial infections. The present invention is also directed to the isolated enterobacteriaceae which produce the Kyberdrug and at the same time exhibit the properties described hereinabove. Finally, the present invention is directed to the process for isolating the Kyberdrug.

### BRIEF DESCRIPTION OF THE DRAWINGS

Figure 1 (a) and (b) are TEM-micrographs of the Kyberdrug.
Figure 2 is a graphical depiction comparing the relative changes in interleuken 10β concentrations (pg/mL) in serum of a patient suffering from sinusitis who is either untreated or treated with the Kyberdrug (100 ug)
Figure 3 is a graphical representation comparing the regulation of various interleukines in a patient suffering from sinusitis who is either untreated or treated with the Kyberdrug.
Figure 4 is a graphical representation of the regulation of various interleukins stimulated by Kyberdrug (100 ug, subcutaneously) in a large number of patients (275) suffering from sinusitis before and after treatment during a 4 week period.
Figure 5 (a) depicts graphically the inhibition of HIV activity in vitro in the presence of Kyberdrug.
Figure 5(b) depicts graphically the anticoagulant activity as a function of concentration of *Kyberdrug* at two different ionic strengths.
Figure 6 is a diffraction pattern of the colloidal microcrystals of the *Kyberdrug* isolated in accordance with the present invention as a function of the scattering wave vector Q at 25°C using visible light at about 630 nm. The y axis is in units of a.u. (arbitrary units).
Figure 7 is a diffraction pattern of the colloidal microcrystals of the *Kyberdrug* as a function of the scattering wave vector Q at 37°C using visible light at about 630 nm. The y axis is in units of a.u. (arbitrary units).
Figure 8A is a X-ray diffraction pattern of the colloidal microcrystal at 25°C.
Figure 8B is a X-ray diffraction pattern of the colloidal microcrystal at 37°C.

### DETAILED DESCRIPTION OF THE PRESENT INVENTION

An aspect of the present invention, as indicated hereinabove, is directed to the Kyberdrug and its isolation thereof from the non-viable enterobacteriaceae, in accordance with the present invention.

The *Kyberdrugs* of the present invention act, communicate and control cellular events which are concerned especially with antagonistic control systems in humans and other mammals. They act like vaccines, but the *Kyberdrugs* are not vaccines or like substances.

Nevertheless, the *Kyberdrug* inhibits bacterial and viral infections. As is well known in the art, bacteria and viruses are mostly responsible for chronic and acute events, as well as inflammation, resulting from the generation of damaging chemical radicals which interfere with mammalian biochemical processes. The *Kyberdrugs* of the present invention inhibit the growth of infecting bacteria and viruses and thus retard their ability to generate these toxic chemical radicals. The *Kyberdrugs* possess lytic activities towards other microorganisms or viruses which may invade the mammal. However, the present inventors have found that the *Kyberdrugs* of the present invention are not genetic determinants. Without wishing to be bound, it is believed that the *Kyberdrug* protects against infection by directly incorporating attenuated DNA (RNA, genes) of the infectious microorganisms or viruses, rather than the proteins encoded by the genes. Moreover, the specific encoded proteins and enzymes are inhibited by the *Kyberdrug.*

The *Kyberdrug* is a large molecule. Studies of the hydrodynamic properties of the *Kyberdrugs* by means of inelastic light & static scattering experiments have shown that the *Kyberdrugs* are almost spherical in shape, having a hydrodynamic radius of < R_{H} > = 0.45 - 0.65 µm, which is almost insensitive to changes in ionic strength and temperature, even at very low concentrations thereof. This finding is of some importance since it generates a constant physical measure during production, and permits the determination of the concentration of the *Kyberdrugs* easily by measuring either size or masses through transmission absorption spectroscopy at 550 nm, or by using a light scattering detector. This is in contrast to the average hydrodynamic radius of viable *E.Coli* which is of the order of 6.2 µm, and has a rather large size variation of almost 50% depending on the nutritional and growth state of the *E.Coli* bacteria. In other words, the *E.Coli* are more than ten times larger than the *Kyberdrug*! Moreover, the production of *Kyberdrug* does not depend on the growth rate and nutritional state of the production of the bacteria, especially since the replicating properties thereof are abolished. MALDI-TOF-mass-spectroscopy and LC-MS-analyses of this material provided a molecular mass of approximately 1,900 (M₂ + Na)⁺, indicating the absence of any proteinaceous components, e.g. oligopeptides, oligonucleotides or nucleosides or derivatives of peptoglycans or muramyl peptides including those of N-acetyl-muramyl-L-alanyl-D-isoglutamine, normally referred to as muramyl dipeptide (Mᵣ= 459). However, the *Kyberdrugs* aggregate in a concentration dependent manner in aqueous solutions in the presence of NaCl or EDTA (0.9% w/w) resulting in the large hydrodynamic radius of < R_{H} > = 0.45 - 0.65 µm which is equivalent to a molar mass of 120,000 to 150,000 daltons having apparent aggregationals numbers of 68 - 80, even at *Kyberdrug* concentrations as low as 10 µg/mL. Thus, in aqueous solutions, it has a molar mass of about 120,000 to about 150,000 daltons.

The *Kyberdrug* is not a protein nor does it contain proteinaceous material. Moreover, it is not a nucleic acid. It is therefore not a glycoprotein nor a lipoprotein. In aqueous solutions, it consists of aggregate units of a monomer. The monomer has a molecular weight of about 1900-2000 daltons, while the aggregate has a molecular weight of about 120,000-150,000. Upon sonification or other methods of dissociation, the aggregate disassociates into the monomer, which is isolated and which as indicated hereinabove has a molecular weight of approximately 1900-2000 daltons.

The *Kyberdrug* is a lipopolysaccharide. The monomer contains an amino hexose. The sugar ring, contains from 6-12 carbon atoms and more preferably 8-10 carbon atoms. The sugar ring is a furanose. The sugar in the *Kyberdrug* is predominantly glucose, although galactose has also been found to be present in the Kyberdrug. It possesses bactericidal action, especially against mycobacteria, e.g., Nocardia.

When the *Kyberdrug* is isolated in an aqueous solution, it forms an aggregate and is believed, without wishing to be bound, to consist of at least 60-80 monomers per particle size of 0.3uM in radius. The *Kyberdrug* has a constant diameter of 0.65uM at constant chemical potential with a fairly small dispersion (δ) of 10-15% only. The size variation (δ) of the *Kyberdrug* at _ionic strength of 0.153 M NaCl at constant pH (of 6.9) and temperature does not change significantly when raising the ionic strength to 0.300 M NaCl. However, a shrinkage of the hydrodynamic radius has been noticed as measured through inelastic light scattering experiments. A change from about 0.65uM at 0.350 M NaCl (20°C) to about 0.57 ± 0.06uM at 0.350 M NaCl (20°C) has been found, which is reversible upon changing the salt concentration back to the initial concentration. Moreover, below 0.153 M NaCl (20°C), the *Kyberdrug* expands to about 0.70 ± 0.08 uM at 0.015 M NaCl, which is reversible too.

The *Kyberdrugs* are composed of a plurality of colloidal crystals. Without wishing to be bound, it is believed that the many colloidal crystals are stabilized and separated through strong electrostatic forces, hydrodynamic interactions brought about by its unique chemical structure and the ionic strength of the solution at ambient temperature. The *Kyberdrugs* have either a face-centered cubic (fcc) crystal lattice or body centered cubic (bcc) lattice, and they are interchangeable under appropriate conditions. For example, when placed in a dilute salt solution, e.g., 0.07% NaCl, a crystal lattice in the fcc is transformed to a crystal lattice in the bcc form.

The *Kyberdrug* of the present invention is an aggregate of the modified form of lipid A or the lipopolysaccharide containing the modified Lipid A molecule. Lipid A consists of a backbone of (β-1,6)-linked D-glucosamine dissaccharide which carries phosphate residues in positions 1 and 4' and amidate or esterified long chain fatty acids (generally D-3-hydroxy and/or acyloxy fatty acids) in each of the possible sites in the glucosamine moieties.

The modified lipid A molecule, collected and isolated in accordance with the procedure described hereinbelow contains:
i) a reducing sugar end, where the sugar is a hexosamine, mainly N-acylated D-glucosamine or D-galactosamine, respectively; and there are two sugars to the hexosamine present per monomeric unit of molecular weight of about 1,900-2,100;
ii) an ester linkage and/or a peptide linkage; these linkages are believed to arise from the condensation of the amino group of the hexamine with a carboxy group of a fatty acid;
iii) fatty acids of (R)-3-hydroxytetradecanoic acid having a free unesterified R-3-hydroxy group;
iv) an esterified (R)-3-hydroxytetradecanoic acid fatty acid residue, which is esterified with another saturated fatty acid at the R-3-hydroxyl of the (R)-3-hydroxy-tetradecanoic acid of chain lengths of n=12 and or n=14; and
v) no phosphate group or one phosphate group per monomeric unit of the *Kyberdrug* of monomer molecular weight of about 1,900 to about 2,100 daltons.

These characteristic chemical features are obtained from either mild acidic or alkaline hydrolysis with subsequent GC-MC determinations through chiral capillary columns, MALDI-TOF-MS in the presence of a suitable matrix, and specific enzymatic attack with acyl specific hydrolases or N-acyl-amidases in apolar solvents in the presence of 0.01% (v/v) water, respectively. The absolute R or S configuration was also determined by means of optical rotation of the isolated enantiomers of 3-hydroxy-tetradecanoic acid of chain lengths of n=12 and or n=14 in additional to chiral GC-MS techniques by comparing the enantiomers with those obtained through asymmetric synthesis including NMR spectroscopy in the presence of a suitable phase shift reagent.

The *Kyberdrugs* of the present invention are lipid A molecules containing predominantly no phosphate groups at either position 1 or 4' in the aggregate (hereinafter also known as modified Lipid A). This modified form of lipid A of the present invention, however, does contain a minimal amount of phosphate groups. The modified lipid A molecule (*Kyberdrug*) has a very low phosphate content. More specifically, it consists of at least about 80% (w/w) of non-phosphorylated lipid A analog and at most about 20% (w/w) of a mono-phosphorylated lipid A analog. An Example of monophosphorylated modified lipid A molecule has the structure: 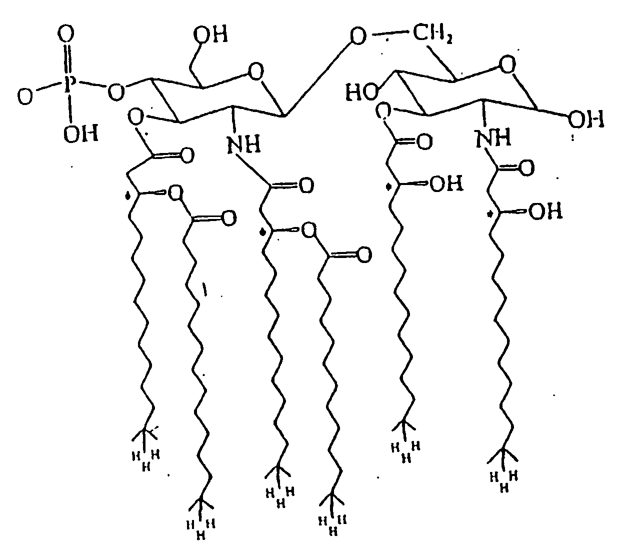 wherein
R and R' are independently hydrogen or 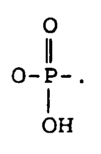
However, when formed in the aggregate, in at least 80% (w/w) of the aggregate, R and R' are hydrogen, and in at most 20% (w/w) in the aggregate, one of R and R' is hydrogen and the other is phosphate. But, a minimal amount, if any, of the monomer is found wherein both R and R',are phosphate.

The amount of monophosphorylated lipid present in the aggregate varies, depending on the patient, including the illness from which the *Kyberdrug* has been cultured and finally isolated in accordance with the procedure described hereinbelow. In addition, the monophosphate may be present on the monomer at the reducing end of the sugar, which is the 1-O-PO₃⁻ or the non-reducing end of the sugar, i.e., 4'-O-PO₃⁻, leaving the reducing end free and active. However, a small fraction of the diphosphorylated form at 1-position (reducing end sugar I) and at the 4' (non-reducing end, sugar II) of the dissaccharide moiety has also been isolated. However, no pyrophosphate derivative at either end has been found.

The Kyberdrug sugar amine, is predominantly a glucose amine with an amine moiety (NOH) substitute at C-2 and C-2' of the pyranose ring. The molecule does not contain an amine, however, for it forms part of an amide bond with a fatty acid, which fatty acid contains an even number of carbon atoms ranging from a total of 12 carbon atoms up to a total of 36 carbon atoms and more preferably from a total of 14 carbon atoms up to a total of 30 carbon atoms, wherein the β carbon to the carboxy group is substituted with a hydroxy group that has been esterified with a fatty acid of 10-20 carbon atoms, and more preferably, from 12-18 carbon atoms. It is most preferred that the size of the substituent on the 2 and 2' positions range from about 22 to 36 carbon atoms and more preferably from about 24 to 30 carbon atoms. It is preferred that the fatty acid is 3- hydroxy- tetradecanoic acid. However, whatever amide is present can be chemically modified by chemical reactions known in the art e.g., hydrolysis of the peptide followed by the reaction of the free amide with the desired fatty acid. Obviously, protecting groups known in the art may be utilized to protect the groups on the molecule which are reactive under the reaction conditions.

If the phosphate groups that are normally present on lipid A at C-1 and C-4' are not present, they are replaced in the modified lipid A molecule by OH groups.
The hydroxy group at the 3 and 3' position of the ring may be esterified with fatty acid containing 12 to 36 carbon atoms and more preferably from 14 to 30 carbon atoms wherein the β carbon to the carboxy group is substituted with a hydroxy group or a hydroxy group esterified with a fatty acid containing 10-20 carbon atoms and more preferably from 12-18 carbon atoms. It is more preferred that when the β carbon atom on the 3 or 3' position is substituted with a hydroxy group, the size of the substituent on the 3 and 3' positions thereon independently ranges from 10 to 20 carbons and more preferably from 12 to 18 carbon atoms. On the other hand, if the β carbon has a hydroxy group esterified to the fatty acid, the size of the substituent on the 3 or 3' position each independently contains 22 to 36 carbon atoms and more preferably 24 to 30 carbon atoms. It is most preferred that the fatty acid is 3-OH-tetradecanoic acid. The other positions, i.e., 4 and 6' are substituted with a hydroxy group. It is to be noted that the fatty acids of the lengths indicated hereinabove may replace those found on the isolated *Kyberdrug* by chemical transformations known in the art, such as by transesterifications, utilizing the desired fatty acids and the appropriate protecting groups known in the art.

It has been found that the monomer contains an even number of fatty acid moieties (either esterified or linked via an amide linkage) per monomer. Thus, the monomer may contain two, four or six fatty acid moieties.

As explained hereinbelow, the lipid A molecules described hereinabove are isolated from non-viable enterobacteriaceae. Thus, the present invention contemplates the various variations of the lipid A molecules described hereinbelow that are naturally present in the viable enterobacteriaceae.

In addition, the *Kyberdrug* has the following characteristics:
(a) They contain a substantially constant hydrodynamic radius of about 0.3 to about 0.40 µM having a low polydispersity index of about 0.05 to about 0.08%.
(b) The monomer molecular weight is about 1900 to about 2000 daltons.
(c) Aggregational numbers are between about 68 to about 75 for the *Kyberdrug,* and are almost constant with temperature, only slightly dependent on ionic conditions.
(d) The *Kyberdrug* has a number weight molecular weight of about 130,000 ± 9,000 daltons, a weight average molecular weight of about 135,000 ± 6,700 daltons and an average molecular weight of about 137,000 ± 10,700 daltons.
(e) The *Kyberdrug* contains two sugars per monomer, predominantly two glucosamines, although it may contain one glucosamine and one galactosamine.
(f) It contains inorganic phosphate, which is believed to be bound to one of the glucosamine molecule with the monomer of the *Kyberdrug*.
(g) The *Kyberdrug* contains 3-hydroxytetradecanoic acid, but no 2-hydroxy fatty acids.
(h) The 3-hydroxytetradecanoic acid is in the R-configuration.
(i) There are most likely an even number of the 3-hydroxy tetradecanoic acid per monomer of *Kyberdrug,* e.g., two, four or six.
(j) The isolated *Kyberdrugs* are colloidal crystals.
(k) The shape of the colloidal crystals, which resemble a liquid like material, can be arranged in the form of hollow spheres having a diameter of about 0.6 uM.
   These hydrodynamic forms act as little osmometers depending on various factors, such as counterions, pH and temperature. The pH of the solution in which the *Kyberdrug* is isolated is critical since at alkaline pH such as pH=7.5 or greater these aggregates can grow to huge aggregate having molecular weights of 1.17 x 10⁷ daltons or higher. This aggregation is reversible when the pH is lowered to 6.5. The huge aggregates are rather flexible, and connected through a hinge to yield a sort of tabular structure where each tubular structure has a tubular diameter of approximately 110 nm.
(l) The stability of the *Kyberdrug* in saline solution, e.g., 0.154 M NaCl as colloidal crystals is enhanced in the presence of about 5-10 nM Ca²⁺, as determined by UV/VIS-spectroscopy at 550-600 nm at 20-35°C.
(m) The monophosphorylated (20%) form within the *Kyberdrug* can be added to the non-phosphorylated form in the form of salts, e.g., Group I salts, such as sodium, potassium, ammonium, as well as the calcium and/or magnesium salts in an equivalent way. Other salts which could also be used include the nitrogen containing salts of choline, phosphocholine, L-lysine, L-arginine, L-histidine and L-proline. In addition, the following naturally occurring sulfur containing compounds can replace the salts:
   (a) L-cysteine
   (b) L-methionine
   (c) S-(+)-adenosylmethionine.
(n) The *Kyberdrug* can be non-covalently bonded to human serum albumin (HSA) as well as to L-polylysine or L-polyarginine (MW 9000-1000).

The *Kyberdrugs* or modified lipid A molecules isolated from the non-viable enterobacteriaceae in accordance with the present invention are substantially pure. It is preferred that the *Kyberdrug* is at least about 75% pure and more preferably that it is at least about 80% and even more preferred that it is at least about 85% pure and most preferred that it is at least 90% pure. Although some of the modified A molecules may have the core carbohydrates of the oligosaccharide as the L-isomer, the preferred embodiments have the core carbohydrates substantially as the D isomer. It is preferred that at least about 75% of the carbohydrates present is in the D isomer, more preferred that at least about 80% of the carbohydrate is in the D isomer, even more preferred that at least about 85% of the carbohydrate present in the D isomer, and especially preferred that at least about 90% of the carbohydrate present is in the D isomer and more especially preferred that at least about 95% of the carbohydrate present is in the D isomer and most preferred that all of the carbohydrate present is in the D isomer.

The *Kyberdrugs* of the present invention are isolated from non-viable enterobacteriaceae. The starting enterobacteriaceae from which the non-viable enterobacteriaceae are obtained generally are found in feces, pus, sputum, urine, skin or sites in the mammal, e.g., human, at which the enterobacteriaceae have infected. The starting enterobacteriaceae may be obtained from any Enterobacteriaceae, including parent organisms and mutants which are present at the sites indicated. By way of example, the following genera are illustrative of the type of organisms that may be utilized as starting enterobacteriaceae: Salmonella, Shigella, Escherichia, Brucella, Bordetella, Citrobacter, Pseudomonas, Pasturella, Neisseria, Proteus, Klebsiella, Serratia, Bacterioides, Bacillus, Carnobacterium, Caulobacter, Clostridium, Corynebacterium, Enterobacter, Halobacteria, Lactobacillus, Lactococcus, Leuconstoc, Listeria, Micrococcus, Mycobacterium, Pedioccoccus, Propionibacterium, Sarzina, Serratia, Staphylococcus, Streptococcus and Vibro. The following species may be employed: *S. minnesota, S. typhimurium, B. pertussis, B. abortus, S*. enteritidis, *E. coli, S. typhi, S. marcescens, S. typhosa, Shigella flexni and S. abortus equi,* and the like, *E.coli* and *Shigella* are the most preferred enterobacteriaceae utilized.

The enterobacteriaceae of the present invention which produce the *Kyberdrug*, and from which the *Kyberdrug* is isolated, are isolated by the present process. These bacteria produce proteinaceous compounds that are lethal against other bacteria. More specifically, these bacteria produce compounds which exhibit antagonistic activities against other bacteria, retroviruses and coated and uncoated viruses, but not against themselves. They possess all the antigenic components for exerting antigenicity, including immune-stimulating effects, without having the deleterious effects of endotoxins mediated bacterial translocation through the gut. Moreover, and most importantly, they are unable to replicate (to grow). Moreover, the non-viable enterobacteriaceae do not carry genetic determinants which are necessary for self-replication. Since the enterobacteriaceae are devoid of any phage-like lytic activities, but reveal certain bacteriocin like activities as well as some kind of lysogeny, these bacteria which produce the *Kyberdrug* have the advantage of being specific in a broad sense, since they have bacteriocin-like activity coupled with lysogeny. In addition, these bacteria induce subsequent colicin production, and are immune to infections from their corresponding original *E. Coli* or enterobacteriaceae from which the *Kyberdrugs* have been obtained.

Furthermore, the non-viable enterobacteriaceae from which the *Kyberdrugs* are obtained are of enteropathogenic serotype (EPEC), so localized adherence to the intestine resulting in cupping of the enterocyte and destruction of microvilli are avoided. In addition, the enterobacteriaceae are also devoid of enterotoxigenic *E. Coli* (ETEC), the entero-invasive *E. Coli* (EIEC) and the enterohemmorhagic *E.Coli* (EHEC).

The enterobacteriaceae utilized as starting enterobacteriaceae as well as the non replicating enterobacteriaceae may be gram-positive; it is preferred, however, that the enterobacteriaceae are gram-negative. It is also preferred that they are aerobic enterobacteriaceae. It is even more preferred that both the starting enterobacteriaceae and the non-viable enterobacteriaceae are rod-shaped. It is even more preferred that the starting and non-viable enterobacteriaceae contain the enzyme β-D-glucuroindase which reveals the fluorophore umbelliferon which is used as an indicator for successful culturing.

The non-viable enterobacteriaceae from which the *Kyberdrugs* are isolated possess the following properties:
(a) it is not viable; i.e., it is unable to grow or replicate.
(b) it does not have the ability to convert hydroxy coumarin-7-glucoside (also known as umbelliform-7-glucoside) to umbelliforme, which is a 7-hydroxy-2H-1-benzo-pyran-2-one also known as hydroxy coumarine). As used hereinafter, this reaction will be identified as the MUG Reaction. This is determined by a standard assay, known to the skilled artisan. See "Fluorogenic and Chromogenic Substrates used Bacterial Diagnostics" by M. Manoti, et al. in Microbiological Reviews, 55(3), 335-348 (1991).
(c) it is unable to convert tryptophan to indole (hereinafter referred to as the indole reaction), as determined by a standard assay, which is known to one of ordinary skill in the art.
(d) it has bactericidal action especially against mycobacteria. As used herein, "mycobacteria" are a genus of aerobic, gram-positive bacteria that are present in soil, water and tissues of various animal. They include the causative agent of tuberculosis and liposy. An example thereof is Nocardia.
(e) it possesses the colicin factor, i.e., bacterial plasmids that permit the organisms to produce colicins, which are a group of proteins produced by certain strains of Enterobacteriaceae, that are bactericial for certain strains of the same family.
(f) it lost the ability to produce hemolysins, an antibody that causes hemolysis.
(g) it cannot produce endotoxins, i.e., a toxic lipopolysaccharide released from the cell of gram- negative bacteria upon destruction of the cell.
(h) it cannot produce verotoxin in any form, neither the heat insensitive form nor the heat labile form.
(i) it does not possess any activation factors for producing c-AMP adenylate-cyclic or cGMP guanylate-cyclase.
(j) it does not have adhesion molecules such as ICAM I-III and does not permit viruses to adhere to cell walls.
(k) it does not have the eae gene sequence, characteristic for EHEC and EPEC.
(l) it is rod shaped.
(m) they belong to the class of Rough (R), smooth (S) or mucoidal forms (m), devoid of any hemolysine active material.

Using some or all of these identification factors, the non-viable enterobacteriaceae can be selected from other bacteria that are grown as described herein.

The *Kyberdrugs* of the present invention are prepared using standard microbial techniques of isolation of enterobacteriaceae strains from infected areas of mammals, including humans, selecting those strains which do not contain endotoxins, as determined by standard assays, and culturing and growing these strains, heat treating the selected strains at temperature and time sufficient to denature bacteriocins that may be present, extracting the *Kyberdrug* therefrom, and then lyophilizing and purifying the resulting product. It should be noted that the lyophilization step may precede the purification step or vice versa.

As described hereinbelow, the enterobacteriaceae are subjected to various assays, identified hereinbelow, which are used to select the appropriate species, and these are incorporated into the automatic VITEK system to verify that homolysine negative strains, e.g., citrobacter or Salmonella, respectively are absent. If they are present, these strains should be subsequently eliminated through continuous culturing and further analysis with VITEK.

These specific microbiological assays are all being applied to exclude pathogenic material originating from pathogenic microorganisms. Particularly, the combination of these sensitive assays, including the indole and MUG reactions ensures that only *E. Coli* bacteria and other non-toxic bacteria are being selected and identified.

During the selection steps, various assays are performed to verify that the correct strain is being isolated, and that the bacteria selected do not possess pathogenic material. Some of the assays test for the presence or absence of two enzymes that are present in enterobacteriaceae capable of producing endotoxins. They are tryptophandesamine and β-glucuroindase. The β-glucuroindase catalyzes the MUG reaction, described hereinabove. Trytophanase (tryptophansesaminase) in *E. Coli* catalyzes the deamination of L-tryptophase to indole, pyruvic acid and ammonium. This is possible if the microorganism is capable of producing L-tryptophase through its own gene, which in this case is characteristic of the enterobacteriaceae, particularly *E. Coli.* In addition, the addition of this enzymatic activity indicates the colinearity of this specific gene, and disregards any mutants which could be present in the enterobacteriaceae, especially, *E. Coli.* Those strains which give a positive test are maintained, while those which do not have those enzymes are discarded.

The presence of these enzymes is determined by standard assays known by one of ordinary skill in the art.

A standard assay for the presence of β-glucuroindase is described in G. Dahlen, et al., App. Microbiol., 26, 863-866 (1973); S.C. Edberg, et al., J. Clin. Microbiol., 24, 308-371 (1986); Kasper, et al., Appl. Environ. Microbiol., 53, 1073-1077 (1987).

Any assay for determining if tryptophendesaminase is present may be utilized. These are known to one of ordinary skilled in the art. For example, this may be determined using the indole reaction described hereinabove. Another assay which is used for verifying the selection of the correct strain is the MUG Reaction Assay, referred to hereinabove, in which hydroxycoumerin-7-glucoside (also known as umbelliform-7-glucoside) is converted to umbelliforme. The procedure is described in "Fluorogenic and chromogenic substrates used in Bacterial Diagnostics", by M. Manofi, et al., in Microbiological_Reviews, 55(3), 335-348 (1991).

The indole assay is also used for verifying that the correct strain is chosen. In the indole assay, tryptophan under the conditions described therein is converted to indole.

Another test which may be used is colicin determination. Colicins are a group of proteins produced by Enterobacteriaceae, that are bactericidal to other strains of bacteria of the same family. The enterobacteria isolated in the various steps have bactericial action against various bacteria including mycobacteria, such as Nocardia and the like. Thus, those strains which show a positive result from this assay are the strains which are desired, while those exhibiting a negative effect are discarded.

Another assay which is used to select the correct strain of enterobacteria tests for the presence or absence of adhesion factors and enterotoxins. These can be detected using the polymerase chain reaction (PCR) assay, which is described by B. Schutz, et al. in Lab Med., 17, 496, 1993, the contents of which is incorporated by reference. It is a well known screening procedure, which takes into account the various toxin types and subtypes occurring in man. More specifically, synthetic oligonucleotide primers complementary to the DNA sequence for the endotoxin genes, e.g., LTI, STI, VTI and VTII are prepared. The genomic DNA is isolated from the bacteria; if DNA polymerase denatures the genomic DNA and elongates the primer, then it is concluded that the strain contains the endotoxin. Those strains are discarded. If there is no reaction, then the strain cannot make endotoxin and it is maintained.

It is to be noted that the enterobacteriaceae that are collected and further harvested give positive results in all of the above assays, except in the colicin test, where it gives a negative result. It should also be noted that no one assay is determinative if the proper strain is collected, but the more assays that are performed which provide the appropriate results, the greater is the probability that the proper strain is collected. Nevertheless, it is preferred that prior to continuing the isolation process described herein, that the desired results are obtained from at least two of the above-identified assays. Moreover, after each harvesting step, as explained hereinbelow, at least two assays should be performed to assure that the selected strain has not been contaminated with other undesired bacteria.

The *Kyberdrugs* are prepared by applying standard, though controlled, microbial techniques of isolation of selected enterobacteria strains from human individuals, suffering from certain diseases, e.g., cough and cold, rheumatism, osteoarthritis, herpes simplex infection, HIV-infection or a serious staphylococcus infection and the like with their destructive side effects, then culturing and identifying the microbial strain, and subsequently culturing the strain and discriminating the desired colonies from contaminated bacterial strains which are unwanted and therefore eliminated. The general procedure is exemplified in Scheme I, which is attached hereto in the Appendix.

A sample from the infected area of the mammalian host is collected by standard techniques and the bacteria thereon are inoculated on Agar, containing nutrients typically used to grow and harvest bacteria, such as solid growth media. Typical nutrients include amino acids and sample carbohydrates. The bacteria are grown at about 37°C for at least about 12-18 hours; then the colonies are isolated. Bacteria samples from each of the colonies are subjected to various assays, such as those described hereinabove, for the determination of the presence of colicin, and absence of endotoxins. In addition, assays are performed to determine the presence or absence of β-glucuroindase and tryptophendisaminase, such as by the MUG reaction and indole reactions, respectively. More specifically, those colonies which undergo lactose utilization and have negative MUG and negative indole reactions and do not possess tryptophendisaminase or β-glucuroindase , and which have a positive colicin test, as determined by standard assays such as those described hereinabove, are isolated.

The selected bacteria are recultured on Microtiter plates, preferably 96-well, with saccharose. They are harvested at effective harvesting temperatures for sufficient time to colonize, preferably for 18-25 hours at about 37°C. Optionally, but preferably the strains are assayed to ensure no new mutations have occurred; thus assays known in the art to verify that the bacteria isolated are non-toxic enterobacteriaceae strains are conducted. Such assays include but are not limited to the standard assays described hereinabove, such as the MUG assay, the tryptophendesaminase assay, the PCR assay, and the colcinin assay, and the like. If all of the strains give negative results in all of the tests, except the colcinin test, and if the colcinin test is positive, then these process is continued. If, on the other hand, they give positive results in any of the above-identified tests (except the colicin assay) or give negative results in the colicin assay, then culturing is restarted or a new sample is obtained and the procedure followed as above.

The strains are identified by techniques known in the art, such as through the use of VITEK, a well known automative system used by the ordinarily skilled artisan for identification of clinical relevant strains and determination of resistance. VITEK is an automated assay system used in clinical microbiology, capable of identifying significant gram-positive and gram-negative bacteria and yeasts and performs antimicrobic susceptibility tests. The use of VITEK is described in an article in "Automation in Clinical Microbiology", by C.C. Pierson & K.D. McClatchey, in "Encyclopedia of Microbiology", Vol. 1, pp. 171-179 (1992), Academic Press, ed. by J. Lederberg, the contents of which are incorporated by reference. The VITEK automated system is commercially available, for example, it is sold by BioMerieux Vitek, Inc. in Missouri. Using the VITEK, those strains which are unidentified are then eliminated.

The selected strains are then cultured and harvested using techniques known in the art. It is preferred that they are harvested on agar containing the essential nutrients, e.g., a sugar (e.g., glucose) and amino acid(s) (or protein) containing sulfur and grown for sufficient time to colonize at temperatures effective for growth and replication. It is even more preferred that the strains are cultured on an endo-Agar or Diagonal Agar resin containing nutrients commonly used for growth under conditions effective for growth and for a time sufficient for growth to be observed. The preferred media and nutrients are depicted in Table I and the most preferred are depicted in Table II, which is in the Appendix attached hereto. It is preferred that the nutrients contain protein and sugar (e.g., glucose) and salt and optionally vitamins, including Vitamins B, e.g., B₆, B₁ and B₁₂ and the like and water soluble vitamins, and sulfur-containing amino acids, such as cysteine, cystine or methionine, yeast extracts and water. It is preferred that they be grown for 18-24 hours at 37°C.

The endo-Agar is considered a very selective resin, particularly for gram-negative aerobic enterobacteriaceae, and especially for those of rod-shaped nature. The preferred strains grown are the strains that contain B-D-glucuroindase such as E.Coli or Shigella, since the enzyme is useful in revealing the fluorophore umbelliferon, which can be used as an indicator.

Another way of culturing small and large amounts of the appropriate strain is through the use of solid Endo-Agar or Diagonal Agar in the additional presence of oxygen at about pH 7.0 and about 30°C, preserving the cell-bound-bacteriocins, e.g., colicin in the case of E.Coli, or the intra-cellular bound column like material.

Alternatively, the identified strains are grown using standard techniques known in the art. For example, they can be grown in bioreactors under sterile conditions using normal media utilized by one of ordinary skill in the art, e.g., broth, for growing for *E.Coli* bacteria.

It is preferred that the selected bacteria are cultivated on a bouillon medium in a bioreactor at effective harvesting temperatures for an effective amount of time to colonize, e.g., at 37°C, for about 12-18 hours. A bioreactor is more efficient, and the bacteria grow faster in a bioreactor than on agar. The broth media can have pH ranging between 6.5 to 7.5. Although agar media can be used, as in the previous harvesting step, the bouillon media is preferred at this stage since the growth of the proper strain is significantly faster than on the agar media and since most of the proper strains of enterobacteriaceae have been selected.

The selected strain and harvested colonies are next cultured on Diagonal Agar Resin containing the nutrients of Table I and more preferably in Table II for an effective amount of time to harvest same at effective temperatures. It is preferred that the strain are incubated at about 37 ± 1.0°C for about 12-18 hours.

Preferably, the bacteria are cultivated on Diagonal Agar under effective harvesting conditions, for a time sufficient to grow the bacteria. When grown on the Diagonal Agar, the bacterial shell is formed or has been formed. Normally, the bacteria are stored between 5-10°C on Diagonal Agar (Solid growth media). These bacteria either do not replicate or if they do replicate, they replicate at a slower rate than prior to this step. In addition, the action by the Col⁺ *E*. *coli* (that is, *E. coli* containing colicin) grow only on solid growth media.

To assure no cross contamination, it is preferable that the strain is assayed using standard techniques known to one of ordinary skill in the art to verify that the strain is enterobacteriaceae which contain minimal amounts, if any, of toxins. As used herein, by the use of the term "minimal", it is meant that the strain contains no pathogens or if toxins are present, that they are present in amounts which are not detected by the PCR assay described hereinabove. Thus, it is preferred that the cultivated bacteria on the agar be tested to determine if toxins are present. If the result from the assay is negative, then the process for preparing the *Kyberdrug* should be continued, otherwise, if positive, then the strain should be discarded.

Deviation from the above protocol or changes in pH, temperature or addition of nutrients to growth media, particularly sulfur containing amino acids such as (+)-S-adenosylmethione or yeast extracts containing water soluble vitamins, affects the amount of Kyberdrug and the ease of selection and further purification of the material. The ordinarily skilled artisan, however, can easily determine the appropriate conditions for the growth of the

enterobacteriaceae. However, the most preferred media are those tabulated in Table 2 in the Appendix, for these tend to maximize the production of colicin-like material (bacteriocins), especially when selected cells are propagated in broth at constant pH 6.5. Furthermore, the synthesis of intracellular and/or extracellular bacteriocin like material is inducible when using solid Agar. In the latter case, extracellular bound proteinacious material is released into the broth, while intracellular bound material remains with the selected microorganism production of bacteriocin-like material and is induced by exposure of the selected microorganism to media containing mitomycin C or UV light in the presence of oxygen. The inventors estimate that about 30-35% of the E.Coli strain obtained from the feces of a patient suffering from osteoarthritis or chronic rheumatism, and then grown in the presence of L(+)cysteine or L(-)methionine, respectively in the presence of O₂ and mitomycin C, is colicin positive (col⁺).

The genes encoding for colicin or colicin-like material including bacteriocins are almost exclusively located on coplasmids which are easily transmissible to other similar strains belonging to the family of Enterobacteria by such methods as conjugation or cell to cell contact. However, in the present culturing and selection, the activity of colicin and bacteriocin like material is effective against a small range of cultures of Enterobacteria.

The cultured strains are able to protect the bacteriocin-like producing strains from their own toxic metabolites. Maximum expression of the proteins responsible for the immunity occurs when the strains are grown via a non-inducing mechanism e.g., in the absence of mitomycin C, but in the presence of sulfur containing amino acid or yeast extract, water soluble vitamins and oxygen. However, expression of these protein compounds also occurs when only a small percentage of the bacteriocin-producing cells are producing bacteriocins; under these conditions, substantially all of the cells reveal evidence of producing free immunity protein. The regulation of these inhibitory system is constitutive and under the direction of the appropriate immunity determining plasmid, respectively.

The selected bacteria at this stage do not contain any replicating machinery. Their properties are tabulated in Table III, in the appendix. However, without wishing to be bound, it is believed that they still contain the LEX A protein which is responsible for preventing continuous synthesis of enzymes that repair damaged plasmid DNA and colicin or colicin-like material, respectively. Due to the selection of the bacteria in accordance with the present invention, especially those bacteria which were grown in the presence of oxygen and in the presence of sulfur containing amino acids including the Endo-Agar at pH 7.0, it is believed no repair process of DNA and synthesis of enzyme specific for DNA biosynthesis is possible; hence the replicating process is inhibited. The formerly present DNAs and RNAs degrade due to the enhanced activities of the DNAse and RNAse, respectively.

Without wishing to be bound, it is believed that the expression of the operon in growing cells e.g. *E.Coli,* is controlled by the SOS system that regulates error-prone DNA repair in these specified bacteria. SOS regulation involves the *LEX* A protein and *REC* protein, respectively. Furthermore, the operon promoter which is a very strong candidate for pore-forming colicins or bactericins, respectively, contains a *LEX* operator sequence consisting of two overlapped SOS boxes to which LEX A protein binds, preventing synthesis of enzymes that repair damaged DNA. Moreover, it is believed that the *LEX A* protein prevents the biosynthesis of colicin-like material and lysis proteins. It is believed that exposure to either DNA damaging agents or inhibitors of DNA replication results in the generation of an induced signal (mitomycin C) responsible for reversible activation of the REC A protein to its protease, respectively. The activated REC A protein then cleaves the *LEX A* repressor, resulting in the de-repression of the SOS regulated genes, subsequently leading to a production of the bacteriocin-like and lysis proteins. So overproduction of lysis protein may result in early cell death, but this seems to be regulated through the immunity gene which is apparently orientated in the opposite direction of the structural and lysis genes, and is not under the control of the SOS. Apparently, this regulator which includes the SOS system can be influenced in this case by the presence of a glucose medium, sulfur containing amino acids and/or the presence of yeast extracts in the presence of oxygen, since colicin production is reduced. So the regulator must be bound to the promoter region for transcription to occur, which is inhibited by the aforedescribed process. Thus, the nutrients available help regulate metabolic activities. Moreover, under the culturing conditions of the present process, replication is inhibited and template carrying DNA fragments are not available; thus the cells apparently shrink considerably, and release the before anchored outer lipooligosaccharides (*Kyberdrug*) having sizes of = 0.6 µm in hydrodynamic radius, when subjected to rinsing, as in the next step.

The selected strain is then rinsed with aqueous solution and more preferably saline solution or phenol. For example, the selected strain may be rinsed with dilute saline solution or phenol solution. It is preferred that the concentration of the saline solution is less than about 2% (w/w) and more preferably less than 1.5% (w/w); and even more preferably less than about 1% by weight. It is preferred that the concentration of the saline solution ranges from about 0.5% (w/w) to about 1.5% (w/w) and more preferably from about 0.7% to about 1.1% by weight and more preferably at about 0.9% saline solution.

A preferred dilute phenol solution has a concentration ranging from about 0.01% to about 1% (w/w) and more preferably from about 0.1% to about 0.5% (w/w) and most preferably at about 0.25%. The gentle rinse of the product forms the preformed *Kyberdrug*.

The last step is killing, that is, destroying the bacteria. This is effected under mild conditions using techniques known to one of ordinary skill in the art, such as by heat treatment, radiation, subjecting the bacteria to extreme pH's, proteolysis and the like. It is preferred that the bacteria is killed by subjecting the "bacteria" to a heat treatment under conditions sufficient to kill the bacteria and denature any protein present. Preferably, the inactivation is conducted at temperatures greater than 60°C and more preferably greater than 65°C, but less than 100°C, and most preferably at 75°C. They are preferably conducted in saline solution (e.g., NaCl). Preferably, the bacteria is subjected to this treatment for about 2 hours to ensure that the objectives are accomplished.

The disclosed killing of the bacteria, e.g. by heat treatment of the finally produced *Kyberdrug* according to Scheme I after washing off from the solid Endo-Agar or Diagonal Agar, respectively, with isotonic NaCl solutions ensures that no contamination of exogenic material including those from extracellular-bound bacteriocin like materials during harvesting with the *Kyberdrug* is occurring. Since the bacteriocins like proteins which are extracellular bound are unstable at temperatures above 55-60°C, the heat treatment at higher temperatures ensures that the final product is free of unwanted endogenic organic material. This can be analyzed through very sensitive HPLC (high performance liquid chromatography) analysis using a sensitive UV-detection system, or in the presence of a bound fluorescent indicator such as ethidium bromide or fluorescein at an excitation wavelength of 295 nm and an emission wavelength of 367 nm, respectively. This heat treatment affords a bacteria shell containing the *Kyberdrug.*

Depending on the loading with bacteria on the Diagonal Agar, viable bacteria can be isolated since they are cultured on Petri dishes. However, i) these grown microorganisms are not pathogens due to the selection process as tested by the PCR method. Moreover, if the suspension is subjected to conditions which kill the bacteria, such as heating, e.g. up to 75°C, as described hereinabove and, according to Scheme I, the replication is completely lost, and only the *Kyberdrug* is left behind.

Furthermore, the *Kyberdrug* released can adopt in the presence of 0.9% salt certain dynamic micellar forms, which are pH, temperature and counterion dependent, particularly Ca⁺⁺ and Mg⁺⁺. These conformational changes, or physical morphology changes is driven by charge interactions as observed by light scattering techniques (colloidal crystals) within the head groups of the *Kyberdrug* molecules (monomer = micelle equilibrium). Very similar interactions are occurring between the *Kyberdrug* molecules from rough as well as smooth strains.

Without wishing to be bound, it is believed that the low osmotic pressure brought about by the low saline NaCl solution also increases the segregation of the *Kyberdrug* from the shell. Optionally additional PCR tests can be performed to verify that the product obtained contains the desired material and does not contain any substances that is capable of being amplified. For example, nucleic acids or oligonucleotides are not present, or contaminating the *Kyberdrug.* Therefore the inactivation step is not only an additional security step, it is an important production step together with the Diagonal Agar. This means that the bacterial "shell" is devoid of any replicating material, because all other contaminants or contaminant bacteria are eliminated. In addition there should be no replicating machinery left, hence there is also no debris from the proteins which are involved in ribosomal biosynthesis.

Alternatively, instead of saline or phenol, the selected strain may be rinsed with sodium EDTA, 0.0025% (w/w) phenol, 0.005-0.015 %(w/w) ZnCl₂, or with a solution of 0.1 - 0.001% (w/w) ZnCl₂ -D-gluconate. The wash contains all the specific medicinal activities associated with the enhanced response towards foreign or hostile bacteria, viruses or immune-modulating effects. By rinsing the outer surface of the microbial shell, the lipooligosaccharide is released, but it is strongly influenced by the ionic strength of the saline solution and temperature. The saline wash with low ionic strength at pH between 4.5 - 7.0 is essential for the preparation of the *Kyberdrug.* Instead of saline, the "bacteria" may be additionally washed with tris(hydroxymethyl aminomethane•HCl. However, it is preferred that low concentrations of tris(hydroxymethyl)aminomethan•HCl (e.g., in the range 15 - 50 mM or less) are not utilized, if used, it is preferred that it is present in concentrations of at least about 100 mM (pH 7.0). Other amines, even at low concentrations, may also be used as the rinsing agent. Again, to ensure that the there is no endotoxin present, the PCR reactive material test may optionally be performed and if the results are positive, then the strain is discarded; but if negative the procedure is continued.

However, the *Kyberdrug* thus obtained may be extracted from the bacterial shell and then purified prior to being made into a pharmaceutical formulation by techniques known in the art. Two exemplary methods are outlined in Scheme 2.

In one methodology, the *Kyberdrug* is extracted with methanol/chloroform or methanol/acetonitrile. In this method, CHCl₃/CH₃OH or CH₃CN/CH₃OH, preferably in a concentration ranging from about 4:1 to 1:4 (v/v) and more preferably about 1:1 (v/v) is used; the *Kyberdrug* is placed therein preferably with stirring, and is heated to reflux under conditions sufficient to extract the *Kyberdrug* from the bacterial shell. The *Kyberdrug* precipitates therefrom and is collected. The crude *Kyberdrug* is then subjected to chromatography, such as HPLC using silica or alumina or methacrylate as the absorbent, or molecule exclusion chromatography, using a Sephadex column or DEAE cellulose column or BioGel using such solvents as chloroform, methanol, acetone, acetic acid, propionic acid, and the like. In a preferred embodiment, the precipitate is purified in preparative HPLC at room temperature wherein the adsorbent is RP-18, (methacrylate) Sephadex, G-100, G-200, Sepharose 2B,DEAE Sepharose, G-75 medium and the like. Preferably, a calibrated HPLC column using a spherical matrix, e.g., methacrylate of mesh sizes of approximately 50 - 100 µm and a light scattering detector attached to the HPLC apparatus is used to validate the size and mass of the *Kyberdrug* after calibration of the HPLC column with standards. This permits the size and shape of the *Kyberdrug* to be continuously monitored, and allows the final product to be validated throughout the production (in-process control).

The HPLC is connected to a UV spectrophotomer and the material which absorbs at 550 nm is collected. The fractions are pooled and the product is lyopholized.

Alternatively, the bacterial shell is placed with continuous stirring in a salt solution to form an aqueous dispersion at 20°-30°C. The salt is present in low ionic strength, e.g., less than 0.5 molar and more preferably less then 0.2 molar, but greater than 0.05M. Most preferably, the salt concentration is 0.154M. The preferred salts are inorganic salts, such as KCl, and especially NaCl and the like. The aqueous dispersion is stirred for sufficient time and under effective conditions to remove substantially all of the *Kyberdrug* from the bacterial shell. The aqueous dispersion is then passed through a Sephadex column, preferably Sepharose 2β or 6β as matrix or BioGel at 25°C in the presence of a solvent, such as mild saline solution (NaCl) having a pH between 6.5 and 7.5 at 20°C at an effective flow rate, such as, for example, between 0.1 and 10 ml/min. The material absorbing at 230 nm and 550 nm is collected, the latter being the adsorption in the UV of the aggregate of the modified Lipid A, while the former is the absorption of the monomer in the UV. The active fractions are pooled and then subjected to lyophilization. The pooled lyophilized fractions are dissolved in CHCl₃/MeOH or CH₃CN/MeOH in a ratio ranging from 6:1 to 1:1 (v/v), respectively and more preferably from about 5:1 to about 3:1 (v/v), respectively and most preferably at about 4:1, respectively and heated to reflux for sufficient amount of time to separate out the *Kyberdrug,* which is then chromatographically purified using standard techniques known in the art. Preferably, it is passed through a DEAE-cellulose chromatographic column at 25°C where the eluent is a mixture of acetonitrile and methanol or CHCl₃/MeOH solution wherein the acetonitrile or chloroform to methanol ratio ranges from about 6:1 to about 1:1 and more preferably from about 5:1 to about 3:1 (v/v) and most preferably about 4:1 (v/v). Added thereto per liter is a gradient of acetate or propionate salt ranging from 0.00 to 0.75M concentration.

The phosphate content thereof is determined using a phosphate ion detector. The phosphate detector may be connected to the column. In any event, as the eluent passes by the phosphate monitor, the phosphate concentration is determined; those fractions which do not contain any measurable phosphate content, or contains a minimal amount of phosphate is collected. The UV of each fraction is also measured at 550 and 230 nm. Again, it is preferred that a UV detector be attached to the column, such that the eluent passes through a UV detector. Only those fractions which exhibit absorptions in the UV at 550 nm and/or 230 nm are collected and pooled.

The size variation of the *Kyberdrugs,* including their mass distribution, and the validation of the monomer state of the material, i.e. no temperature induced or salt induced aggregation, can be monitored using either method through Gel-permeation chromatography using Sepharose 2B or 6B as described before. The exclusion limits are 10 x 10⁶ Da (Sepharose 2B) and the inclusion volume is assigned to molecules smaller than 100,000 Da, respectively. Simultaneous determination of the eluting material at 220, 265 and 280 nm in the absence of a fluorescent dye will record the purity and homogeneous chemical composition (absence of any contamination with nucleotides and nucleic acids, respectively, and absorbing proteins) of the *Kyberdrug.* The actual absorbance of the material on an absolute scale can be monitored at 660 nm using a light scattering detector with the appropriate cell by measuring the changes of the refractive index increment, (dn/dc)_{T,p} The results obtained are consistent with those derived from static light scattering experiments, transmission electronmicroscopy and HPLC runs as described above. A preferable eluting system for determination and validation of the size and concentration of the *Kyberdrug* utilizes an isocratic gradient and a Microsphere column (Beckman, USA), in which the *Kyberdrugs* are dispersed in an aqueous solvent containing 0.10 -0.190 M NaCl. The refractive index of *Kyberdrug* samples, (dn/dc)_{µ,T}, where µ is the chemical potential and T the absolute temperature, was determined to be about 0.160 ± 0.0005 mL/g, after calibration of the refractive index increments applying inter-ferometric methods as described in H.H.Paradies et al., J.Phys.Chem.,100, 9881-9891, 1996 and H.H.Paradies, J.biol.Chem., 254,7495-7505,1979.

By following either of these two techniques, a substantially purified *Kyberdrug* is obtained. Preferably, it is greater than about 70% pure and more preferably greater than about 85% pure and most preferably at least 90% pure.

Using the procedure described herein permits the selection only of those strains which are devoid of these pathogenic factors as stated before. Applying amplification methods according to the procedures known in the art permits the isolation of the appropriate strain, ensures the separation of the suitable strain of microorganism from the toxic one and provides the final product, i.e., the *Kyberdrug,* which is devoid of any lethal or pathological factors.

This selective process for obtaining the microorganisms before culturing at large is an essential part of the success of obtaining the desired material since it discloses the absence of any toxic or other pathological contamination of the *Kyberdrug* used for further culturing and harvesting, respectively. Particularly, the selection of said microorganisms from those containing the typical features of pathological appearances makes this process very special and attractive for analytical reasons as well as safe with regard to elimination of pathologic bacterial strains, viral or bacteriophage-like material (PCR-method) for further processing on an industrial scale.

### Physical -Chemical Analysis and Stability of Kyberdrug.

Analyses of samples obtained according to this disclosure have been performed by means of static and inelastic light scattering experiments including diffuse wave spectroscopy in order to deduce size and shape of the material in the absence of salt and in the presence of salt solutions. The techniques utilized are described by Paradies, Colloids & Surfaces, 74, 57-69, 1993, the contents of which are incorporated by reference. The inventors have found that the samples of *Kyberdrug* contain a constant diameter of 0.65 µm at a constant chemical potential with a fairly small dispersion (δ) of 10-15% only. The size variation (δ) of the *Kyberdrug* at ionic strength of 0.153 M NaCl at constant pH (6.9) and temperature did not change significantly when the ionic strength was raised to 0.350 M NaCl. However, a shrinkage of the hydrodynamic radius was noticed when measured through inelastic light scattering experiments and obtained from the autocorrelation function of the decay spectra, in which a change from an average of 0.65 µm at 0.153 M NaCl (20°C) to 0.57 ± 0.06 µm at 0.350 M NaCl (20°C) was found, which is reversible upon changing the salt concentration back to the initial concentration. Moreover, below 0.153 M NaCl (20°C) the *Kyberdrug* expanded to an average 0.70 ± 0.08 µm at 0.015 M NaCl which is reversible, too.

The *Kyberdrug* obtained is a colloidal solid, which exists in crystalline form.

The *Kyberdrug* of the present invention are stable.

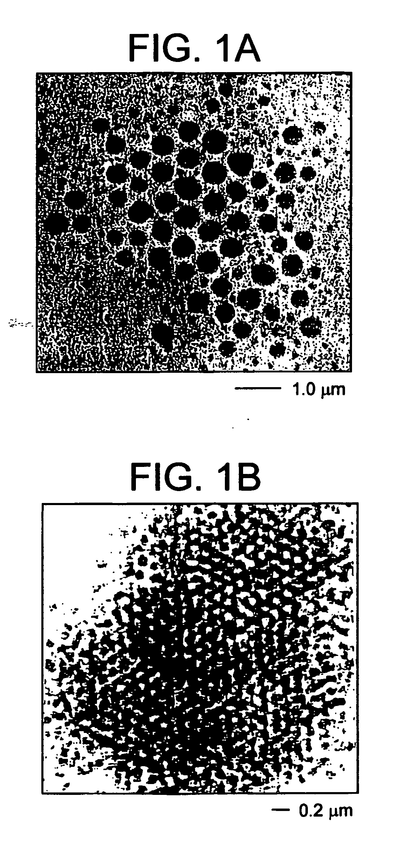

Without wishing to be bound, it is believed that the colloidal stability of the *Kyberdrug* can be understood in terms of the DLVO theory (see e.g. E.J.Verwey, J.Th.Overbeek: Theory of the Stability of Hydrophobic Colloids; Elsevier, Amsterdam 1948) which takes into account the electrostatic and the Lifshitz-van der Waals electrodynamic interactions between particles of certain sizes. The colloidal stability of the *Kyberdrug* and of *E.Coli*, respectively, which both carry negative charges at their external surface areas, as determined from electrophoretic light scattering and particle electrophoresis, are then interpreted in terms of the nature of the energy distance curves. For two spheres e.g., A and B, where A can be equal to B or different from B, and taking the determined average values for the *Kyberdrug* into account (0.65 µm), the mode of dependency of the A-B interaction on the separation distance can be calculated. Surprisingly, for the *Kyberdrug,* the values are positive, whereas those for *E.Coli* are all negative at all distances. When the A-B interactions are predominant over the electrostatic and van der Waals interactions, *E.Coli* (replicating state) has to flocculate rapidly (which is actually the case), while the *Kyberdrug,* indeed due to their repulsive A-B interactions, will remain dispersed at constant chemical potential, e.g. ionic strength and constant temperature. Unlike typical double-layer interactions, the A-B interaction potentials are largely insensitive to variation in electrolyte concentration and pH, as noticed for the *Kyberdrug;* hence their narrow size distribution and insensitivity to small changes in ionic strength finds its explanation here. The *Kyberdrug* remains stable for a period of time of several weeks. This situation does not change upon the presence of 0.25% (g/g) phenol. The colloidal stability of the *Kyberdrug* is further demonstrated by measuring the changes of the degree of hydration by means of NMR techniques, as well as of the actual mass using a ng-balance (quartz) for a certain number density of *Kyberdrug.* These measurements on samples obtained in accordance with the procedure described hereinabove revealed a mass density of an average of (1.51 ± .0.0095) x 10⁻⁴ g/mL, a degree of hydration of an average of 0.15 x 10⁻⁴ g/g (g water/g *Kyberdrug*), which is approximately 30% of the total mass of the material in the presence of 0.153 M NaCl. The translational diffusion coefficient and its dispersion under these conditions, as determined from the autocorrelation function, obtained through inelastic light scattering experiments (the method and the setup is disclosed by Paradies et al. in J.Phys.Chem. 98, 11143-11162, 1994, or ibid., 100, 9881-9891, 1996) yielded values of D = 0.432 µm²/s and a particle density of <n> = 2.18 x 10¹⁸ m⁻³ with a surface potential of 0.17 ± 0.02 V. Furthermore, the observation of opal formation of the *Kyberdrug* arising from the weak interaction of µm particles can be documented also through transmission electron microscopy (TEM, 2,000 keV) with a 1.7 nm point to point resolution (Fig.1). This finding with respect to the colloidal stability demonstrates that the observed "configuration" of the *Kyberdrug* corresponds to the minimization of the mesoscopic van der Waals energy of polydisperse particles. However, the driving force is the size dependence of the dispersion attractions. In addition there are surprisingly three important features to note: The radial distribution of particles sizes within the "island" is highly ordered, with the largest particles (approximately 0.6 - 0.65 µm) positioned at the center, and the smallest particles at the extreme borders. Secondly, the separation between nearest neighbors is independent of the particular *Kyberdrug* core sizes involved. Finally, even though the large particles constitute only = 3% of the total size distribution, they are able to nucleate and drive the formation of this opal *Kyberdrug* in the presence of 0.153 M NaCl. The results obtained are consistent with static light scattering measurements revealing a hydrodynamic radius of about 0.56 µm and a shape which is close to a hollow spherical particle with a thick outer shell that is highly negatively charged and pH dependent as well as salt dependent.

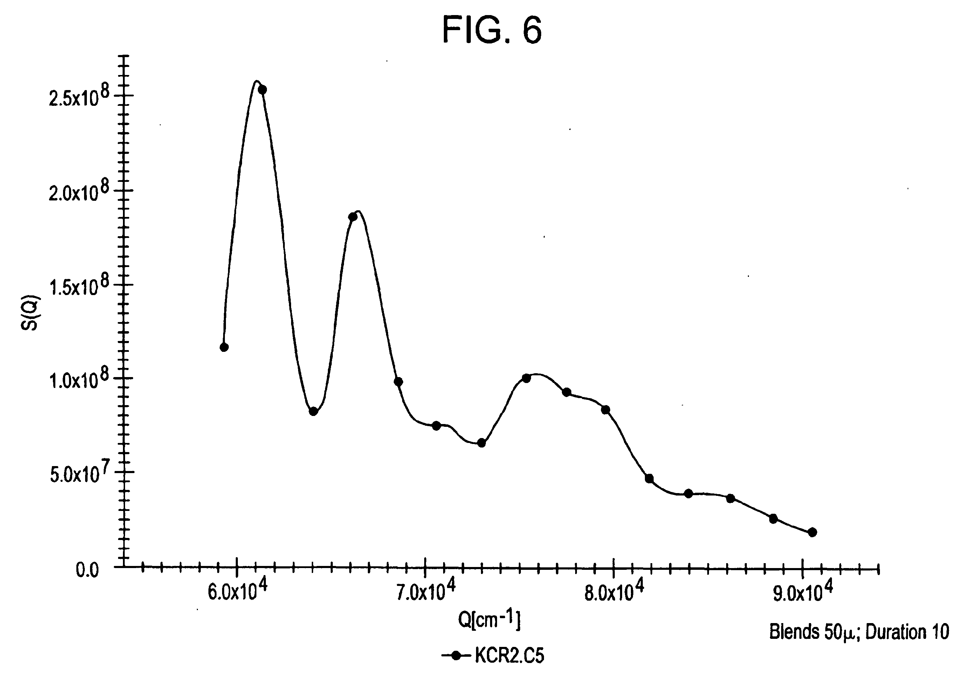

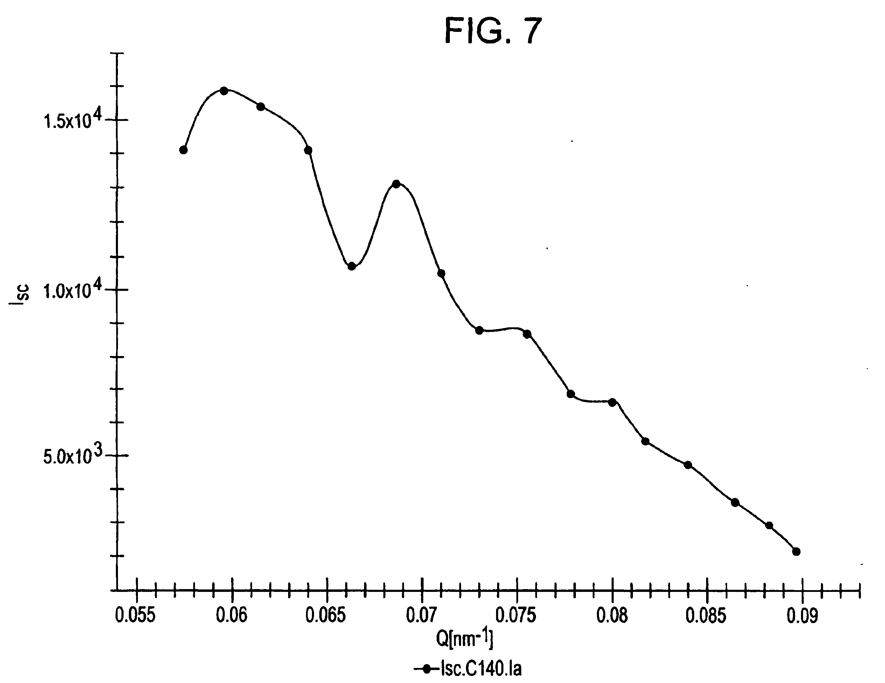

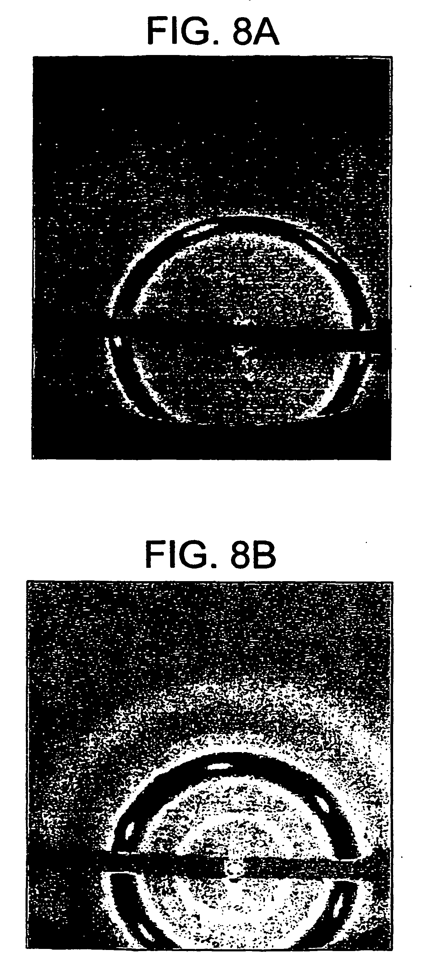

Light scattering curves can be obtained from colloidal crystals of *Kyberdrug* at low volume fractions in the presence of 0.154 M NaCl (25°C). (See, e.g., Fig. 6). The results can be compared to light scattering curves obtained from colloidal crystals of *Kyberdrug* under the same conditions at 37°C, which is about the melting point of the *Kyberdrug* (Fig. 7). These results combined together with the results obtained from inelastic light scattering experiments, and diffusive wave spectroscopy, performed simultaneously on the same material under identical conditions support the conclusions that this material (*Kyberdrugs*) is composed of many colloidal crystals which are stabilized and separated from each other through strong electrostic forces and hydrodynamic interactions. This conclusion is further supported from the X-ray diffraction pattern at 25°C (Fig. 8A) as compared to the X-ray diffraction pattern at 37°C (Fig. 8B).

The dynamic light scattering measurements and the static light scattering measurements were performed on a ALV light scattering goniometer 5000 (Langen, FRG). The goniometer spans from 15° to 250° in the scattering angle (θ) and a He-Ne-laser (10 mW) was used as a light source at 633 nm. The source of light was vertically polarized after passage through two pinpoint holes, and the focuses on the center of the cell. The measurements reveal that the suspensions of the *Kyberdrug* are crystal-like and structures of the *Kyberdrug* are fcc or face closed packed and/or bcc. Moreover, it was noted that the crystal lattice of the *Kyberdrug* can be transformed from a fcc subphase to the bcc phase under certain conditions. More specifically, it was noted that the bcc lattice transformation is high i.) when the salt concentration is lowered to less than about 0.1 M and more preferably less than 0.07 M NaCl, ii.) the suspension temperature is lowered below about 50°C and more preferably to about 37°C (~20-25°C), iii.) the charge density of the spheres is low due to the configuration charge rather than the unit charge of the polyelectrolyte (see above), or iv.) the osmotic pressure is low and time has elapsed since the preparation of the suspension containing the *Kyberdrug* or the technique of the preparation of the *Kyberdrug* in the presence of salt or pH is not changing during the formation of the colloidal crystals.

The *Kyberdrugs* do not self-aggregate under physiological and/or pharmaceutical conditions. At low ionic strength (1 mM NaCl and lower, e.g. 0.05 mM NaCl), the size distribution increases only slightly due to hydration, but the initial mass of the *Kyberdrug* does not change significantly. Increasing the ionic strength (250 mM NaCl) does not change the actual mass of the *Kyberdrug* that is being detected; it only decreases the hydration of 10-15% as determined by NMR measurements.

This behavior has also been observed from measurements of the viscosity of the *Kyberdrug* suspension under different ionic conditions at constant temperature, confirming that there is no large changes with dilution, thereby supporting the virtual non-existence of large electrostatic interactions without conformational change This means that the interactions are not directly related to the Debye length.

The *Kyberdrug* produced in accordance with the present invention are useful therapeutics. For example, they are useful in treating various diseases, including cough and colds, mainly caused by rhinoviruses and influenza viruses, catarrhal inflammations, skin infectious and infective dermatoses; dermatomycose, bacterially induced skin lesions, such as pyroderma, acne in patients suffering from inflammatory forms with pipules and pustules, acne vulgaris; otitis media, microbial and secondary infected exzema; mycoses, bacterial infections and secondary infectious to the skin due to gram-positive and or gram-negative bacterial infections, sinusitis, candida infections, particularly against candidaces of the skin, nails and mucus membrance, squamous cell carcinoma of the skin and mucosa, acute and chronic rheumatism and dermatological malignant growth. It also is useful in preventing viral infection and bacterially induced lesions such as pyoderma, and it represses carcinogen induced (line 10) hepatatocellular carcinoma.

It is believed, without wishing to be bound, that the ability of the *Kyberdrug* to induce a series of different antibodies in the early stimulating event of cellular and humoral immunity in a specific array makes it possible and applicable to protect the human (mammalian) organism against foreign invaders. Particularly, the *Kyberdrug* are capable of stimulating those cells which produce antigens and can increase these specific antigens in order to delete the invaded hostile microorganism, or chronic inflammation. The fast selection is brought about by the stimulated B-cells in order to generate specific antibodies.

It also reduces the side effects of asthma attacks. Without wishing to be bound, it is believed that it inhibits the key allergic factor, the atopy. Responding in an unique way to the *Kyberdrug,* the atopy response is reduced through the interaction of the antigen with the *Kyberdrug,* e.g., thereby reducing swelling from the influx of water and immune cells into tissue, local blood-blood vessel deletion and smooth muscle spasm.

The *Kyberdrug* of the present invention, as shown hereinbelow, influences the production of the cytokines and interferons.

It has antibacterial and anti-viral activity.

*Kyberdrugs* confer humoral and intestinal immunity.

*Kyberdrugs* may also be incorporated into regular and clinical immunization, also with other injectable vaccines, e.g. diphtheria, pertussis, tetanus;

*Kyberdrugs* are non-replicating agents; they exclude the potential for mutation and reverse virulence as well as bacterial infections; the absence of replicating virus and/or bacteria of the *Kyberdrug* permits its use in immunodeficient or immune-suppressed individuals and their hosts;

*Kyberdrugs* possess antiviral activities due to its polyvalent character; they inhibit viral replications of Herpes simplex, yellow fever, and polio viruses, without having any significant anticoagulant activity;

*Kyberdrugs* stabilize mast cells and prevent mast cells from secreting from their own internal granules such mediators of inflammation as histamine and leukotrienes when allergens bind to IgE molecules on mast cell surfaces. Therefore the *Kyberdrug* is useful for treating chronic inflammatory rheumatic diseases, particularly for chronic rheumatory diseases; e.g. osteoarthritis, muscle arthritis and primary chronic arthritis.

It is believed, without wishing to be bound that the ability of the *Kyberdrug* to induce a series of different antibodies in the early stimulating event of cellular and humoral immunity in a specific array makes it possible and applicable to protect the human (mammalian) organism against foreign invaders. Particularly, the *Kyberdrugs* are capable of stimulating those cells which produce antigens and can increase these specific antigens in order to delete the invaded hostile microorganism or chronic inflammation. The fast selection is brought about by the stimulated B-cells in order to generate specific antibodies.

The *Kyberdrugs* are useful in treating bacterial or viral infections and chronic as well as allergic reactions. The *Kyberdrug* inhibits bacterial and viral activities. They are useful in treating allergic diseases including asthma and in treating diseases caused by DNA or RNA viruses, including HIV viruses, and coated viruses. Without wishing to be bound, it is believed that they inhibit the rate of transcriptions of the reverse transcriptase of retroviruses.

The *Kyberdrug* is effective in treating bacteria and viral infections in mammals. Patients treated with the *Kyberdrug* exhibit a decrease in the magnitude of the allergy or asthma attack as well as the frequency of the symptoms, including the frequency of seizures. The effect has also been observed in patients suffering from the rheumatic diseases.

Moreover, due to their small particle size and colloidal crystals dispersed in diluted saline solutions e.g. at concentrations ranging from about 0.05 to about 1M NacL, and more preferably from about 0.1 to about 0.5M and most preferably at about 0.1M to about 0.2M, the *Kyberdrug* can be used as a vaccine against viruses and bacteria, e.g. against Hepatitis B surface antigen. Moreover, the *Kyberdrugs* can be used as an adjuvant against Type I hypersensitivity to allergens, e.g. pollen allergens, insect saliva allergens, insect part allergens, food allergens and the like.

The present inventors have found that its biological activity and its efficacy as a drug is enhanced when administered with a calcium salt, even at concentrations as low as 5-10nM calcium.

The *Kyberdrug* is administered in therapeutically effective amounts. The Kyberdrug is quite efficacious; only minute amounts need to be administered.

It is preferred that the *Kyberdrugs* of the present invention be administered in amounts ranging from about 0.01 mg to about 2 mg per kilogram of body weight per day. This dosage regimen may be adjusted by the physician to provide the optimum therapeutic response. For example, several divided doses may be administered daily or the dose may be proportionally reduced as indicated by the exigencies of the therapeutic situation. A decided practical advantage is that the *Kyberdrug* of the present invention may be administered in a convenient manner, such as by oral, intravenous, intramuscular or subcutaneous routes.

The *Kyberdrug* prepared from individual patients can be administered to these individuals again without showing any allergic reactions nor anaphylactic shock symptoms.

Moreover, the *Kyberdrug* is non-toxic. Without wishing to be bound, it is believed that the non-toxicity results from the absence of any endotoxin activity as well as to its efficacy, since only small doses are applied.

The *Kyberdrugs* of the present invention may be orally administered, for example, with an inert diluent or with an assimilable edible carrier, or it may be enclosed in hard or soft shell gelatin capsules, or it may be compressed into tablets, or it may be incorporated directly into the food of the diet. For oral therapeutic administration, the *Kyberdrug* may be incorporated with excipients and used in the form of ingestible tablets, buccal tablets, troches, capsules, elixirs, .suspensions, syrups, wafers, and the like. Such compositions and preparations preferably contain *Kyberdrugs* or concentrations ranging from about 0.1% to about 99% by weight. The amount of *Kyberdrug* in such therapeutically useful compositions is such that a suitable dosage will be obtained. Preferred compositions or preparations according to the present invention are prepared so that an oral dosage unit form contains between about 50 mg and 2000 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the *Kyberdrug,* sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the *Kyberdrug* may be incorporated into sustained-release preparations and formulations. For example, sustained release dosage forms are contemplated wherein the *Kyberdrug* is bound to an ion exchange resin which, optionally, can be coated with a diffusion barrier coating to modify the release properties of the resin.

The *Kyberdrug* may also be administered parenterally or intraperitoneally. Dispersions can also be prepared in glycerol, liquid polyethylene glycols, and mixtures thereof and in oils.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the *Kyberdrug* in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

As used herein, "pharmaceutically acceptable carrier" means a medium which does not interfere with the medicinal activity of the active ingredient and is not toxic to the patient to whom it is administered. Examples include any and all solvents, dispersion media, coatings, antibacterial and antifungal agents, isotonic and absorption delaying agents, oil-in-water or water-in-oil emulsions, aqueous compositions, liposomes, microbeeds, microsomes, aluminum hydroxide (aluminum hydroxide salt) and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier.

The *Kyberdrug* may be compounded for convenient and effective administration in effective amounts with a suitable pharmaceutically acceptable carrier in dosage unit form as hereinbefore described. A unit dosage form can, for example, contain the *Kyberdrug* in amounts ranging from about 50 mg to about 2000 mg. In the case of compositions containing supplementary active ingredients, the dosages are determined by reference to the usual dose and manner of administration of the ingredients.

The *Kyberdrug* may be administered in an aerosol spray using techniques known to the skilled artisan.

It is preferred that the *Kyberdrug* is administered in a subcutaneous form in the presence of 0.25% (w/w) phenol, or as a suspension of the desired concentration of *Kyberdrug* in 1% NaCl, or as a saline formulation in the presence 0.05%(w/w) ZnCl₂ having the same iso-osmotic pressure as a 1% NaCl solution. Another formulation comprises of 0.05-0.5 (w/w) Zn-D-gluconate or gluconic acid phosphate, and the desired concentration of *Kyberdrug,* in the presence of physiological concentrations of NaCl.

Apart from these formulations, another form consists of inclusion of the *Kyberdrug* in "carbomers", named for various Carbopol^{®} homopolymer resins according to the USP-NF, British Pharmacopoeia, United States Adopted Names Council (USAN) and the Deutsches Arzneibuch, which calls e.g. Carbopol^{®} 980 NF "polyacrylic acids". Carbopol resins, e.g. Permulen^{®} as a polymeric emulsifier and Noveon^{®} as polycarbophils, are polymers of acrylic acid crosslinked with polyalkenyl ethers or divinyl glycols. The powdered materials are supplied as flocculated materials (powders) of primary particle size averaging about 0.15 µm in diameter. The flocculated powders average 2 to 7 µm in diameter as determined by inelastic light scattering measurements, consistent with the measurements of the supplier, BF. Goodrich Specialty Chemicals, Cleveland, Ohio, 44141-3247, USA.

Since bioadhesion, particularly mucoadhesion, is desired for the mode of action of the *Kyberdrug,* the pharmaceutical inactive material must interact with mucus, which is a highly-hydrated, viscous anionic hydrogel layer protecting the gastric mucosa. The *Kyberdrug* is dispersed in bioadhesive material such as polyacrylic acid polymers, e.g. Carbopol^{®}, or Novean ^{®}AA-1 USP polycarbophil resins and Permulen^{®} polymeric emulsifiers, which make excellent bioadhesives for the encapsulated *Kyberdrug.* The polyacrylic acid polymers are preferred due to the advantage of the swelling and adhesion properties of these high-molecular weight polymers, and they are especially very suitable for inclusion of the *Kyberdrug.* Furthermore, the additional large adhesive surface area of these resins, in addition to the large surface area of the *Kyberdrug,* makes it very suitable for maximum contact with the gastric mucus or intestinal mucus layer.

A preferred formulation uses the *Kyberdrug* in combination with polyacrylic acid based material, such as Noveon^{®} AA-1 USP or Carbopol® 943P. These polymers, as produced, are flocculated powders having particle sizes on the average of 0.2 µm in diameter. Since the Carbopol^{®} resins as well as the Noveon^{®} polycarbophils are high molecular weight polymers of acrylic acid, which are chemically crosslinked with polyalkenyl alcohols, the crosslinked materials are water insoluble, but swellable in water or saline solutions.

Preferably a polyacrylic acid gel of 1.0-1.7% (w/w) and preferably between 0.7-0.8% (w/w) solids are used, dispersed in deionized water and neutralized to a pH of 4.5-5.0 at 25°C. To the polyacrylic gel is added the desired amount of *Kyberdrug* material in a saline solution, which is preferably less than about 1M saline solution and more preferably less than about 0.5 saline solution and even more preferably less than about 0.2M saline solution. It is most preferred that the *Kyberdrug* is dissolved in about a 0.154 M saline solution. Alternatively, the *Kyberdrug* is deionized and lyophilized, and finally dispersed, under continuos stirring, with the acrylic based materials in the presence of water or any desired solutions if the *Kyberdrug* are administered as a solution, and then sterilized. Whichever methodology is used, it is preferred that the pH of the *Kyberdrug* solution ranges between about 3.5 to 6.5 and more preferably between 4 and 5 and most preferably the pH is about 4.5 ±0.2 (25°C).

Another pharmaceutical formulation comprises the desired amount of *Kyberdrug* in an aqueous solution of dilute, i.e., less than 5% and more preferably less than 3% and most preferably about 1% (w/w), human or bovine serum albumin at pH 4.5-5.5. This is prepared readily from a 40% (w/w) stock solution by dilution with the saline solution.

Another pharmaceutical formulation comprises the desired amount of *Kyberdrug* in association with a hydrogel or polymers of acrylic acid, such as Noveon® A-2-USP, Noveon® AA-USP polymers or Cabopol® 934 PNF. These polymers form a gel when exposed to a pH environment greater than 6.5 ± 0.5, i.e., in acid pH. Upon swelling, the *Kyberdrugs* are released especially when the Ca⁺² salt of polycarbophils (Noveon®) is applied.

The hydrogel formulations containing the desired amount of *Kyberdrug* are discrete microgels comprised of many polymer particles in which the *Kyberdrugs* are dispersed. Although the hydrogels are not water soluble, when fully hydrated, osmotic pressure from within forms holes in structure, permitting the *Kyberdrug* to diffuse through the gel layer.

The hydrogel formulations described hereinabove containing *Kyberdrug* are also useful for phonophoresis. The *Kyberdrug* is in the form of a gel and is applied directly to the skin at the infected area. Frequencies in the range of 11-15 MHz are applied thereto; this action helps to diffuse the high molecular weight material (*Kyberdrug*) through the skin. Furthermore, in this special formulation, low-frequency ultrasound just above the range that is audible to the human ear enables dissolved *Kyberdrug* in carbophils in lipid regions between skin cells to penetrate and start to move into the interior.

Furthermore, the system described hereinbelow in association with the polymers of acrylic acid can be made into an adhesive-backed tablet using techniques known in the art. The tablet is placed between the gum and the lip. As the tablet with the *Kyberdrug* dissolves, it delivers the *Kyberdrug* directly into the bloodstream via the mucous membrane of the mouth. The tablets, containing the *Kyberdrug,* according to the disclosed formulation, can be less than 1 cm in diameter. The tablet comprises the carbophil-permeation-enhanced oral transmucosal system described above. Additionally, they may contain an enhancer, such as alginate as the Ca-salt or highly pyruvylated Xanthan, respectively.

Another preferable pharmaceutical formulation for the *Kyberdrug* comprises complexes between *Kyberdrug* and enantiomers or racemates of lysine, ornithine, arginine and methionine. Another embodiment for solid and liquid formulations, especially for administration parenterally, consists of the Kyberdrug in association with 1-amino-1-deoxy-D-glucitol (glucamine), or ribamine (which is the galactose stereoisomer), D-gluconic acid or its δ-lactone.

Another preferable formulation comprises the Ca²⁺, Mg²⁺ and Zn²⁺- salts of the gluconic acid or its corresponding δ-lactone in association with *Kyberdrug.* The Zn²⁺ salt of D-gluconic acid, Zn[OOC-(CHOH)₄-CH₂OH]₂ or the corresponding Ca²⁺ or Mg²⁺ salts are very water-soluble having optical rotations of [α]²⁰_{D} -6.7° (c=1), [α]²⁰_{D} -5.9 (c=0.75), and [α]²⁰_{D}-9.5 (c=1), respectively, at pHs between 4.7-5.5 (20°C) in the presence of *Kyberdrug.* This complex between the *Kyberdrug* and the metal-gluconic acid compounds when placed in aqueous solution forms a clear and transparent solution and can be formulated into a large range of concentrations of the *Kyberdrug,* e.g. 1.0 to 10 mg. In addition, these solutions have a very low surface tension of about 32 mN/m in the presence of 1.0 x 10⁻⁵ g/mL *Kyberdrug,* versus 79 mN/m just for the D-gluconic acid salt complexes alone. Moreover, when the material is lyophilized, the re-dissolved powder in water or saline solution yields a transparent solution again, revealing after analysis no deterioration of the *Kyberdrug* or loss of biological activity, respectively.

Simple formulations for the *Kyberdrug* make use of the binding capacity of this material with Ca²⁺, Mg²⁺ and Zn²⁺ ions at concentration of about 0.001% by weight to about 0.1% by weight and most preferably about 0.01% (w/w) Zn²⁺ (e.g. as a chloride), 0.001% by weight to about 0.1% (w/w) and most preferably about 0.002% (w/w) Ca²⁺ or 0.01% (w/w) to about 0.1% (w/w) and most preferably 0.04% (w/w) Mg²⁺ (preferably as chlorides or hydroxides). These solutions can be prepared also in the presence of 1% (w/w) NaCl solutions without changing the osmolarity of the total solutions. These formulations have the advantage of not having phenol in the formulation, but showing the same stability (e.g. shelf life time, storage) and antimicrobial activities as solutions containing 0.25% (w/w) phenol when administrating the *Kyberdrug* subcutaneously.

### Delivery of Kyberdrug in the presence of liposome- like formulations.

The *Kyberdrug* can also be formulated with cationic lipids or derivatives thereof, e.g., dipalmityl- or dipalmitoylphosphatidylcholine, dipalmityl-dimethyl ammonium chloride or racemic or enantiomeric lactate, as well as the corresponding C₁₈-di n-alkyl dimethylammonium derivatives in a pharmaceutical formulation. More specifically, they can be applied as an aerosol, which contains the *Kyberdrug* in a liposome. Thus, the *Kyberdrug* can be administered to the lung non-invasively.

Either dipalmitoylphosphatidylcholine (e.g., 1% w/w) which is a zwitterionic lipid that can act as a weak cation at physiological pH, dispersed in NaCl solution (e.g. oilsum), or dipalmitoylphosphatidylcholine (preferably 1:1 as a 1% w/w solution) in the presence of the wanted *Kyberdrug* concentration can be used, and the liposome-like dispersions easily prepared by sonication using standard equipment (50 W, 35°C, 1.5 min.). Microemulsions can be prepared by using the same cationic lipid materials; additionally, the carrier may be medium olive oil, or oleyl alcohol [(Z)-9-octadecen-1-ol] or oleic acid [(Z)-9-octadecenoic acid] as well as the corresponding sodium salts of oleic acid in the presence of 10% (w/w) water. Also a preferable preparation is the liposome-like formulation of dipalmityl- or dipalmitoyl-dimethylammonium lactate ® or S-enantiomer) or pyruvate as counterions in a solution with dioleyl phosphatidylamine (1:1w/w) in 0.154 M NaCl. The *Kyberdrug* is encapsulated in these lipids through spiking of the *Kyberdrug* with ethidium bromide or covalently fluorescent labeling *Kyberdrug,* i.e, with fluorescein thiocynate or dansyl chloride.

Aerosols containing *Kyberdrugs* can be generated by an ultrasonic nebulizer, e.g., Model 646. DeVilbiliss, Sumerset, PA, which produces aerosol droplets of approximately 5 um in diameter.

Unless indicated to the contrary, percentages are by weight.

Moreover, the singular include the plural and vise versa.

The term *Kyberdrug* includes not only the material isolated from the bacterial shell but also the product that is obtained after completion of the isolation steps described in Scheme 1. As used herein, the term *Kyberdrug* consists of the aggregate of monomer units. When referring to the monomer, the term monomer or modified lipid A molecule is utilized interchangeably.

The term "mammal" includes any species of the class mammalia and includes without limitation cat, dog, horse, goat, pig, and human. The preferred mammal is a human.

The following non-limiting examples further illustrate the present invention.

### EXAMPLE 1

### Upstream processing & downstream processing of Kyberdrug.

Non-pathogenic E.coli cells obtained from specimens of patients were grown on one of the media listed in Tables I & II after having met the selective criteria according to Table III in a Büchi Reactor-1.5 liter fermenter (Büchi Glas Muster, 1991, Fab.# 111 010,936,1385) at 37°C for 12-14 hrs. The cells were harvested by low speed centrifugation (3,000 rpm at 37°C), washed several times with deionized water (37°C), and either stored for further processing in water at 5°C as a sediment, or freeze dried and stored over Silica Gel at 5°C. The *Kyberdrug* was detached form the cells either by i.) 0.154 M NaCl at 20 °C, by continuously rinsing the cells with an 100 fold excess of salt solution (0.154 M NaCl), or in the presence of 0.01 %(w/w) ZnCl₂-gluconate; or ii.) by 11.5% (w/w) phenol/chloroform extraction, subsequently followed by 88.5% chloroform extraction, and adding six volumes of ether/acetone (1:6 v/v) in the presence or absence of ZnCl₂, or ZnCl₂-gluconate or ZnCl₂- gluconic acid phosphate, respectively; or iii.) by rinsing with 0.025% (w/w) phenol in the presence of 0.154 M NaCl. The resulting precipitate was collected and filtered, subsequently lyophilized, and stored over Silica Gel at 20°C. The yield was 4.8 g of crude material (*Kyberdrug*) per 150 g dry E. coli cells.

500 mg of the crude material was dissolved in 50 mL acetonitrile-methanol (5:1,v/v). The resulting slightly opal solution was subjected to heating at a temperature ranging from 50-60°C for 60 min. The resulting product was then centrifuged again at 20,000 rpm (Beckman Centrifuge, J2-21 Rotor) at 20°C. A clear and transparent solution was obtained. This prepared material was subjected to Sepharose 2B (Pharmacia) column chromatography (1.5x150 cm) at 20°C. The column had been previously equilibrated by using the acetonitrile-methanol co-solvent (5:1 v/v) as described above. After loading (normally 50 mg) with the crude *Kyberdrug,* the column was eluted with a different concentration of acetonitrile-methanol solution (3:1, v/v, 20°C). Fractions of 1.5 mL were collected, analyzed for phosphorous content applying standard methods, and active fractions were collected, pooled and subsequently freeze dried. About 50 mg crude *Kyberdrug,* was obtained. This material was then subjected to the selective criteria outlined in Table III, or the screening process according to Scheme I to avoid any contamination with LPS-like material, lipid A, or other cell debris, which could contaminate the pooled fractions.

Fractions obtained by Sepharose 2B column chromatography were also analyzed by thin layer chromatography using inactivated Silica Gel H plates (Merck Darmstadt, FRG). 1.5 mg of *Kyberdrug* were loaded onto the plate (500 µm x 20 cm). The solvent system was acetonitrile-methanol-water-conc.NH₃-water in a ratio of 80:10:5:2 (v/v) at 30°C. The bands of *Kyberdrug* were visualized by spraying with either iodine or alkaline Cu-tartrate solutions. Alternatively, 0.001 M NaOH may be used instead of conc.NH₃-water in the ratios indicated hereinabove in combination with ninhydrin (spray). Other purification steps for low concentrations of *Kyberdrug* (100 µg scale or less) include high liquid performance chromatography (Scheme II) using a 100 RP-18 column (LiChrosphor, Merck, Darmstadt, FRG), and applying an isocratic gradient ranging from 80% (v/v) of acetonitrile and 20% (v/v) methanol to 95 % (v/v) acetonitrile containing 5%(v/v) methanol. The resolution of the *Kyberdrug* on the column was monitored either by 220 nm, or by measuring the changes of the refractive index increment, (dn/dc)_{µ,T} with retention time at 550 nm by light scattering detector techniques routinely, or by determinations of the phosphate content applying a phosphate sensitive electrode. In this case the samples have to be either hydrolyzed (10 µg/1mL) routinely, or after calibration of the phosphate sensitive electrode with a *Kyberdrug* standard directly.

### EXAMPLE 2

1 mg of pure *Kyberdrug* was dissolved in 10 mL of methanol (25°C) and diluted with aqueous solutions of 0.154 M NaCl (25°C), yielding the concentrations of *Kyberdrug* of 500 µg/mL down to 10 .0 µg/mL. The final concentration of *Kyberdrug* is determined by dry weight measurements, or by determination of the scattering at 550 nm using a standard for calibration, or by determining the phosphate content e.g. according to Bartlett, J. Biol.Chem., 234, 466-471, (1959**)**. 0.52 µg phosphate/mg *Kyberdrug* equals 0.48 µg/mg glucosamine-a typical constituent of the *Kyberdrug-*resulting in a constant ratio of glucosamine to phosphate of 1.08 ± 0.04 for the monomeric form of the *Kyberdrug.* The weight average molecular weight of the *Kyberdrug* in saline solutions is of the order of 80,000-120,000, resulting in 80-120 particles of monomer molecular weight of 1,900 ([m+Na]⁺). Thus, the total phosphate content or the ratio of phosphate to glucosamine is constant and not at variance, hence reflecting the equivalent total amount of *Kyberdrug* per mL. The monomer molecular weight of the *Kyberdrug* has been determined of these preparations by MALDI-TOF mass-spectroscopy (matrix: 3.5-dihydroxy-benzoic acid, or sinapic acid) and Fast-Atom-Bombardment (FAB)-mass-spectroscopy with thioglycolic acid as a matrix in the presence of butane and it has been found to be about 1900 daltons.

### EXAMPLE 3

The preparation of *Kyberdrug* according to Scheme 1 was followed. The number of cells can easily and with confidence be determined by plaque test counting, since the number of cells is proportional to the amount of *Kyberdrug* released during the precise washing process with e.g. 0.154 M NaCl. For instance, a population of 10⁹ cells per Diagonal Agar releases after extensive washing 0.51475 mg/mL *Kyberdrug* in the presence of 0.154 M NaCl, or 0.5104 mg/mL *Kyberdrug* in the presence of 0.025% phenol, respectively. The determinations of the final concentrations of *Kyberdrug* can be performed through a standard as mentioned in example 2, or by absolute dry weight measurements using a ng-balance, or can be controlled through standard phosphate determinations as described in example 2.

### EXAMPLE 4

### Kyberdrug-Lysine Complex

(a) various *Kyberdrug*-Lysine complexes were prepared as follows:
   1.46 g (0.01 mol) L-(+)-lysine or D,L-lysine were dissolved in 200 mL deionized water at 25°C; 500 µg or 1000 µg *Kyberdrug,* respectively, were each added to the (L)lysine or D,L-lysine solution. The temperature was raised to 40°C for a period of time of 2 hrs. The initial turbidity of each of the solutions vanished after 30 minutes, and the resulting transparent solutions were filtered through a 1G4 glass filter in order to remove any particulate matter. The various solutions were subsequently lyophilized. Yield: 1.95 g for 500 µg *Kyberdrug,* or 2.42 g in case of 1000 µg *Kyberdrug,* respectively. The product obtained was clear in a solution in 0.154 M NaCl; it was odorless and colorless. Melting point (under decomposition) m.p.= 156°C.
(b) The products of (a) were freeze dried. The flakes produced through freeze drying were pulverized to small particles by means of a mortar. When milling in a ball mill (Centrifugal Ball Mill model S 1000) in 30 minutes, a white powder was obtained which was free-flowing. The mean particle size (AAAS 79 and AAAS 82) was determined to be 5.0-6.0 µm through the use of a Coulter Multisizer II, employing 0.9% sodium chloride solution; however, the particle size can be increased to 50-60 µm.
(c) Preparation of a tablet:
   A tablet is prepared containing 500 ug. The inactive pharmaceutical ingredient consists of 1.0 mg gelatin, 1.5 mg cross-linked sodium-carboxymethylcellulose, 1 mg magnesium stearate in a total weight of 4 mg per tablet.
   The amount of gelatin was dispersed in water at 40°C, and the *Kyberdrug*-lysine complex previously mixed in a mixer under low mixing capacity was also added at 40°C. The resulting granules were dried in a drum roll at 40°C, and subsequently sieved through a sifting machine having a pore size of 1.6 mm. The final dried granulate was pressed to a tablet having a final weight of 4.0 mg.
(d) The procedure of (c) was repeated except 1000 ug *Kyberdrug* is used as the active ingredient. The total weight of the tablet was 4.5 mg.

### EXAMPLE 5

### Kyberdrug -(-)-N-Methylglucosamine Complex

2.0 mg *Kyberdrug* and 1.98 mg (0.01mol) D-(-)-N-methylglucamine were dispersed in 50 mL isopropanol/water (50/50,v/v) at 20°C. The solution was heated to 50°C for 30 minutes until a clear and transparent solution was obtained. Under continuous stirring and at 20°C, 50 mL of water was added thereto. The solution was turbid; in addition, a microcrystalline precipitate was formed which was filtered off, and the supernatant was concentrated until no more microcrystalline precipitate developed. The combined microcrystalline precipitates were dried over P₄O₁₀ in vacuum at 20°C. The final product was further dried in an oven over silica gel at 25°C until no weight changes were observed. Yield: 3.5 g. A tablet was formed using the conditions pressed under similar conditions and inactive ingredients as described in Example 4.

### EXAMPLE 6

### Kyberdrug-Poly-L-lysine-Complex

0.15 mg poly-L-lysine (25.6kD) was dissolved in 5 mL 0.9% (w/w) NaCl at 20°C in the presence of 500 µg *Kyberdrug.* The dispersed solution was sonicated (100W, Branson Sonifyer) for 30 minutes until a transparent solution was obtained. The resulting solution was purified by Sepharose 2B column chromatography (1.0x10 cm) applying a linear gradient ranging from 0.9% NaCl (0.154 M) to 1.7 M NaCl (25°C). The flow rate was maintained at 10 mL/h, and optically active fractions were monitored by 220 nm and/or by means of refractometry (550 nm) using an Abbe Refractometer (Zeiss). The peak eluting at a salt concentration of 0.75 M NaCl contained the *Kyberdrug*-poly-L-lysine complex according to chemical analysis and phosphate determination, respectively. The optically active fractions were pooled, lyophilized and stored over Silica Gel in a desiccator at 20°C. Yield: 0.1-0.12 mg dry weight.

### EXAMPLE 7

Poly-L-lysine-double chained cationic lipid-*Kyberdrug*-complex 0.15 mg of poly-L-lysine (25.6 kD) was mixed with 0.5 mg distearyldimethylammonium hydroxide (DSDMAOH) in 5 mL water at 25°C. To the clear solution, 500 µg *Kyberdrug,* which was dispersed in 1.0 mL of 0.154 M NaCl, was added. The resulting product was heated to 30°C for 30 minutes. A turbid solution resulted. The turbid solution became transparent upon sonication (100W, Branson Sonifyer) for 5 minutes. The solution was filtered through a Millipore Filter (0.9 µm) and subsequently lyophilized. The lyophilized material containing NaCl was dissolved in water (2 mL) and dialyzed against 100 ml deionized water at 25°C, applying several changes of the dialyzing water. The solution in the dialysis bag was lyophilized, and stored over Silica Gel at 20°C. No decomposition of this material was observed at 20°C to 30°C,. Yield: 0.92 mg poly-L-lysine-DSDMA-*Kyberdrug*-complex.

### EXAMPLE 8

### Cationic-Kyberdrug-Liposome Complex Preparation.

A mixture of Dioleoyl-phosphatidylcholine and dioleoyl-trimethylammonium propane was prepared in a 1:1 (w/w) ratio of 10 mg/mL in a chloroform/methanol solution (1:1 v/v). This was used as a stock solution. To this solution, 500 or 1000 µg *Kyberdrug* dissolved in 0.154 M NaCl was added with continuous stirring at 25°C, in order to ensure complete and intensive mixing of the two phases. The obtained solution was separated into both the organic and aqueous phases. The organic phase was washed with deionized water, separated again, and the organic phase dried over CaCl₂. This solution was diluted with the stock solution until the volume was 100 mL, and then the diluted *Kyberdrug* solution was subsequently sonicated to obtain a transparent dilute solution of *Kyberdrug.* The concentration of the *Kyberdrug* in the liposome was further diluted to 100 mL with chloroform/MeOH (1:1, v/v), dried under nitrogen in a narrow glass beaker, and subsequently desiccated under vacuum for 12 hours. After the addition of 1.0 mL deionized water (Millipore water), and 4 hours incubation at 37°C, the vesicle suspension was sonicated again to clarify for 10 minutes. The resulting solution of cationic liposomes containing 15 mg/mL material including the desired concentration of *Kyberdrug* was filtered through 0.2 µm Nucleopore -filters. For optical measurements the concentration of single unilammellar vesicles applied was between 0.1 to 0.25 mg/mL. The cationic liposome-*Kyberdrug* complex containing e.g. 500 µg *Kyberdrug* sizes was measured by dynamic light scattering (ALV 5000, Langen, FRG). The determined size distribution ranged between 0.02 to 0.1 µm in diameter with a peak around 0.085 µm. [Note: The *Kyberdrug* in aqueous solution has a size distribution around 0.6 µm in the presence of 0.154 M NaCl, or in organic solvents (such as chloroform, methanol, and the like or mixtures thereof); the sizes were measured at 0.002 µm, indicating that the *Kyberdrug* is completely differently orientated with regard to size and form in the cationic-liposome-*Kyberdrug* complex, when compared to the highly aggregated state of the *Kyberdrug* in aqueous solution.] The material with the encapsulated *Kyberdrug* in the desired concentrations of *Kyberdrug* in the liposomal preparation was stored at -20°C without any deterioration of the preparation. The solution was stable over two years time when stored at 20°C under nitrogen in the absence of any environmental radicals or outside contaminations, respectively.

### EXAMPLE 9

### Cationic-Kyberdrug-Liposome Complex Preparation using synthetic cationic lipids.

A mixture of dioleoyl-phosphatidylcholine and distearyldimethylammonium hydroxide (DSDMAOH), or dihexadecyldimethylammonium hydroxide (DHDMAMOH) were mixed together in a 1:1 (w/w) ratio in 60% chloroform/40% MeOH (w/w), or in neat chloroform or cyclohexane, respectively at 25-30°C. 1000 µg *Kyberdrug* dispersed in 0.154 M NaCl (10 mL) was added to 20 mL dioleoyl-phosphatidylcholine/(DSDMAOH) or (DHDMAMOH), respectively, under continuous stirring at 40°C and a N₂-stream until the two phases separated. This separation of the phases was almost instantaneous. The aqueous phase contains the inorganic material only, whereas the organic phase contained the *Kyberdrug*-liposome-complex.

### EXAMPLE 10

### Encapsulation of Kyberdrug in L-(+)-Lysine-dipalmitoyl-α-phosphatidylpropanolamine liposomes.

Covalent coupling of L-(+)-lysine to dipalmitoyl-)-α-phosphatidylpropanolamine or di myristoyl--)-α-phosphatidylpropanolamine, respectively, was accomplished by reacting 1,1-(dimethylethoxy)carbonyl-succinimidyl-L-(+)-lysine, which was dissolved in 1 mL chloroform/MeOH (1:1, v/v) containing 0.5 µmol triethylamine with 100 µmol dipalmitoyl-)-α-phosphatidylpropanolamine. The reaction was carried out at 60°C for 6 hours. Thin layer chromatography (TLC) analysis (Silica Gel H, solvent chloroform/MeOH/water, 65:25:5) of the reaction mixture revealed quantitative conversion, confirmed also by HPLC-analysis on a RP-18 column. After removing the solvent system and remaining unreacted material, e.g. 1,1-(dimethylethoxy)carbonyl-succinimidyl-L-(+)-lysine, the purified BOC-L-(+)-lysine-BOC- dipalmitoyl-)-α-phosphatidylpropanolamine was treated with 5 mL chloroform/trifluoroacetic acid (30:70). The mixture was gently stirred for 3 hours at 20°C, the solvent was removed under vacuum, and the residue was dissolved in chloroform. The deprotected product was analyzed by TLC, and phosphorous content, R_{F} ≅ 0.41 (CHCl₃/MeOH/H₂O: 50:40:10). The lysinyl- dipalmitoyl-)-α-phosphatidylpropanolamine was purified by carboxymethyl-cellulose (CM 52, Whatman) using CHCl₃/MeOH as elution solvent, or acetonitrile/MeOH in a ratio of 95:5. The product eluted in 15% MeOH, and was lyophilized and was stored at -20°C under N₂ where over the time of 1 year no deterioration of the material has been detected.

Liposomes of this material containing 1000 µg *Kyberdrug* was prepared in the usual way by probe sonication and dispersing in a buffer containing e.g. 10 mM TRIS-HCl, pH 7.0 -7.4 (20°C) in the presence of 0.154 M NaCl. The determination of the encapsulation efficiency with respect to *Kyberdrug* concentration and leakage etc. was determined by extracting the *Kyberdrug* with 1% (w/w) TRITON X 100, and subsequently analyzing the phosphorous content or the characteristic peak distribution in MALDI-TOF-mass-spectroscopy with 2,5-dihydroxy benzoic acid as a matrix. The stability of the *Kyberdrug* loaded liposomes was assayed under three different conditions; in the preparation buffer as described above at 4°C at various times; in a culture medium with or without calcium or magnesium (MEN 500 or MEN 400, respectively; ref: Gibco) and with or without 5% (v/v) fetal bovine serum after 4 hours incubation at 4°C, and in human plasma after incubation at 37°C. For all conditions the loaded liposomes were treated with Triton x 100 or with desoxycholate after incubation, and fractionated by column chromatography (Sepharose 2B), or by HPLC as described before.

### EXAMPLE 11

### Conductive skin gel containing Kyberdrug

The gel is prepared by mixing the contents of A, B and C together.

| Ingredients | % by Weight |
|---|---|
| *Part A* | |
| *Deionized water* | 68.00 |
| *Carbopol ETM 2001 Resin* | 0.85 |
| *Potassium hydroxide* | 0.05 |
| Propylene glycol | 15.00 |

| Part B | |
|---|---|
| Deionized water | 10.0 |
| Disodium EDTA | 0.05 |
| Carboxymethylcellulose (2%) | 15.00 |
| Potassium hydroxide | 0.50 |

| Part C | |
|---|---|
| *Kyberdrug* | 1000 µg |
| Propylene glycol | remainder |

### EXAMPLE 12

### Antipruritic Spray containing Kyberdrug

A spray was prepared by mixing together the following ingredients:

| Ingredients | % by Weight |
|---|---|
| Carbophol^{™} 041 NF Resin | 0.60 |
| *Kyberdrug* | 2000 µg |
| Ethanol | 50.00 |
| Glycerol | 20.00 |
| Water | q.s. |
| Total | 100% |
| pH | 6.15 |

This translucent, non-greasy aerosol preparation with *Kyberdrug* as an active ingredient is easy to dispense. It is designed to provide effective symptomatic relief of pain and itching associated with skin irritations and allergies.

### EXAMPLE 13

### Anti-inflammatory gel in the presence of Kyberdrug

The gel was prepared by mixing the following components:

| Ingredients | % by weight |
|---|---|
| Kyberdrug | 2,000 µg |
| Carbophol 9345 NF Resin | 2.00 |
| Tris Amino | 4.000 |
| Deionized water | q.s. |
| Total | 100.00% |

| Properties | |
|---|---|
| pH | 7.35 |
| Viscosity | 75,800 cPs |

In Experiments 14-17, a *Kyberdrug* was isolated in accordance with the procedure described herein. The chemical analysis performed in the isolated *Kyberdrug* in all four examples were performed through alkaline and acidic hydrolysis under strictly controlled conditions enzymatic hydrolysis according to general chemical and biochemical standard procedures. The methods for analysis were MALDI-TOF-MS, matrix assisted laser desorption ionization mass spectroscopy and ion-spray mass spectroscopy, respectively. The matrices used are DHP(3,5-dihydroxybenzoic acid) and 2-cyano-4-hydroxy-cinnamic acid. The mass spectra of MALDI-TOF were recorded in the positive and negative ionization mode for detection.

### EXAMPLE 14

Using the procedure described herein from the cultures of E-coli bacteria isolated from the urine and feces of patients suffering with symptoms of otitis media, a mixture was isolated. It was then subjected to the chemical analysis, described hereinabove.

The product isolated had the following structure; identified as Compound 1. 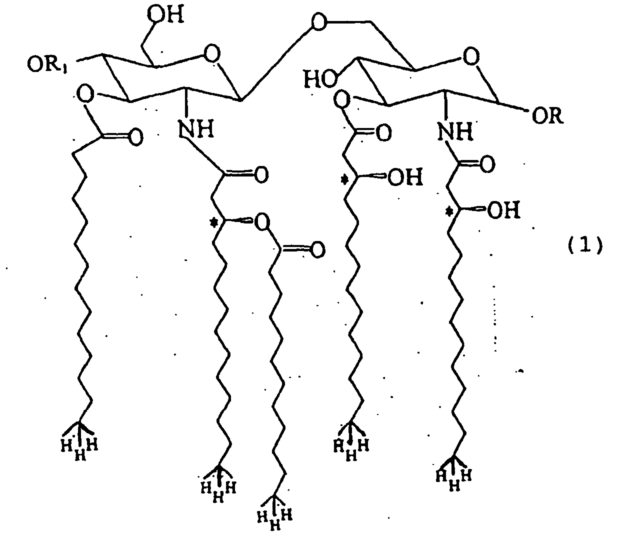 wherein R is hydrogen or 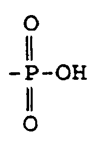 or salt thereof.

The isolated product was a mixture of about 80% (w/w) unphosphorylated glycolipid and about 20% (w/w) mono-phosphorylated glycolipid, where the inorganic phosphate is located at the 1-position or the 4'position at the other end of the disaccharide. A small amount of 1,4-diphosphoryl product of glycolipid was also isolated.

### EXAMPLE 15

Using the procedure described herein, from the cultures of E-coli bacteria isolated from the urine and feces of patients suffering from sinusitis, a mixture was isolated. It was then subjected to the chemical analysis described hereinabove.

The product isolated had the following structure, identified as Compound 2. 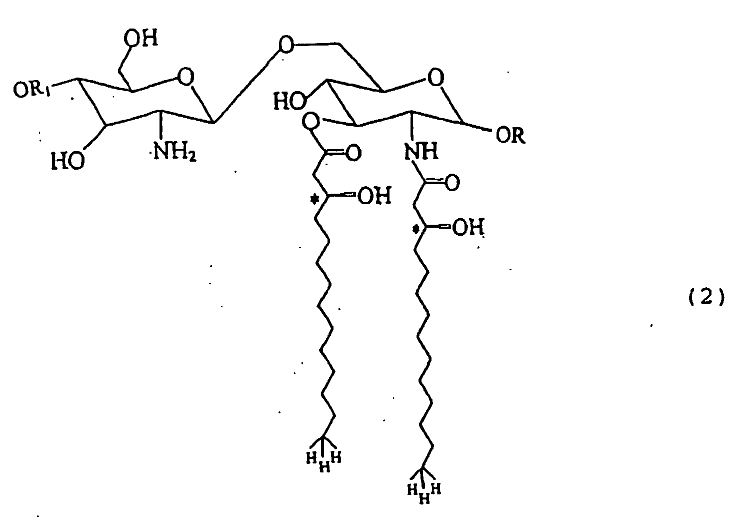 wherein R is hydrogen or 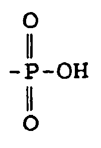 or salt thereof.

The isolated product was a mixture of about 80% (w/w) unphosphorylated glycolipid and about 20% (w/w) monophosphorylated glycolipid, wherein the inorganic phosphate is located at the 1-position or the 4'position at the other end of the disaccharide. A small amount of 1,4'-diphosphorylated product of the glycolipid was also isolated.

### EXAMPLE 16

Using the procedure described herein, from the cultures of E-coli bacteria isolated from the urine and feces of patients suffering from chronic rheumatism (osteoarthritis), a mixture was isolated. It was then subjected to the chemical analysis described hereinabove.

The product isolated had the following structure, identified as Compound 3. 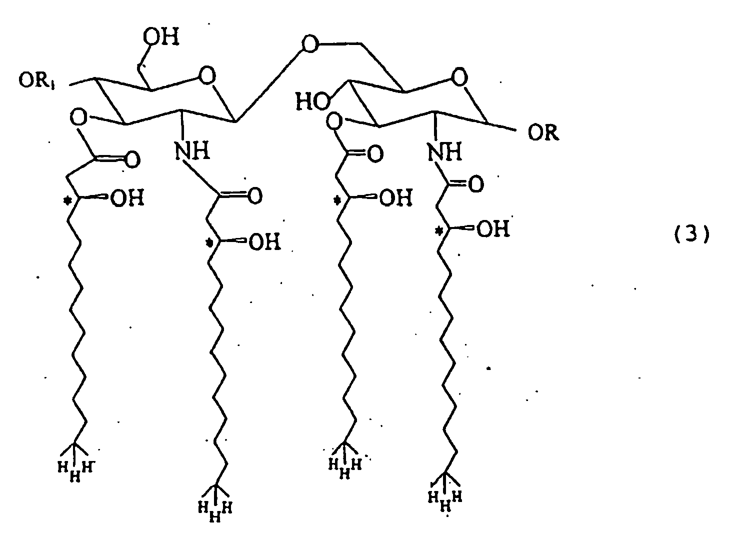 wherein R is hydrogen or 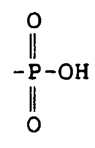 or salt thereof.

The isolated product was a mixture of about 80% (w/w) unphosphorylated glycolipid and about 20% (w/w) monophosphorylated glycolipid, wherein the inorganic phosphate is located at the 1-position or the 4'position at the other end of the disaccharide. A small amount of 1,4'-diphosphorylated product of the glycolipid was also isolated.

### EXAMPLE 17

Using the procedure described herein; from the cultures of E-coli bacteria isolated from the urine and feces of patients suffering from asthma bronchial, a mixture was isolated. It was then subjected to the chemical analysis described hereinabove.

The product isolated had the following structure, identified as Compound 4. 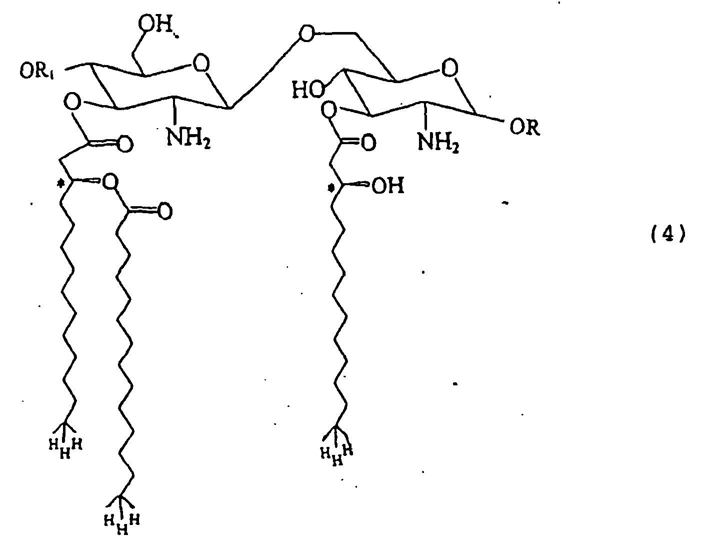 wherein R is hydrogen or 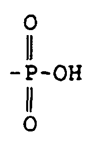 or salt thereof.

The isolated product was a mixture of about 80% (w/w) unphosphorylated glycolipid and about 20% (w/w) monophosphorylated glycolipid, wherein the inorganic phosphate is located at the 1-position or the 4'position at the other end of the disaccharide. A small amount of 1,4'-diphosphorylated product of the glycolipid was also isolated.

As shown hereinabove four different products were isolated. These products are modified free-lipid A molecule. The product contain a mixture of about 80% (w/w) unphosphorylated glycolipids and about 20% (w/w) of the corresponding monophosphorylated glycolipids, wherein the inorganic phosphate is located at the 1-position or the 4'-position at the other end of the disaccharide. The sugar component of the disaccharide in all four examples is the N-acylated glucosamine. To some extent, a small amount of the 1,4'-diphosphorylated product of the glycolipid was also present. The hydroxyl at C-6 is free, it is not acylated or bound to another component, e.g., amino acid residue or another sugar component.

Various stereoisomers of Compounds of 1-4 are contemplated to be within the scope of the present invention; the varius chiral centers are marked with an asterik and each chiral center may be in the R or S configuration. However, it is preferred that all of the chiral centers so marked are in the R configuration.

The unphosphorylated Compounds 1-4 can be separated from the phosphorylated compounds by techniques known to one of ordinary skill in the art, such as by chromatography, column chromatography HPLC, and the like. Once separated, other ratios of unphosphorylated compounds to monophosphorylated compounds can be prepared. It is preferred that the weight ratio of the Compounds 1-4 range from about 90:10 of the unphosphorylated glycolipid to the corresponding mono phosphorylated compounds to about 60:40, and the most preferred ratio is 80:20.

The unphosphorylated Compounds 1-4 as well as the phosphorylated Compounds 1-4 may also be present as pharmaceutically acceptable salts. Examples include pharmaceutically acceptable metal salts, e.g., especially Groups 1 and 2 metal salts, e.g., sodium, potassium, calcium, magnesium and the like.

In the aggregate, these mixtures isolated in Examples 14-17 will form *Kyberdrugs,* as defined herein.

The mixture isolated in Examples 14-17 exhibit the utilities of the *Kyberdrugs* described herein.

### EXAMPLE 18

78 patients in a multicenter study who were suffering either from cough and cold or sinusitis and infectious pulmonary systems were treated with *Kyberdrug,* isolated therefrom using the procedure described herein. The *Kyberdrugs* daily under the supervision of a medical doctor for four weeks. They all received the same dosage regime, either a daily dose of 100 ug/ml parenterally or 200-300 ug/mL subcutaneously or 1000 ug/ml.

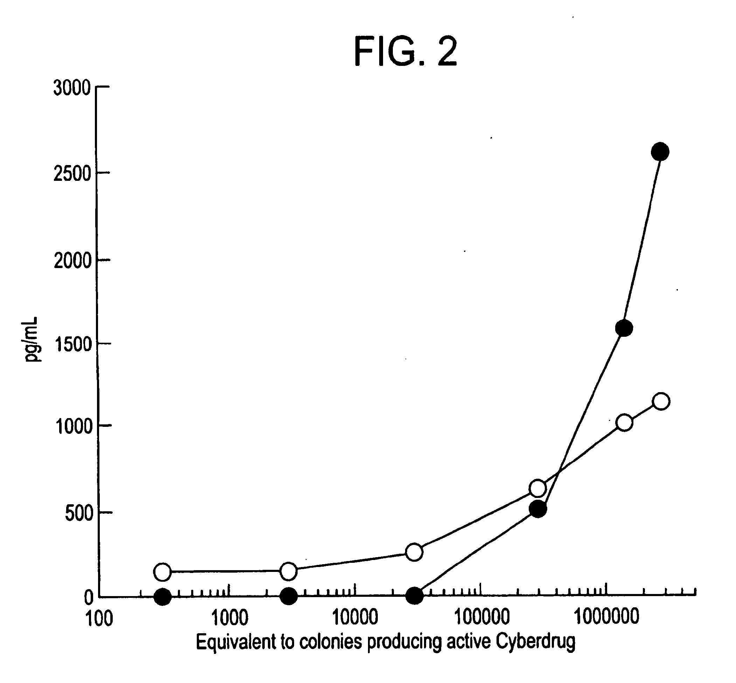

All patients recovered significantly and individually without increasing the dose. In addition, eight different cytokines were analyzed, and their changes during the period of therapy were monitored before, during and at the end of the therapy. In addition, these cytokines were monitored in patients with light symptoms of the above mentioned infections who were not receiving *Kyberdrug.* Particularly, the observed changes in concentration of cytokines was determined before, during and after the treatment with *Kyberdrug.* Fig. 2 shows the relative changes in concentration of interleukin-10β in patients which were not treated with *Kyberdrug* and those treated with *Kyberdrug.* In the graph, the line indicated by (0-0) are those corresponding to the untreated patient and the line indicated by (● 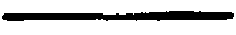 ●) are those treated with *Kyberdrug* for 4 weeks. It is clearly seen that the number of colonies per volume before and after the therapy increases rapidly between 10⁵ to 10⁶ colonies per mL, and the concentration of.interleukin-10 in the patients' serum raises from 250 pg/mL up to 1,500 and higher in pg/mL. This is consistent with an exponential increase of interleukin-10 through stimulation of the *Kyberdrug* by a magnitude of almost 100 µg/mL which is equivalent to 10⁵ cells per mL, indicating a very high therapeutic index and stimulation index. This is also confirmed through *in vitro* and in-vivo experiments using human blood specimens under the same assay conditions.

Bioassays for IL-1β and IL-10 were performed as ex-in vitro test using patient's serum. Bioassays for IL-1α and β commonly utilize the ability of these cytokines to induce IL-2 production by T-cell lines such as EL4.6.1 and LBRM, or in primary cultures of thymocytes (LAF assay). A typical assay system is shown in the protocol 1 and protocol 2, respectively attached hereto in the appendix. These assay systems are very close to the protocols published by M. Wadhwa, C. Bird, L. Page, A. Mire-Sluis and R. Thorpe, in "Cytokines, A Practical Approach", 2nd ed., edited by F.R. Balkwill, IRL-Press at Oxford University Press, 1995, pp. 358-361. The protocol for IL-10 is essentially the same as offered by M.Wadhwa, et al. in the same book at pages 368-369.

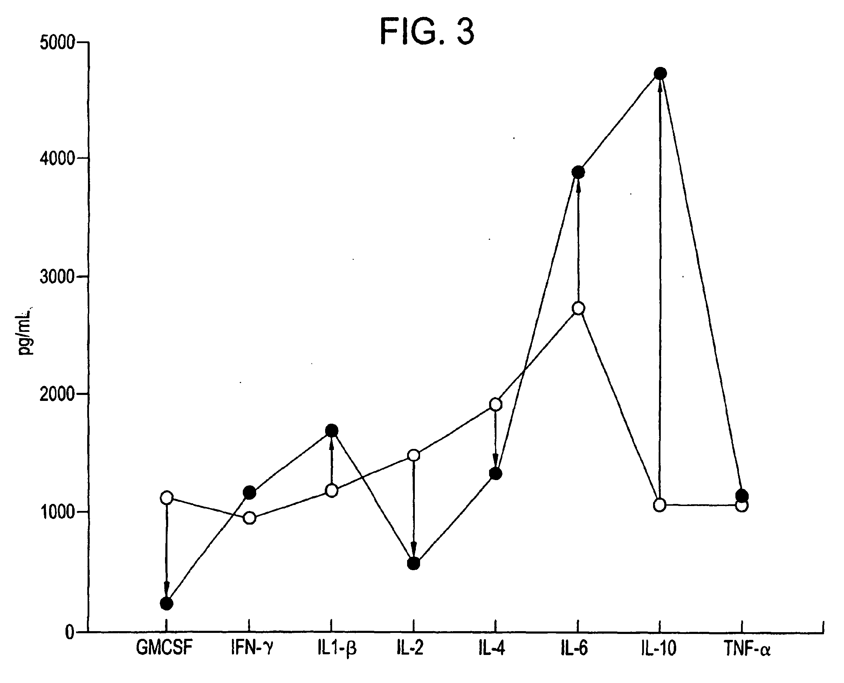

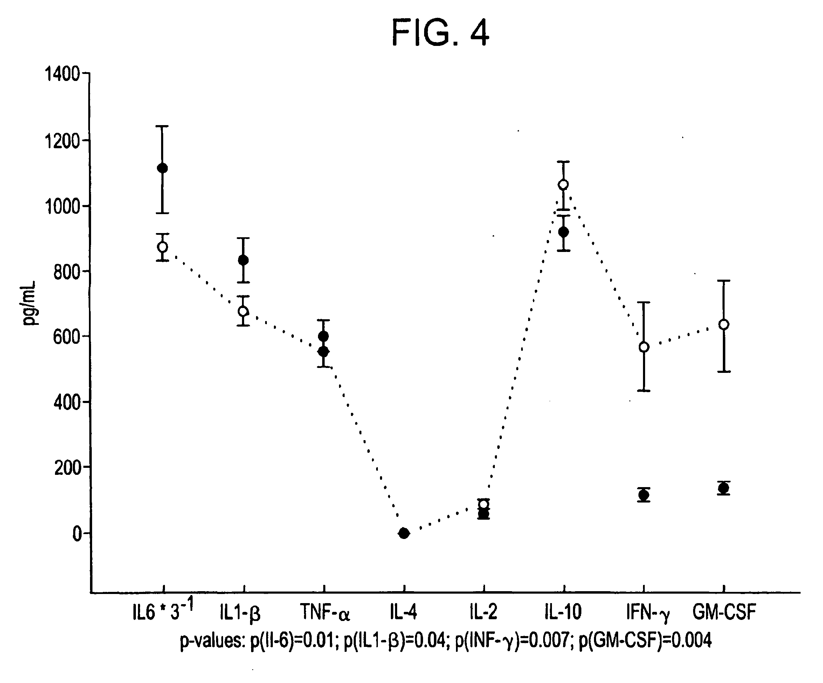

Fig. 3 shows a significant decrease of interleukin-1β within the same colony population with respect to numbers of *Kyberdrug* before and after therapy with the autovaccines. The line (0-0) is the concentration of interleukin B before treatment, while (● 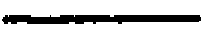 ●) represents the concentration thereof after treatment. A significant exponential decrease of the relative production of interleukin-1β was observed in the serum of patients treated with the *Kyberdrug,* which is invariant to the increase of interleukin-10. The differences or the modulation of the different cytokine biosynthesis under the treatment with *Kyberdrug* within this dosage regime with respect to these groups of patients is shown in Fig. 4., revealing also the changes in numbers and absolute values before therapy and after therapy, respectively. In the experiment described in Figure 4, 275 patients suffering from sinusitis were treated with 100 ug subcutaneously. The change in the interleukines are graphically depicted before treatment (● 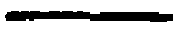 ●) and after treatment (-) for 4 weeks. As clearly shown, the various concentrations of interleukines changed before and after therapy. This was also confirmed through *in-vitro* studies in human blood samples by determining the increase of interleukin-10 after administration of *Kyberdrug;* it showed high stimulation index of more than 14 at 2.0 micrograms/ml of *Kyberdrug,* which is a magnitude lower than the usual LPS as a reference sample. These clinical examples verify the usefulness of the *Kyberdrug* in the treatment of various diseases, e.g. cough and cold, bronchitis or sinusitis as well as rheumatism, where similar factors were modulated or stimulated through the *Kyberdrug* by exerting their inhibitory actions during the infectious pathway, or exacerbation of osteoarthritis, which is followed by a relief of symptoms caused by the disease and welcomed by the patients.

For instance, patients suffering from osteoarthritis (N=14, average age 55), who received a daily dose of 100 µg of *Kyberdrug* subcutaneously (in the morning, after breakfast), i.e., over a period of four weeks reported a relief of symptoms, particularly in the joints and a significant pain relief. As a result, a significant reduction in nonsteroidal anti-inflammatory medicament e.g., normally in the amount of 50-60% in case of ibuprofen or S-(+)-naproxen, of the daily dose were observed. In addition, the exacerbation of this form of rheumatism was significantly reduced, which could be strongly correlated with the measured interleukins and cytokines by clinical blood tests from these patients with the tests used above. The *ex-in* vitro tests of these patients treated with *Kyberdrug* reveal normally all the same pattern as shown in Figure 3.

The *Kyberdrug* causes changes in the concentrations of various cytokines, e.g., enzymes such as protease inhibitors. This same change in interleukin concentrations has been seen in patients who have been suffering from rheumatic diseases or fractures, contusions, chronic rheumatic pain, osteoarthritis, spondylitis, fibrositis, neuritis or infectious diseases caused by streptobacteria or staphylobacteria and treated with *Kyberdrug.*

Without wishing to be bound, it is believed that *Kyberdrug* influences the transformational properties of cells and their agglutininability through the stimulation of the protease. It is believed that the administration of *Kyberdrug* induces proteolysis of previously unmasked and inaccessible membrane lectin-binding sites, which is the reverse process of action of the protease inhibitory system. On the other hand, the protease inhibitors systems induce lectin agglutinability in normal cells. Moreover, they are associated with the agglutination of transformed cells (tumor), and mimic the effects of exogenous plant lectins on the control of cell growth. When the *Kyberdrug* is administered in vitro to the cells, the cells become nonagglutinable and cell division is considerably retarded.

It has also been observed that the administration of *Kyberdrug* results in the loss of normal restriction of cell mobility that accompanies cell transformation and protease treatment. Fibroblast migration into wounds is a plasmin-dependent process, which is suppressed by protease inhibitors. When *Kyberdrug* is administered, fibroblasts have the capacity to ingest particles and display Fc receptors. They, therefore, appear to be susceptible to immune complex activation of protease production. Polymorphonuclear leukocyte chemotaxis, exocytosis, phagocytosis, and superoxide anion generation are typically protease-dependent processes, and hence the influence of the *Kyberdrug* can be studied, including lab-tests of patients suffering from chronic rheumatism. It has been postulated that the superoxide, particularly the HOO⁻ anion (H.H.Paradies et al., Apotheker-zeitung, 126, 477-483, 1985; H.H.Paradies et al., J. Eur.Med. Chem., 25, 143-156, 1990; H.H.Paradies &K.E. Schulte, Ann. New York Acad. Sci., Vol. 529, 221-228, 1988) has been found to be one of the main factors in the pathogenesis of inflammatory arthritis and is responsible for the reduced response of rheumatoid synovial fluid lymphocytes to plant mitogens or nonsteroidal antirheumatic drugs. T cells are more sensitive to the effects of the superoxide anions and HOO⁻ than the B cells. However, when effective amounts of *Kyberdrug* are administered the i.) superoxide and HOO⁻ anions are present in negligible concentrations, ii.) surprisingly the concanavalin A responsive lymphocyte (suppresser cell) is no more sensitive than when treated with concanavalin A alone, iii.) superoxide dismutase (SOD), which is present in negligible amounts in synovial fluid, particularly in patients suffering from inflammatory arthritis, does not inhibit the suppresser effect of the superoxide anions on lymphocyte function, but it does surprisingly during the treatment with *Kyberdrug*.

Furthermore, surprisingly it has been found that *Kyberdrug* has a certain auto-esterase activity which is specifically regulated through the stimulation of interferon and interleukins; when released during an inflammatory process these interferons and interleukins enhance, in addition to the esterase activity, both natural and interferon-stimulated killer cell activity due to a surface active manner on part of the *Kyberdrug.*

Thus *Kyberdrugs* inhibit the aggregation of IgE molecules on the mast cell surface and hence reduce the release of histamine, serotonin, heparin, proteases and further inhibits the release of slow reacting release substances of anaphylaxis e.g. leukotrienes and prostaglandins. *Kyberdrugs* are able to regulate according to this mechanism, the occurrence of C-reactive protein in serum of patients with chronic inflammation.

Circulating proteases which are mitogenic for fibroblasts and those which are characteristic for chronic inflammatory processes have been noted in patients with osteoarthritis and scleroderma. This effect is regulated by *Kyberdrug* particularly in severe cases of osteoarthritis. It is believed, without wishing to be bound, that this is attributable to its unique molecular conformation. The *Kyberdrugs* may be regarded as a substance that is capable of inactivating cytotoxic agents present in the serum of the patients, and thus preventing endothelial cells from being attacked by these cytotoxic endogenously produced substances. Without wishing to be bound, it is believed that a possible role for the stimulation of the proteases through the *Kyberdrug* in the acute inflammatory reaction is the infiltrate response to dermal thiol proteinase injection and the protease-induced increase in chemotaxis and exocytosis as well as superoxide or HOO⁻ anion generation. It is believed, without wishing to be bound, that the infiltrate response is due to the size, charge and shape dynamics of the *Kyberdrug,* while the increase of the chemotaxis as well as the increase in enzymatic activity of the protease is also mainly due to the *Kyberdrug.* Inflammatory synovium contains increased elastase activity that is capable of cartilage degradation, which is significantly decreased or abolished under treatment with *Kyberdrug.* Chondrocyte-induced cartilage destruction by collagenase and proteoglucanese which are metalloproteins is "down-regulated" by the *Kyberdrug* due to enzyme inhibition.

Without wishing to be bound, it is believed that the *Kyberdrug* regulates inhibitory protease activities endogenously by a decreased cytotoxic lymphocyte activity, including both antibody-dependent cell-mediated cytotoxicity and natural killer cell activity. Furthermore, the action of the *Kyberdrug* as inhibitor of proteases in enhancing macrophage inhibitory factor activity enhances also macrophage surface adherence, phagocytosis, and tumor cytotoxicity. Moreover, the polymorphonuclear leukocyte chemotaxis, phagocytosis, degranulation, and superoxide or HOO⁻ generation are surprisingly *Kyberdrug*-sensitive processes. Thus diseases caused by these radicals can be treated with the *Kyberdrug.* The *Kyberdrug* also reduces endogenously and stimuli-related superoxide and HOO⁻ anions generated by alveolar macrophages, peripheral blood mononuclear cells, polymorphonuclear leukocytes, and basophils. The *Kyberdrug* exhibits also sialoprotein-related antigenicity as noticed from in vitro and in vivo studies of patients suffering from cold & rhinitis, respectively, as determined from *in vitro* studies applying the hemagglutenin test, which measures the competition of the *Kyberdrug* with the cell surface sialyl-oligosaccharides for viral hemagglutenin binding, and the infectivity test, which measures the reduction of plaque reduction in MDCK cells (Madin-Carby Canine Kidney) after or prior to infection with influenza A virus. Inhibition of influenza A virus plaque test assays were performed according to K. Tobita, et al., Med. Microbiol. Immunol., 1975, 162, 9-14; Hayden, F.G., Cote, K.M., Douglas, R.G., Jr., Antimicrob. Ag. Chemoth., 1980, 17, 865-870. Specifically, MDCK cells were inoculated with influenza A/PR/8/34 (ATCC, Rockville) and diluted in Eagle's minimal medium, pH 7.3-7.5, containing 4 µg/mL trypsin to yield approximately 50 plaques per well. The cells were left for 1 h at 25°C for the virus to absorb, subsequently overlaid with cell growth medium (DCCM-1, Boehringer Mannheim, FRG) containing 1% agarose, 2 µg/mL trypsin, 0.001% DEAE-dextran (Pharmacia), and the amount of *Kyberdrug* to be tested. After 72h at 32°C, plaques were visualized by fixing with 2.5% glutaraldehyde followed by staining with carbol fuchsin. The percentage inhibition of plaque formed in the absence of any *Kyberdrug*, were calculated for each inhibitory *Kyberdrug* concentration. The mean % inhibition values from these experiments in triplicate were used to estimate the inhibitory concentration at 50%. Depending on the presence of specific counterions, e.g., Ca²⁺ or Mg²⁺, respectively the IC₅₀ were found to be in the range of 10 µg/mL to 50 µg/mL *Kyberdrug.* The hemagglutenin-inhibition test (a standard test) was performed according to G.N. Rogers, T. Pritchett, J.L. Lane and J.C. Paulson, Virology, 1983, 131, 394-408. The IC₅₀ values for Kyberdrug were in the range of 2.0 µg/mL to 0.2 µg/mL, depending on the addition or absence of Zn gluconate (0.001%), respectively.

α-2 macroglobulin is also a pivotal protease inhibitor which is regulated by the *Kyberdrug.* α-2 macroglobulin functions as a carrier protein in transfer of proteases from other inhibitors such as the α-1-antitrypsin protease inhibitor, and has a protective function against the activity of other more specific inhibitors e.g. protecting plasmin from antithrombin III neutralization, plasmin-sensitive surface-associated fibronectin molecules on fibroblasts from degradation, and/or papain destruction of cartilage (D.A.Lewis, "Endogenous anti-inflammatory proteins", Biochem. Pharmacol., 26, 693, 1977) Since α-2-macroglobulin is an important mechanism for clearance of immune complexes in joint fluid, the subsequent clearance of such complexes by macrophages with any enhancement of the inflammatory processes is believed to be through the action of macrophage activation and plasminogen release. Although the spectrum of α-2- macroglobulin has some similarities to that of the α-1-antitrypsin protease inhibitor, it is surprising that reactions with trypsin are considerably stimulated through the *Kyberdrug* in a much faster way than with the α-1-antitrypsin protease inhibitor, and are unaffected by heparin, but are affected strongly by the *Kyberdrug.* This is in contrast to the heparin inhibition of α-2-macroglobulin-thrombin complex formation since *Kyberdrugs* do not bind to thrombin, but heparin does. α-2- macroglobulin is localized on endothelial surfaces and can regulate activities of adherent cells and reduce inflammatory response. Since the *Kyberdrug* stimulates the α-2- macroglobulin, the inhibition of the carrageenan, histamine, prostaglandin E₂, serotonin, and bradykinin induction in inflammation can be reconciled in conjunction with the very sensitive parameters measured before. The relative proportions of free and complexed α-2 macroglobulin with the *Kyberdrug* determines the biologic effects which have been observed in patients with inflammatory diseases. Furthermore, the stimulation of α-2 macroglobulin through the *Kyberdrug* derived from patients with rheumatoid arthritis is shown through the activity of polyclonal B cell activator. α-2- macroglobulin from healthy individuals does not manifest this activity, but patients with rheumatoid arthritis do, so it can be reconciled that the *Kyberdrug* is responsible for the polyclonal B cell activation, and the α-2- macroglobulin-proteinase complexes for macrophage activation.

Furthermore, inherent α-2-macroglobulin antigenic determinants when stimulated by the *Kyberdrug* are also responsible for the ability of the *Kyberdrug* to diminish the survival of bacteria in humans. The organism's mechanism due to the support of the *Kyberdrug* has to be an important factor since it must produce excess proteases to overcome blood inhibitors. The presence of a "blind" intestine necessitates reflux of the excess proteases which can produce additional α-2-macroglobulin complexes as they diffuse from the infected site. The immunsuppressive effect of such complexes in the absence of *Kyberdrug* is a factor in persistent infestation, and provides a continuous incident of inflammation and infection, respectively.

Moreover, the importance of free α-2-macroglobulin is reflected in the observation that saturation of α-2- macroglobulin in the circulation or abdominal cavity is normally followed by shock and death which can be prevented through the administration of these *Kyberdrugs.* Furthermore, the degree of saturation of this particular inhibitor seems to be a life threatening determinant, so the sensitive balance is introduced through the administration of the *Kyberdrug.* Accordingly only about 15 µg of trypsin is required per milligram of α-2 macroglobulin for the *Kyberdrug* to be fully complexed. The *Kyberdrug* itself as well as the complexes comprising *Kyberdrug* and α-2 macroglobulin have a key role in preventing autodigestion including the protection of the gastric and intestinal mucus.

Deficiencies of α-2- macroglobulin has been found in patients with respiratory distress syndrome, sepsis including consumptive coagulopathy, regional enteritis, multiple myeloma, in small-for-gestational-age infants, or under streptokinase therapy. Moreover, α-2 macroglobulin levels are also depressed when plasmin is generated in vivo but do not become depressed in thrombotic states, or in the presence of *Kyberdrug,* respectively. The presence of α-2-macroglobulin in the lung, normally lower than 25% than that found in the serum, explains the lack of protective effect of α-2- macroglobulin in the early onset of emphysema, which can now been avoided through the administration of *Kyberdrug.* Depressed levels as being observed in respiratory distress syndrome, and the failure of patients with α-1-antitrypsin deficiency to develop emphysema suggest that the role of α-2 macroglobulin is not inconsequential including the regulatory effect of the *Kyberdrug.*

Serum levels of α-2 -macroglobulin have been reported to be almost normal in patients with rheumatoid arthritis, even when serum α-2-globulin levels are elevated (J.R. Ladd & J.T. Cassidy, " Serum and synovial fluid concentrations of α-2-macroglobulin in patients with rheumatoid arthritis", Arthritis Rheum., 12, 309, 1969). In addition a significant proportion of α-2- macroglobulin in synovial fluid of patients with rheumatoid arthritis or degenerative arthritis has been found to be functionally inactive due to binding of synovial fluid enzymes such as collagenase and cathepsin, which can be shown to be reversed in the presence of *Kyberdrug.*

*Kyberdrugs* also suppress inflammatory arthritis. In inflammatory arthritis, α-2 macroglobulin inhibits synovial collagenase which degrades the collagen triple helix, cathepsin D degrades proteoglycans, and cathepsin A degrades both. These degradation processes are significantly inhibited in patients with rheumatoid arthritis after administration of *Kyberdrug.* Furthermore, the role and the modulation of the α-2 macroglobulin in limiting autodigestion of endothelial surfaces which occurs in the presence of free plasmin are significantly reduced in the presence of *Kyberdrug,* and is of importance of the vasculature in the pathogenesis of collagen vascular disease.

Another factor being modulated through *Kyberdrug* is antithrombin III, which is an inhibitor of a number of enzymes that, along with their other functions, modulate fibrinolysis and complement systems, which is important in this context of the invention. The more direct immunomodulation has been observed for antithrombin III together with *Kyberdrug* in the course of inhibition of cell division and mitogen-induced T-cell proliferation possibly reflecting inhibition of thrombin. Antithrombin III, when stimulated by the *Kyberdrug,* enhances the stimulation of the macrophage activation factor and in response thereafter the macrophage migration inhibitory factor.

### Inhibitory effects of Kyberdrug on Retroviruses.

Another unexpected effect of the *Kyberdrug* which has been observed is the inhibitory action in lymphocyte cell cultures which has been infected with the human immunodeficiency viruses type 1 & 2 (HIV 1 & 2) from blood of an AIDS patient, and which has been determined through assay systems for virus production (load) and infectivity. The course of the infection was monitored by i.) the amount of HIV particles by quantitatively assaying virion-associated protein directly, e.g. p24 antigen capture or indirectly through the reverse transcriptase activity (RT), and the gp120 glycoprotein; ii.) particle infectivity using TCID₅₀ (tissue culture infectious dose, half-maximal) determinations, and the syncytium formation (SCF) assay. The advantages of using the p24 antigen capture assay, the RT-assay as well as the gp120 protein assay is related to the sensitivity and quantitation possibility in order to assess quantitatively the changes of particle associated proteins including their concentrations vs. concentration of the *Kyberdrug.* The RT-assay is known to be less sensitive than the p24 antigen capture assay, but found to be as sensitive as the gp120 assay. The assay systems are those as described in the scientific literature and regarded as the state of art, specifically in "HIV, Vols. 1&2, A Practical Approach, Virology and Immunology", edited by J. Karn, PAS-Series, IRC-Press, Oxford University Press, 1995**,** and as described in the German Patent **DE #196 32 823.3** by Zimmermann & Paradies (**1997**). In addition a modified anti-HIV assay system was also used with application of the MTT method introduced by R. Pauvels et al., J. Virol. Methods, 20, 309, 1988. MT-4 cells which is a human T4-positive cell line carrying human T-lymphotropic virus type 1 were infected with HIV-1_{HTLV-IIIB} at the multiplicity of 0.01, and HIV-1 and mock-infected MT-4 cells were incubated in the presence of various amounts of *Kyberdrug* (µg/mL) for 4 days at 37°C in a CO₂ incubator. The viability of both HIV-1 and mock-infected MT-4 cells was assayed spectrophotometrically via the reduction of 3-(4,5-dimethylthiazol-2-yl)-2,5-diphenyltetrazolium (MTT). The anti-HIV activity is represented as the IC₅₀ which denotes the concentration needed for the inhibition of 50% infection of MT-4 cells from HIV. The cytotoxicity concentration CC₅₀ was determined by the 50% cytotoxic concentration of the test material on the MT-4 cell. A modified RT assay system has been used on the basis of these infected lymphocytes. Furthermore, in order to show that the *Kyberdrug* are devoid of anticoagulant activity, this activity was evaluated using bovine plasma in accordance with the modified procedure described in (K.Hatanaka et al., J. Med Chem., 30, 810, 1987, using dextran sulfate as a standard having an anticoagulant activity of 25 units/mg as a reference.

The HIV-envelope glycoprotein, gp120, has a distinct secondary structure consisting of six α-helices in which four of the six α-helices, i.e. α₁, α₄, α₅ and α₆ were found in the conserved C₁, C₄ and C₅ regions, and two other helices, i.e. α₂, and α₃ were found in the V₂ region and pseudo-conserved C₃ regions (J-F.Hansen et al., Proteins, 25, 1, 1996; L.Ratner et al., Nature 313, 277, 1985). In light of the results obtained for the *Kyberdrug* with regard to the protease inhibitory and stimulating effects in patients receiving *Kyberdrug* either as oral forms or parental, it is believed without wishing to be bound, that the *Kyberdrugs* block the action of the HIV-specific protease enzyme during viral replication. Protease cuts the viral proteins which is formed from the viral genetic material into shorter chains. This is essential for successfully assembling new viral particles in the host cells. Therefore, protease inhibitors attack the HIV particle at a later stage in its replication cycle than the reverse transcriptase inhibitor which prevents virus replication of its genetic material once it has entered the host cell.

Without wishing to be bound it is believed that the *Kyberdrug* acts as an non-competitive inhibitor with a Kₘ value or 500 µg and k_{cat} = 20 min⁻¹ and decreases without any significant influence on the value of Kₘ. However, a significant reduction in the number of HIV particles and a decrease in the concentration of gp120 and gp24 were observed, respectively, when treated with *Kyberdrug* at much lower concentrations than applied for in the RT-assay. These concentrations, however, do vary with the pH, revealing values of 100 µg at pH 6.5 (37°C) versus 20 µg at pH 7.8 (37°C).

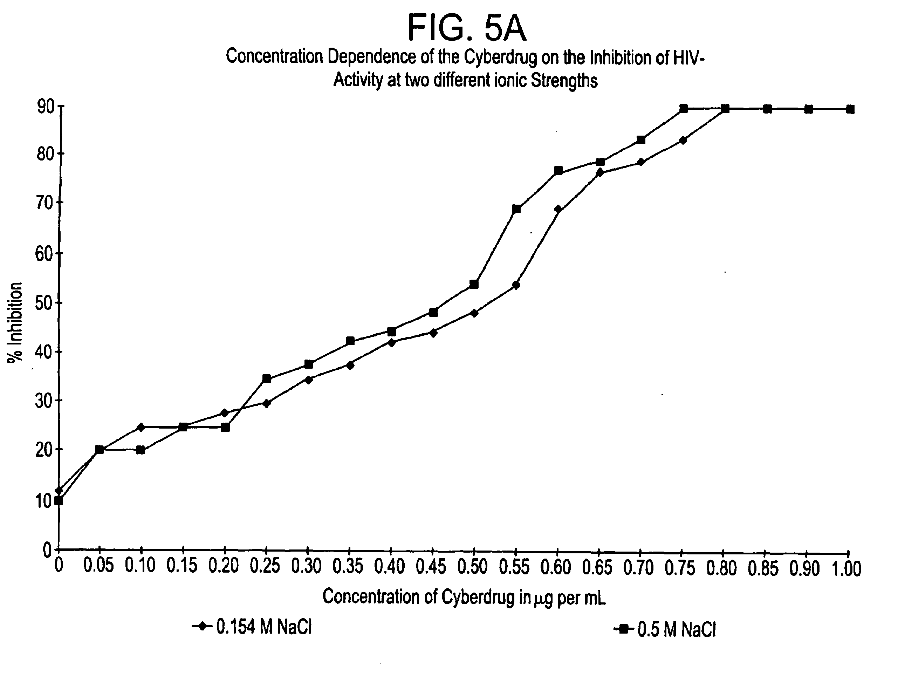

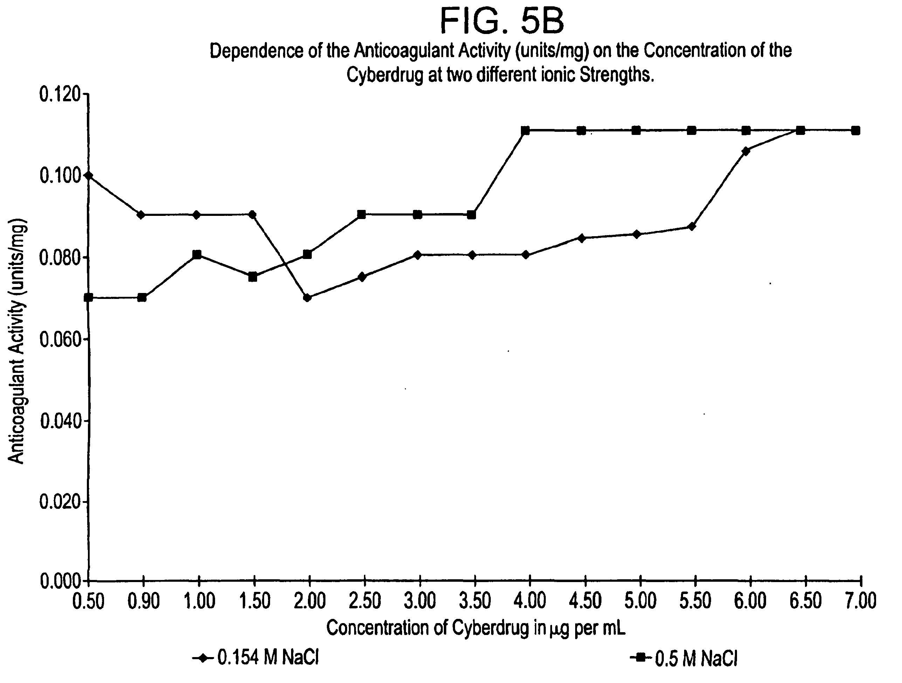

The anti-HIV activity of the *Kyberdrug* was assayed by the MTT method using the MT-4 cell line and the HIV_{HTLV-IIIB} strain to give the EC₅₀ value, which is the concentration effective for 50% inhibition of the virus infection to MT-4 cells by the *Kyberdrug.* In spite of the hydrodynamically large size of the *Kyberdrug* in contrast to drugs with low molecular weights administered to patients suffering from HIV infections, all preparations of the *Kyberdrug* exhibited anti-HIV activities represented by low EC₅₀ values ranging from 0.4 to 0.6 µg per mL when administered to the cell cultures in 0.154 M NaCl. pH changes or different ionic strength did not change the EC₅₀ values significantly. Furthermore, the cytotoxicity of the *Kyberdrug* as obtained through the CC₅₀ value were greater than 800 µg per mL and no changes have been observed in the CC₅₀ values above 1000 µg per mL which is above the amount. The inventors assessed also the dependence of the anticoagulant activity of the *Kyberdrug* in this concentration range by the activated partial thromboplastin time using heparin as a reference, or dextran sulfate as a standard reference according to the United States Pharmacopoeia. They determined no anticoagulant activities at all concentrations of *Kyberdrug* applied, which is very different from those reported for sulfated polysaccharides having also anti-HIV activities (Fig. 5).

The above preferred embodiments and examples are given to illustrate the scope of the present invention. The embodiments and examples described herein will make apparent to those skilled in the art other embodiments and examples. These other embodiments and examples are within the contemplation of the present invention. Therefore, the present invention should be limited only by the appended claims.

### APPENDIX

### Protocol 1. Bioassay of IL-2 using CTLL cell-line

### Equipment and regents

- CTLL cell culture
- RPMI 1640 medium
- RPMI 1640 medium containing 10% FCS
- Centrifuge (MSE-benchtop)
- Trypan blue
- IL-2 standard
- Test samples
- 96-well microtitre plates
- 37°C, 5% CO₂, humidified incubator
- [³H] thymidine (25 Ci/m Mol, 5mCl/5mls)
- Filter mats
- Liquid scintillation counter system

### Method

1. Wash CTLL cells (3 days after feeding) three times with RPMI 1640 by centrifuging the cells at 250 g for 10 min.
2. Determine viability of the cells, e.g., by Trypan blue dye exclusion^{a} and resuspend cells to a final concentration of 1 x 10⁵ cells/ml in RPMI 1640 medium containing 10% FCS.
   ^{a} Cells should be >80% viable.
3. Titrate the IL-2 standard in triplicate in 96-well microtitre plates. Start the titration at 40 IU/ml IL-2 and then make serial two-fold dilutions down to 0.019 IU/ml IL-2. Prepare dilutions of the samples in triplicate. Include a negative control, i.e., culture medium alone. Each well should contain a volume of 50 µl.
4. Add 50 µl of the cell suspension to each well and incubate the plates for 18 h at 37°C in a humidified CO₂ incubator.
5. Add 0.5 µCi of tritiated thymidine to each well and return the plates to the incubator for approximately 4h.
6. Harvest the contents of each well on to filter mats and determine the radioactivity by liquid scintillation counting.
7. Plot a standard curve of c.p.m versus concentration of IL-2. For quantitation of activity in unknown samples, compare test results with standard curve.

### Protocol 2. Bioassay of IL-1^{a} using EL4/NOB-1 cell line^{b}

### Equipment and reagents

- EL4/NOB-1 cell culture
- RPMI 1640 medium containing 5% FCS
- IL-1 Standard
- Test samples
- Plus equipment and reagents as Protocol 1
^{a} IL-1n and β have equal sensitivity in this assay.
^{b} The EL4/NOB-1 cell line also responds to murine TNFα.

### Method

1. Wash EL4⁻/NOB-1 cells (2 to 3 days after feeding) twice in RPMI 1630 medium by centrifuging the cells at 250 g for 10 min and determine the viability as in step (2) of Protocol 1.
2. Resuspend the cells to a final concentration of 5 x 10⁵ cells/ml in RPMI 1640 medium containing 5% FCS.
3. Distribute titrations of an IL-1 standard in triplicate in 96-well microtitration plates. Start the titration of the standard at 100 pg/ml IL-1 (10 IU/ml) and make serial two-fold dilutions down to 0.09 pg/ml IL-1 (0.009.IU/ml) . Make appropriate dilutions of the samples to be measured for IL-1 activity (either two-fold or ten-fold serial dilutions) in triplicate. The negative control is culture medium. Each well should contain a volume of 100 µl at this stage.
4. Add 100 µl of the washed cell suspension to each well and incubate the plates for approximately 24 h at 37°C in a humidified CO₂ incubator.
5. Remove 50 µl of the supernatant from each well and determine the IL-2 present using the CTLL-2 bioassay^{c} (see Protocol 1). The amount of IL-2 in the supernatants will be proportional to the amount of IL-1 in the original samples. Supernatants from the EL4/NOB-1 cells can be removed and stored frozen until the IL-2 can be conveniently assayed.
   ^{c} Since the NOB-1 bioassay uses the CTLL-2 bioassay as a second stage, IL-2 and mIL-4 could interfere if present in the samples being tested for IL-1 activity. This can be overcome by pre-incubating the samples with the EL4/NOB-1 cell line for 4-5 h followed by thorough washing of the cells prior to steps 4 and 5.

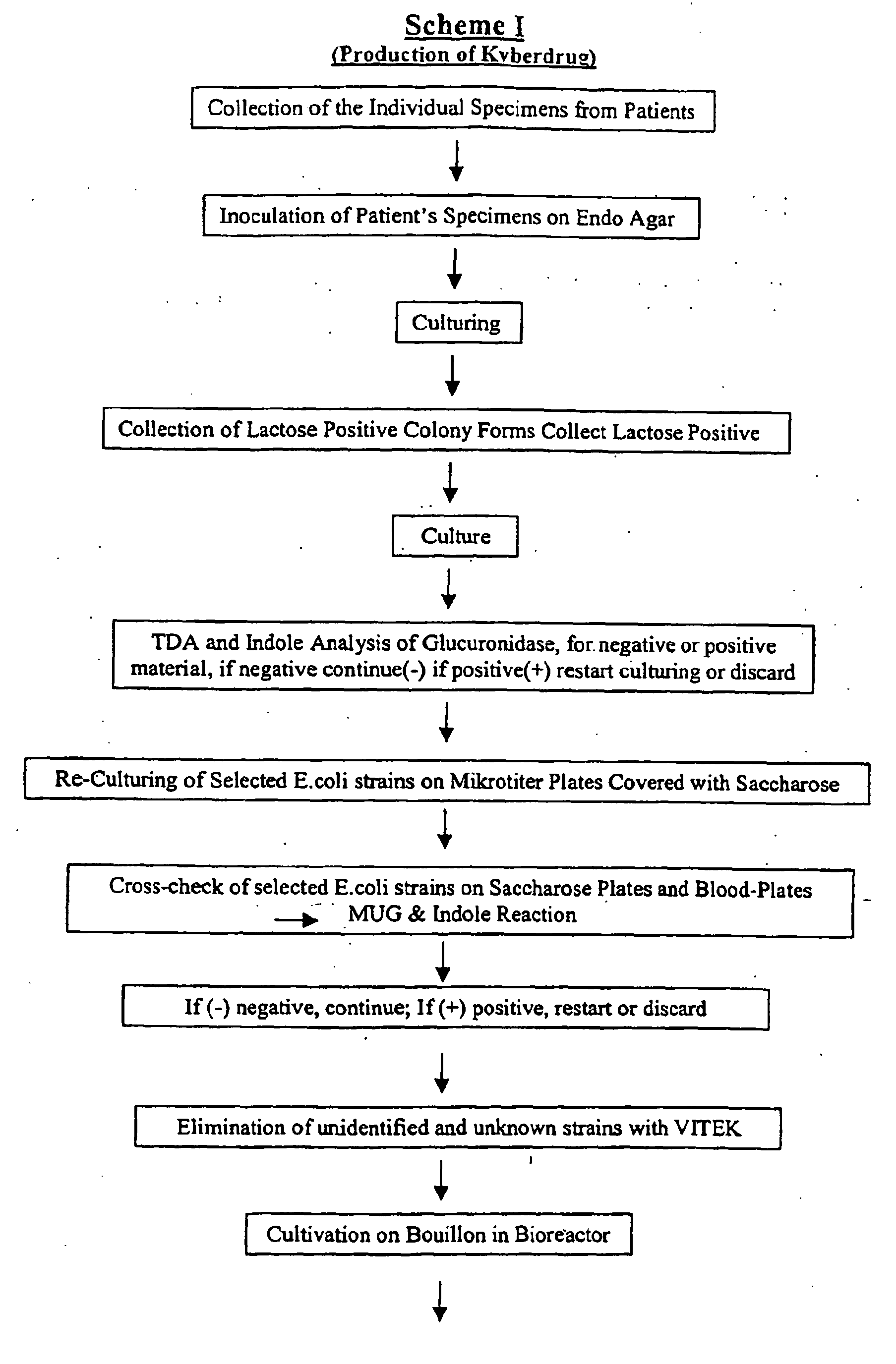

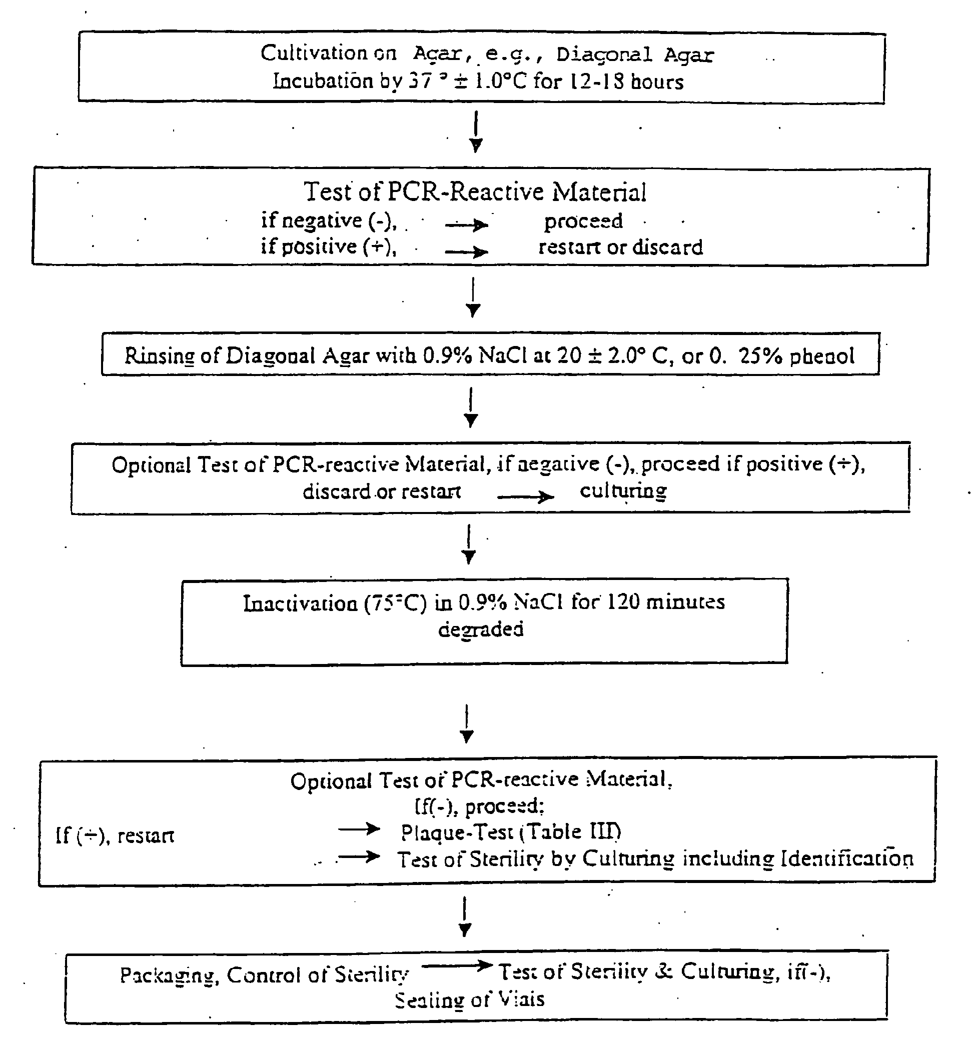

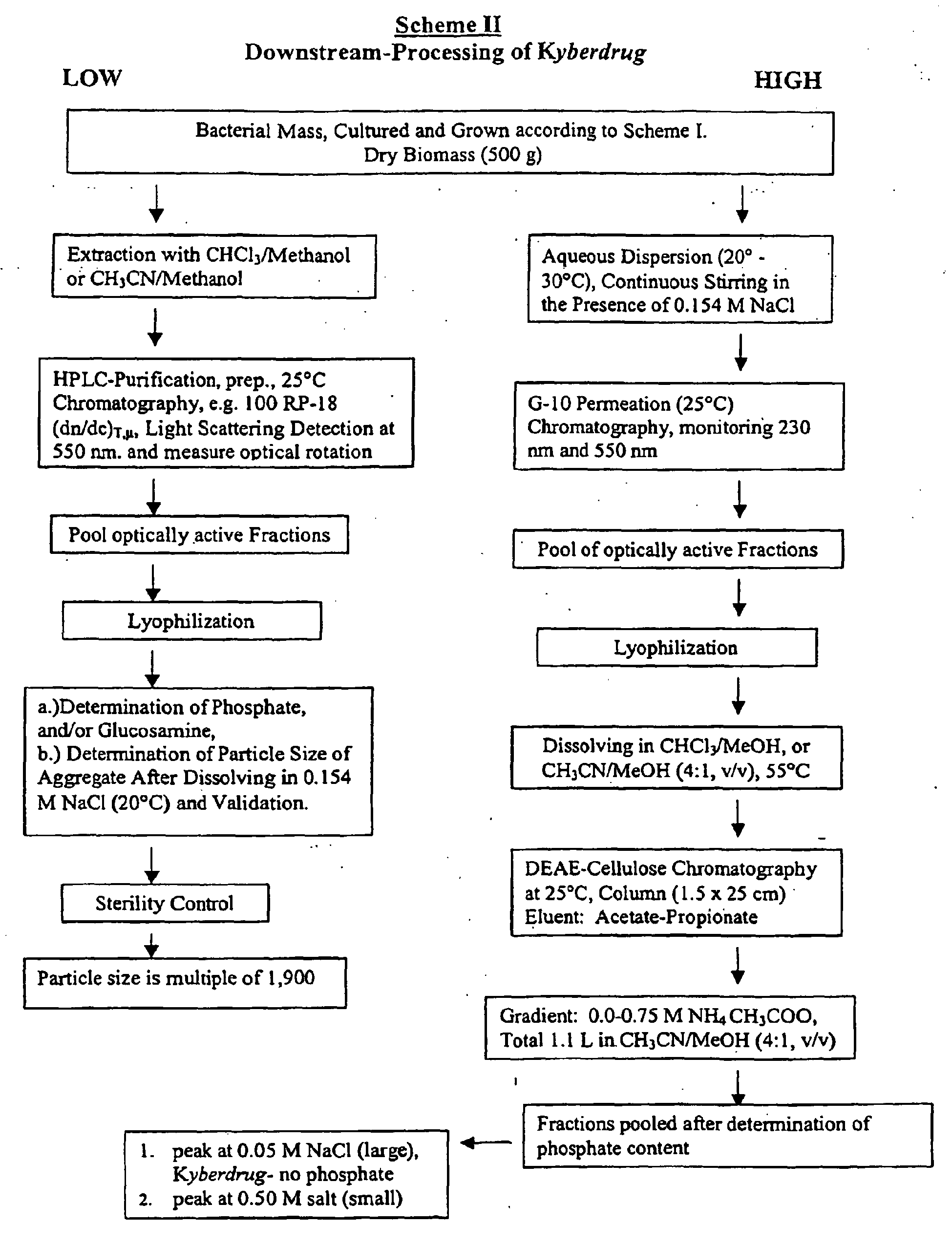

**TABLE I**

| Growth and Selection Media, formulated for the specific Microorganisms | |
|---|---|
| A. | |
| Peptone | 10.0g |
| D-(÷)-Glucose | 40.0 g |
| Agar-Agar | 30.0 g |
| KH₂PO₄ | 4.0 g |
| Na₂HPO₄ | 6.0 g |
| Distilled Water | 1.0 L |
| pH, or adjusted to | 7.0-7.5 |

| B. | |
|---|---|
| Peptone | 5.0 g |
| Yeast Extract | 2.5 g |
| D-(÷)-Glucose | 1.25 g |
| Maltose | 1.25 g |
| L-(-)-Cysteine | 0.125 g |
| Salts Solution | 10.0 mL |
| Reazurin (0.0025% aq. sol.) | 1.0 mL |
| Distilled Water | 250 mL |

| C. | |
|---|---|
| Salt Solution, Composition of 3 | |
| KH₂PO₄ | 0.10 g |
| KH₂PO₄ | 0.10 g |
| NaHCO₂ | 1.0 g |
| NaCl | 1.0 g |
| CaCl₂ (anhydrous) | 0.20 g |
| MgSO₄ | 0.02 g |
| Na₂MO₄ • 2 H₂O | 0.02 · 0.05 µg |
| CoCl₂ • 6 H₂O | 5.0 µg |
| H₂SO₄ (50%) | 0.3 mL |
| Distilled Water | 100 mL |
| pH (37°C) | 6.8 to 7.8 |

| D. | |
|---|---|
| Agar-Agar | |
| Soybean Agar | total of 100.0 g |
| White Soybean | 100.0 g |
| Grew Soybean | 75.0 g |
| Distilled Water | 1.0 L |
| Soak beans overnight. Autoclave 1 hour at 121°C. Filter broth through cotton. Measure broth and add 1.5% (W/W) Agar. Sterilize. | |

| E. | |
|---|---|
| Yeast-Glucose-Citrate Medium | |
| Glucose | 10.0 g |
| Peptone | 10.0 g |
| Yeast Extract | 5.0 g |
| (-)-S-Adenosylmethionine • H₂PO₄ | 0.125 g |
| Ammonium Citrate | 5.0 g |
| Sodium Acetate | 2-0 g |
| MnSO₄ • 4 H₂O | 0.05 g |
| MnSC₄ • 7 H₂O | 0.25 g |
| Tween 30 | 1.0 g |
| Distilled Water | 1.0 g |
| Adjust to pH | 3.5 |
| F. The same media as listed in E., but in addition add 100 µg mitomycin C, pH 6.5, 37°C. | |
| G. The same media as listed under F., but in addition to 100 µg mitomycin C add 0.150 g L- (-) -methionine, but no s-(+) - adenosylmethionine • H₂PO₄, pH 6.5 at 37°C. | |

**TABLE II**

| **Growth Selection Media Formulated for the Optimized Culture and Production of *Kyberdrug*** | |
|---|---|
| Peptone, Beef | 7.8 g |
| Peptone, Casein | 7.8 g |
| Yeast Extract 2.8 | g |
| NaCl | 5.6 g |
| D-(+)-Glucose | 1.0 g |
| Agar-Agar | 12.0 g |
| Distilled Water | 1.0 L |
| **or** | |
| Peptone, beef | 5.0 g |
| Peptone, casein | 5.0 g |
| Yeast Extract | 3.0 g |
| NaCl | 6.0 g |
| Water soluble Vitamins | 600 µg |
| B₆, B₁, B₁₂ | 250, 150 200 µg |
| L-(-)-Methionine | 0.150 g |
| D-(+)-Glucose | 1.0 g |
| Agar-Agar | 12.0 g |
| Distilled Water | 1.0 L |

**TABLE III**

| **Selective criteria for the Production of *Kyberdrug*** | |
|---|---|
| **1. Identification of Enterobacteriaceae** | |
| E. Coli | |
| Endo Agar | R or S-Forms |
| Preferable Diagonal Agar | |
| MUG Reaction | negative |
| Indole Reaction | negative |
| Colicin Determination | positive |

| **2. Exclusion Criteria and Requirements for Pathological Strains and Factors** | |
|---|---|
| Generation of Hemolysins | negative |
| Formation of Endotoxins | negative |
| Absence of Genes responsible for Verotoxins: | |
| -heat insensitive | no activities |
| -heat labile forms | no activities |
| Absence of activation factors for cAMP Adenylate-cyclase | positive |
| Absence of activation factors for cGMP Guanylate-cyclase | positive |
| Determination of adhesion molecules, e.g., ICAM I-III | negative |
| Absence of eae gene sequences, characteristic for EHEC or EPEC | positive |
| Stability during storage and effective for shelf-life of the col* strain in isotonic salt solution | positive |
| stability during storage and effective for self-life of the col* strain in isotonic solution, but having a positive indole & MUG" reaction | positive |
| No detrimental effects e.g. protease attack on proteins or degradation of glycolipids and lipid A on the selected strain for application in further culturing and harvesting | positive |
| Proven effective at low cell numbers | 10⁴-10⁶ cells/mL |
| Proven effective at low concentrations | 1.0 x 10⁻⁵ g/mL to 5.0 x 10⁻⁶ g/mL |

| | |
|---|---|
| ** MUG=hydroxycoumarin-7-glucoside which is also known as umbelliforme-7-glucoside. PCR = polymerase chain reaction | |

## Claims

1. An isolated substantially pure biological material optionally associated with a pharmaceutically acceptable salt thereof which has the following characteristics:
(a) has a constant hydrodynamic radius of about 0.3 to 0.40 µm, having a low polydispersity index of about 0.05 to about 0.08%;
(b) has an aggregate of monomeric units in saline solution, containing from about 68 to 75 monomers in the aggregate;
(c) the aggregate has a number molecular weight of about 130,000 to 150,000 Dalton;
(d) the monomer has a molecular weight of about 1, 900 to 2,000 Dalton;
(e) contains two sugar amine moieties, wherein the sugar is glucose or galactose, provided one of the sugars is glucose;
(f) contains no pyrophosphate groups;
(g) contains 1,6 β-linkage between the two sugars;
(h) the monomer contains no phosphate group or may contain a phosphate at the 1 position or the 4' position of the sugar; however, the aggregate contains at least 80% by weight a sugar moiety which does not have any phosphate thereon, and at most 20% by weight a sugar moiety having a monophosphate;
(i) contains an amino functionality at the 2 and 2' positions which may form amide bonds with a 3-hydroxytetradecanoic acid;
(j) contains an hydroxy functionality at the 3 and 3' position which may be esterified with hydroxytetradecanoic acid;
(k) contains an even number of 3'-hydroxytetradecanoic acids per monomer; and
(l) has the X-ray diffraction pattern of Fig. 8A at 25°C.

2. A method of preparing a non-toxic biological material comprising:
(a) providing an endotoxin extract derived from Enterobacteriaceae at the situs of infection;
(b) screening and collecting those enterobacteria which produce colicin but which do not convert tryptophan into indole and which do not react in the MUG assay;
(c) harvesting those selected bacteria;
(d) selecting those strains of step (c) which cannot make endotoxins;
(e) killing the strains of step (d).

3. The method according to Claim 2, in which step (e) comprises heating the strains of (d) at sufficient temperatures to denature the protein therein.

4. The method according to Claim 2, in which isolating from the product of step (e) comprises extracting the lipid material exhibiting absorbances at 230 nm and 550 nm.

5. The method according to Claim 4, in which extracting comprises precipitating the lipid material with chloroform/methanol or acetonitrile/methanol solution and purifying the product and lyophilizing same.

6. The method according to Claim 4, in which extracting comprises placing the lipid material in an aqueous saline solution, purifying the crude lipid product by chromatography, collecting those fractions which have a UV absorption at about 230 and 550 nm, lyophilizing the collected fractions and precipitating the desired product with CHCl₃/MeOH or CH₃CN/MeOH and purifying the precipitated product.

7. The method of Claim 4, in which the non-toxic biological material has a monomer molecular weight of about 1900 Dalton and an aggregate molecular weight in saline solution ranging from about 120,000 to 150,000 Dalton.

8. The method of Claim 2, comprising placing the non-toxic biological material in aqueous solution.

9. The method according to Claim 2, wherein the Enterobacteriaceae are aerobic Enterobacteriaceae.

10. The method according to Claim 2, in which the Enterobacteriaceae are in the R form.

11. The method according to Claim 2, in which the Enterobacteriaceae are in the S form.

12. The method according to Claim 11, in which the Enterobacteriaceae belong to the strain selected from the group consisting of Bacillus, Bacteroides, Brucella, Carnobacterium, Caulobacter, Citrobacter, Clostridium, Corynebacterium, Enterobacter, Escherichia coli, Halobacteria, Klebsiella, Lactobacillus, Lactococcus, Leuconstoc, Listeria, Micrococcus, Mycobacterium, Neisseria, Pasteurella, Pediococcus, Propionibacterium, Proteus, Pseudomonas, Salmonella, Sarcina, Shigella, Serratia, Staphylococcus, Streptococcus, and Vibrio.

13. A colloid crystal of the biological material of Claim 1 or of a pharmaceutical composition obtained by the method according to Claim 2.

14. A pharmaceutical composition comprising a pharmaceutically effective amount of the biological material of Claim 1 or a pharmaceutical composition containing a non-toxic biological material obtained by the method according to Claim 2 in association with a pharmaceutical carrier.

15. The pharmaceutical composition according to Claim 14 further comprising calcium, magnesium and zinc salts.

16. The pharmaceutical composition according to Claim 15 in which the salts are ZnCl₂, Zn-D-gluconate, Zn-meglumine, Zn-D-citrate or Zn-salicylate.

17. The pharmaceutical composition according to Claim 14 in the form of a liposome.

18. The pharmaceutical composition of Claim 14 wherein the composition is in a lyophilized form.

19. The pharmaceutical composition according to Claim 14 in the form of an oil drop emulsion.

20. The biological material according to Claim 1 or the biological material obtained by the method according to Claim 2 in a pharmaceutically effective amount for use in a method for treating a disease in a mammal which is afflicted with a viral or bacterial infection.

21. An isolated microorganism having the following attributes:
(a) is an enterobacteria;
(b) produces colicin;
(c) does not form any endotoxin;
(d) does not possess genes responsible for making verotoxins;
(e) does not contain activation factors for CAMP adenylate cyclase;
(f) does not contain activation factors for cGMPM guanylate cyclase;
(g) does not have adhesion molecules;
(h) does not have eaegene sequence;
(i) which does not convert tryptophan into indole and which does not react in the MUG assay;
(j) is rod-like in appearance; and
(k) obtained by the method of
(i) providing an endotoxin extract derived from Enterobacteriaceae at the situs of infection;
(ii) screening and collecting those enterobacteria which produce colicin but which do not convert tryptophan into indole and which do not react in the MUG assay;
(iii) harvesting those selected bacteria;
(iv) selecting those strains of step (iii) which cannot make endotoxins.

22. A substantially pure strain of the microorganism of Claim 21.

23. An isolated host cell containing therein the biological material of Claim 1 or a pharmaceutical composition containing a non-toxic biological material obtained by the method according to Claim 2.

24. A vaccine comprising the biological material of Claim 1 or a pharmaceutical composition containing a non-toxic biological material obtained by the method according to Claim 2.

25. A pharmaceutically effective amount of the biological material of Claim 1 or a non-toxic biological material obtained by the method according to Claim 2 for use in immunotherapy of bacterial and viral infections in humans in need of such treatment.

26. A mixture of a first compound according to Claim 1 of the formula (1) given below and a second compound according to Claim 1 of the formula (1) given below: 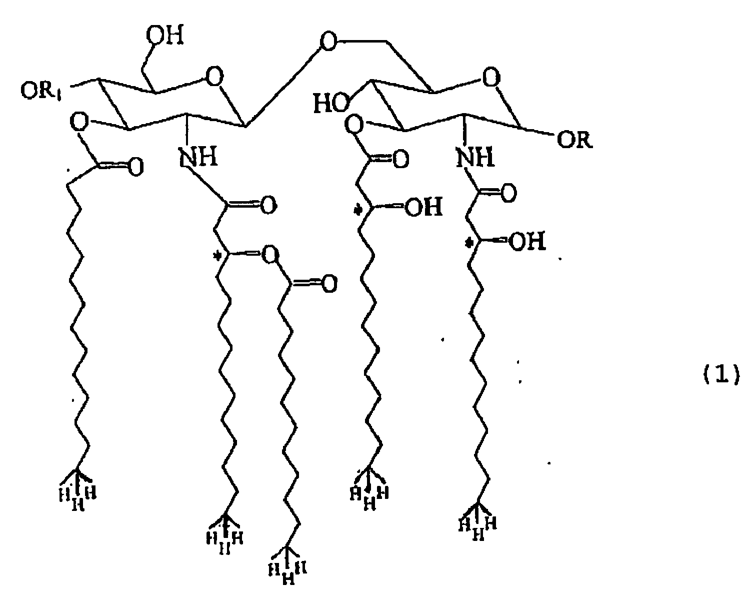 or their pharmaceutically acceptable salts thereof wherein in the first compound. R and R₁ are both H and in the second compound, one of R and R₁ is H and the other is 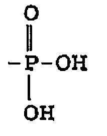 or the pharmaceutically acceptable salts thereof, wherein the weight ratio of the first compound to the second compound ranges from about 60:40 to about 90:10.

27. The mixture of Claim 26 wherein the weight ratio is about 80:20.

28. The mixture of Claim 26 wherein chiral centers therein identified with an * are in the R configuration.

29. The mixture of Claim 26 wherein a third compound according to Claim 1 is present having the formula: 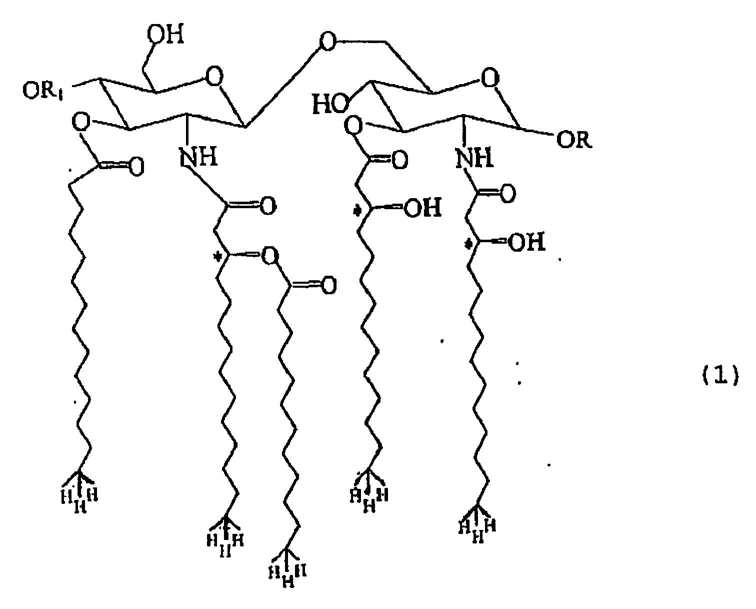 wherein R and R₁ are both 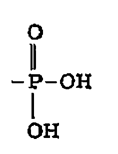 or the pharmaceutically acceptable salts thereof.

30. A mixture of a first compound according to Claim 1 of the formula (2) given below and a second compound according to Claim 1 of the formula (2) given below: 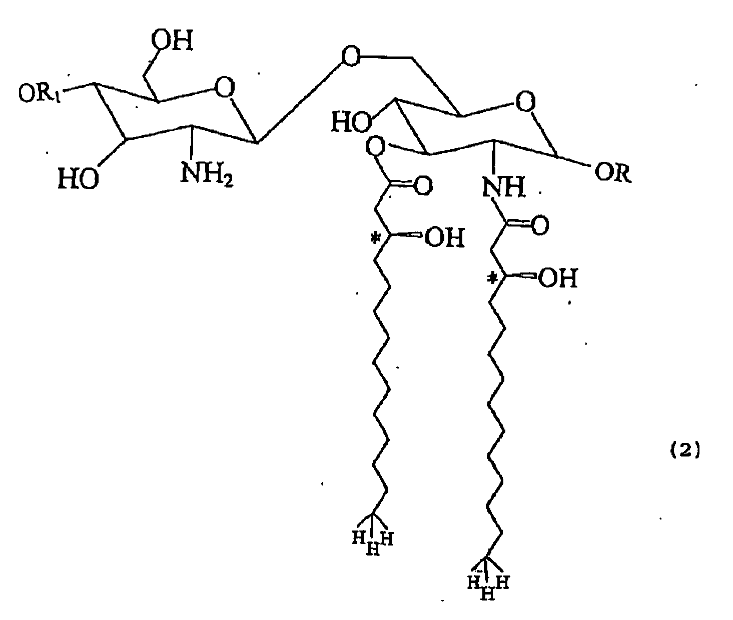 or their pharmaceutically acceptable salts thereof wherein in the first compound, R and R₁ are both H
and in the second compound, one of R and R₁ is H and the other is 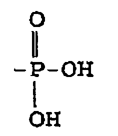 or the pharmaceutically acceptable salts thereof, wherein the weight ratio of the first compound to the second compound ranges from about 60:40 to about 90:10.

31. The mixture of Claim 30 wherein the weight ratio is about 80:20.

32. The mixture of Claim 30 wherein chiral centers therein identified with an * are in the R configuration.

33. The mixture of Claim 30 wherein a third compound according to Claim 1 is present having the formula: 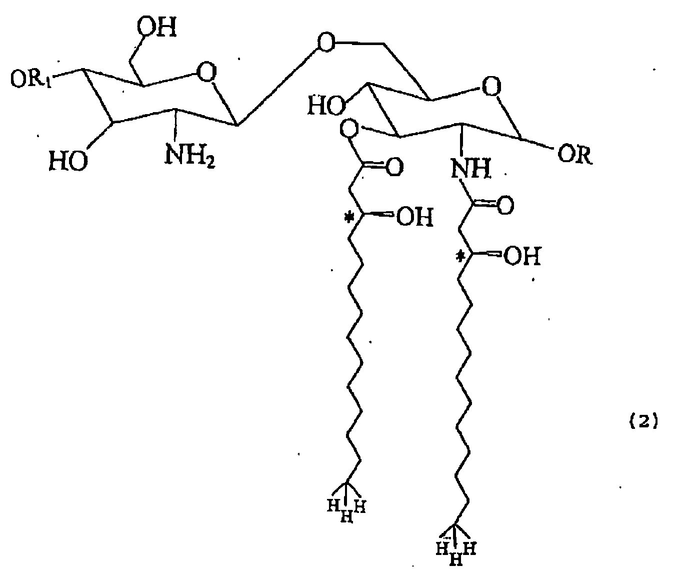 wherein R and R₁ are both 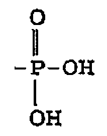 or the pharmaceutically acceptable salts thereof.

34. A mixture of a first compound according to Claim 1 of the formula (3) given below and a second compound according to Claim 1 of the formula (3) given below: 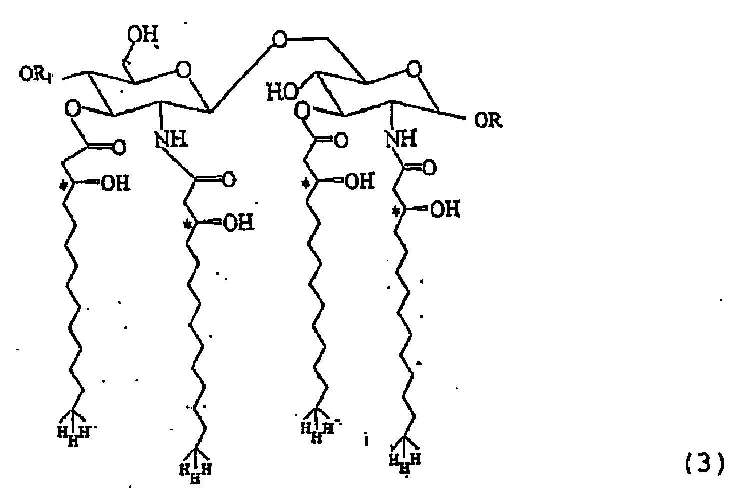 or their pharmaceutically acceptable salts thereof wherein in the first compound, R and R₁ are both H
and in the second compound, one of R and R₁ is H and the other is 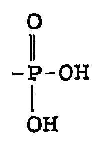 or the pharmaceutically acceptable salts thereof, wherein the weight ratio of the first compound to the second compound ranges from about 60:40 to about 90:10.

35. The mixture of Claim 34 wherein the weight ratio is about 80:20.

36. The mixture of Claim 34 wherein chiral centers therein identified with an * are in the R configuration.

37. The mixture of Claim 34 wherein a third compound according to Claim 1 is present having the formula: 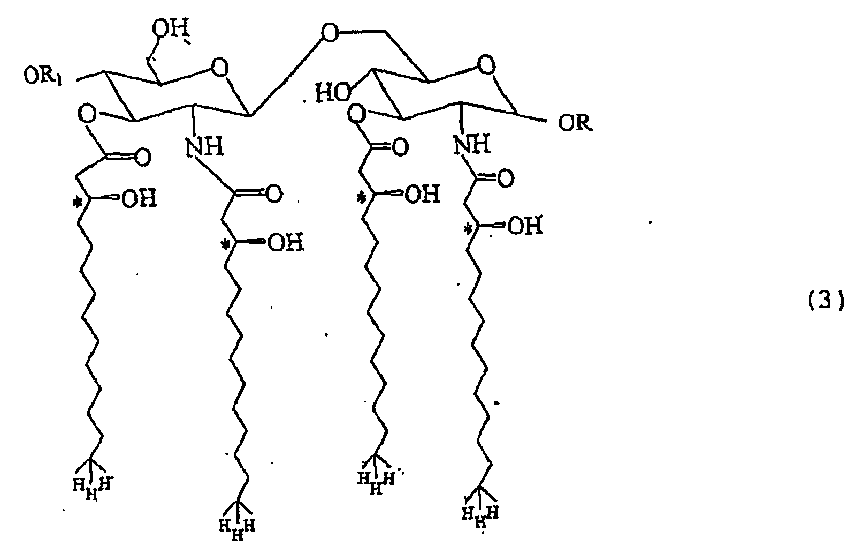 wherein R and R₁ are both 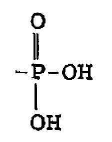 or the pharmaceutically acceptable salts thereof.

38. A mixture of a first compound according to Claim 1 of the formula (4) given below and a second compound according to claim 1 of the formula (4) given below: 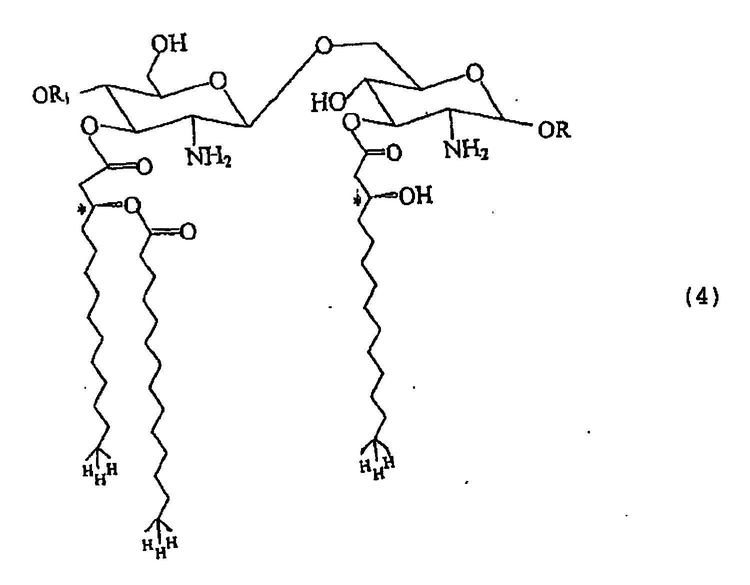 or their pharmaceutically acceptable salts thereof wherein in the first compound, R and R₁ are both H
and in the second compound, one of R and R₁ is H and the other is 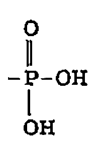 or the pharmaceutically acceptable salts thereof, wherein the weight ratio of the first compound to the second compound ranges from about 60:40 to about 90:10.

39. The mixture of Claim 38 wherein the weight ratio is about 80:20.

40. The mixture of Claim 38 wherein chiral centers therein identified with an * are in the R configuration.

41. The mixture of Claim 38 wherein a third compound according to Claim 1 is present having the formula: 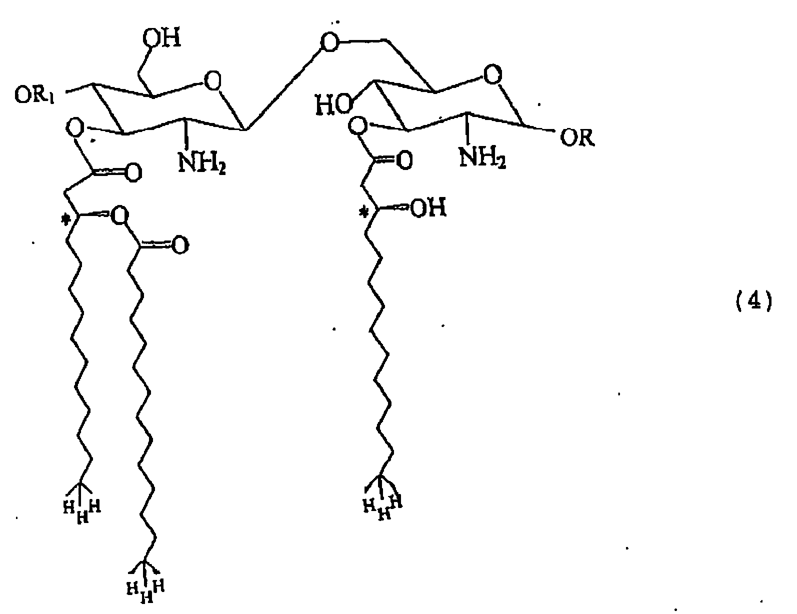 wherein R and R₁ are both 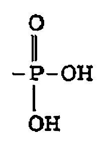 or the pharmaceutically acceptable salts thereof.

## Patentansprüche

1. Ein isoliertes, im Wesentlichen reines biologisches Material, das gegebenenfalls mit einem pharmazeutisch akzeptierbarem Salz davon assoziiert ist, welches folgende Eigenschaften hat:
(a) hat einen konstanten hydrodynamischen Radius von ungefähr 0.3 bis 0.40 µm, und hat einen niedrigen Polydispersitätsindex von ungefähr 0.05 bis ungefähr 0.08%;
(b) hat ein Aggregat von Monomereinheiten in Salzlösung, beinhaltend von ungefähr 68 bis 75 Monomere im Aggregat;
(c) das Aggregat hat ein zahlengemitteltes Molekulargewicht von ungefähr 130,000 bis 150,000 Dalton;
(d) das Monomer hat ein Molekulargewicht von ungefähr 1,9000 bis 2,000 Dalton;
(e) umfasst zwei Aminozuckerreste, wobei der Zucker Glukose oder Galactose ist, sofern einer der Zucker Glukose ist;
(f) umfasst keine Pyrophosphatgruppen;
(g) umfasst eine 1,6 β-Verknüpfung zwischen den beiden Zuckern;
(h) das Monomer umfasst keine Phosphatgruppe oder kann ein Phosphat an der 1-Position oder der 4'-Position des Zuckers enthalten; jedoch umfasst das Aggregat zumindest 80 Gew.-% eines Zuckerrests, der keine Phosphate daran hat, und höchstens 20 Gew.-% eines Zuckerrests, der ein Monophosphat hat;
(i) umfasst eine Aminofunktionalität an den 2- und 2'-Positionen, welche Amidbindungen mit 3-Hydroxytetradecansäure bilden kann;
(j) umfasst eine Hydroxyfunktionalität an den 3- und 3'-Positionen, welche mit 3-Hydroxytetradecansäure verestert sein kann;
(k) umfasst eine gerade Anzahl an 3'-Hydroxytetradecansäuren pro Monomer; und
(l) hat das Röntgenbeugungsmuster der Fig. 8A bei 25°C.

2. Verfahren zur Herstellung eines nicht-toxischen biologischen Materials umfassend:
(a) Bereitstellen eines Endotoxinextrakts, der von Enterobakterien an der Stelle einer Infektion abgeleitet ist;
(b) Vorauswahl und Sammeln von den Enterobakterien, welche Colicin herstellen, aber nicht Tryptophan zu Indol umwandeln, und welche nicht im MUG-Assay reagieren;
(c) Ernten der ausgewählten Bakterien;
(d) Auswahl der Stämme von Schritt (c), die keine Endotoxine machen können;
(e) Abtöten der Stämme von Schritt (d);

3. Verfahren gemäß Anspruch 2, bei dem Schritt (e) ein Erhitzen der Stämme von Schritt (d) bei geeigneten Temperaturen zur Denaturierung der Proteine darin umfasst.

4. Verfahren gemäß Anspruch 2, bei dem ein Isolieren aus dem Produkt von Schritt (e) eine Extraktion des Lipidmaterials, das Absorptionen bei 230 nm und 550 nm aufweist, umfasst.

5. Verfahren gemäß Anspruch 4, bei dem die Extraktion ein Ausfällen des Lipidmaterials mit Chloroform/Methanol- oder Acetonitril/Methanol-Lösung und Reinigung des Produkts und Gefriertrocknung desselben umfasst.

6. Verfahren gemäß Anspruch 4, bei dem die Extraktion ein Platzieren des Lipidmaterials in einer wässrigen Salzlösung, Reinigung des Rohlipidprodukts durch Chromatographie, Sammeln der Fraktionen, welche eine UV-Absorption bei ungefähr 230 und 550 nm haben, Gefriertrocknen der gesammelten Fraktionen und Ausfällen des erwünschten Produkts mit CHCl₃/MeOH oder CH₃CN/MeOH und Reinigung des ausgefällten Produkts umfasst.

7. Verfahren gemäß Anspruch 4, bei dem das nicht-toxische biologische Material ein Monomermolekulargewicht von ungefähr 1900 Dalton und ein Aggregat-Molekulargewicht in Salzlösung im Bereich von ungefähr 120,000 bis ungefähr 150,000 Dalton hat.

8. Verfahren gemäß Anspruch 2, umfassend ein Überführen des nicht-toxischen biologischen Materials in wässriger Lösung.

9. Verfahren gemäß Anspruch 2, wobei die Enterobakterien aerobe Enterobakterien sind.

10. Verfahren gemäß Anspruch 2, bei dem die Enterobakterien in der R-Form sind.

11. Verfahren gemäß Anspruch 2, bei dem die Enterobakterien in der S-Form sind.

12. Verfahren gemäß Anspruch 11, bei dem die Enterobakterien zu einem Stamm ausgewählt aus der Gruppe bestehend aus Bacillus, Bacteroides, Brucella, Carnobacterium, Caulobacter, Citrobacter, Clostridium, Corynebacterium, Enterobacter, Escherichia coli, Halobacteria, Klebsiella, Lactobacillus, Lactococcus, Leuconstoc, Listeria, Micrococcus, Mycobacterium, Neisseria, Pasteurella, Pediococcus, Propionibacterium, Proteus, Pseudomonas, Salmonella, Sarcina, Shigella, Serratia, Staphylococcus, Streptococcus, und Vibrio gehören.

13. Kolloidaler Kristall aus dem biologischen Material von Anspruch 1 oder aus einer pharmazeutischen Zusammensetzung, der durch das Verfahren gemäß Anspruch 2 erhalten wurde.

14. Pharmazeutische Zusammensetzung, umfassend eine pharmazeutisch wirksame Menge des biologischen Materials von Anspruch 1 oder eine pharmazeutische Zusammensetzung enthaltend ein nicht-toxisches biologisches Material, erhalten durch das Verfahren gemäß Anspruch 2, in Verbindung mit einem pharmazeutischen Träger.

15. Pharmazeutische Zusammensetzung gemäß Anspruch 14, weiterhin umfassend Calcium-, Magnesium- und Zinksalze.

16. Pharmazeutische Zusammensetzung gemäß Anspruch 15, bei der die Salze ZnCL₂, Zn-D-gluconat, Zn-Meglumin, Zn-D-citrat oder Zn-salicylat sind.

17. Pharmazeutische Zusammensetzung gemäß Anspruch 14 in Form eines Liposoms.

18. Pharmazeutische Zusammensetzung gemäß Anspruch 14, wobei die Zusammensetzung in einer gefriergetrockneten Form ist.

19. Pharmazeutische Zusammensetzung gemäß Anspruch 14 in Form einer Öltropfenemulsion.

20. Biologisches Material gemäß Anspruch 1 oder biologisches Material, erhalten durch das Verfahren gemäß Anspruch 2, in einer pharmazeutisch wirksamen Menge für die Verwendung in einem Verfahren zur Behandlung einer Krankheit in einem Säugetier, welches von einer Virus- oder Bakterieninfektion befallen ist.

21. Isolierter Mikroorganismus mit den folgenden Eigenschaften:
(a) ist ein Enterobakterium;
(b) produziert Colicin;
(c) bildet kein Endotoxin;
(d) besitzt keine Gene, die für die Bildung von Verotoxinen verantwortlich sind;
(e) beinhaltet keine Aktivierungsfaktoren für CAMP Adenylatcyclase;
(f) beinhaltet keine Aktivierungsfaktoren für cGMPM Guanylatcyclase;
(g) hat keine Adhäsionsmoleküle;
(h) hat nicht die eae Gensequenz;
(i) setzt nicht Tryptophan zu Indol um und reagiert nicht im MUG-Assay;
(j) ist stäbchenförmig im Aussehen; und
(k) erhalten durch das Verfahren
(i) Bereitstellen eines Endotoxinextrakts, der von Enterobakterien an der Stelle einer Infektion gewonnen wurde;
(ii) Vorauswahl und Sammeln von den Enterobakterien, welche Colicin herstellen, aber nicht Tryptophan zu Indol umwandeln, und welche nicht im MUG-Assay reagieren;
(iii) Entnahme der ausgewählten Bakterien;
(iv) Auswahl der Stämme von Schritt (iii), die keine Endotoxine machen können.

22. Im Wesentlichen reiner Stamm des Mikroorganismus gemäß Anspruch 21.

23. Isolierte Wirtszelle, beinhaltend darin das biologische Material von Anspruch 1 oder eine pharmazeutische Zusammensetzung, enthaltend ein nicht-toxisches biologisches Material, erhalten durch das Verfahren gemäß Anspruch 2.

24. Impfstoff umfassend das biologische Material von Anspruch 1 oder eine pharmazeutische Zusammensetzung, enthaltend ein nicht-toxisches biologisches Material, erhalten durch das Verfahren gemäß Anspruch 2.

25. Pharmazeutisch wirksame Menge des biologischen Materials gemäß Anspruch 1 oder eines nicht-toxischen biologischen Materials, erhalten durch das Verfahren gemäß Anspruch 2, zur Verwendung bei der Immuntherapie von Bakterien- und Vireninfektionen bei Menschen, die eine solche Behandlung benötigen.

26. Mischung aus einer ersten Verbindung gemäß Anspruch 1 der untenstehenden Formel (1) und einer zweiten Verbindung gemäß Anspruch 1 der untenstehenden Formel (1): 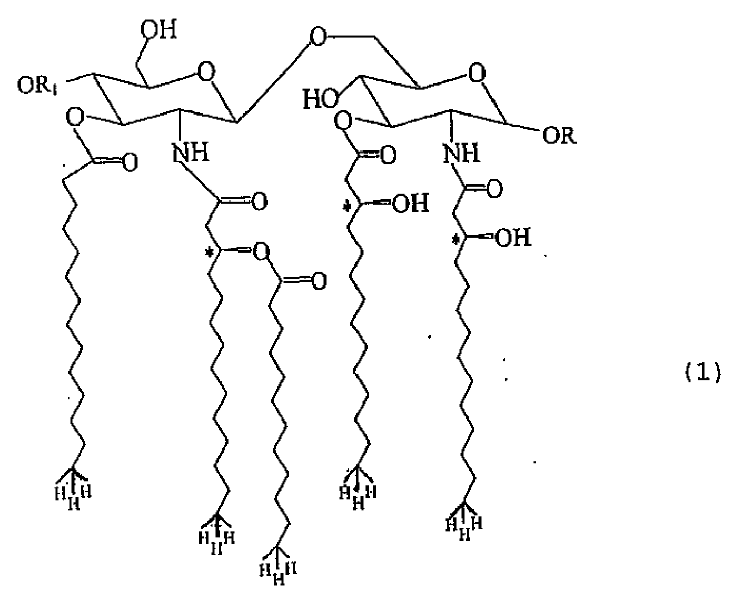 oder ihrer pharmazeutisch verträglichen Salze, wobei in der ersten Verbindung R und R₁ beide H sind,
und in der zweiten Verbindung einer von R und R₁ H und der andere 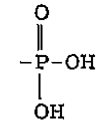 ist
oder ihrer pharmazeutisch verträglichen Salze, wobei das Gewichtsverhältnis der ersten Verbindung zur zweiten Verbindung im Bereich von ungefähr 60:40 bis 90:10 ist.

27. Mischung gemäß Anspruch 26, wobei das Gewichtsverhältnis ungefähr 80:20 ist.

28. Mischung gemäß Anspruch 26, wobei die chiralen Zentren, die darin mit einem * identifiziert sind, in der R-Konfiguration sind.

29. Mischung gemäß Anspruch 26, wobei eine dritte Verbindung gemäß Anspruch 1 vorhanden ist mit der Formel: 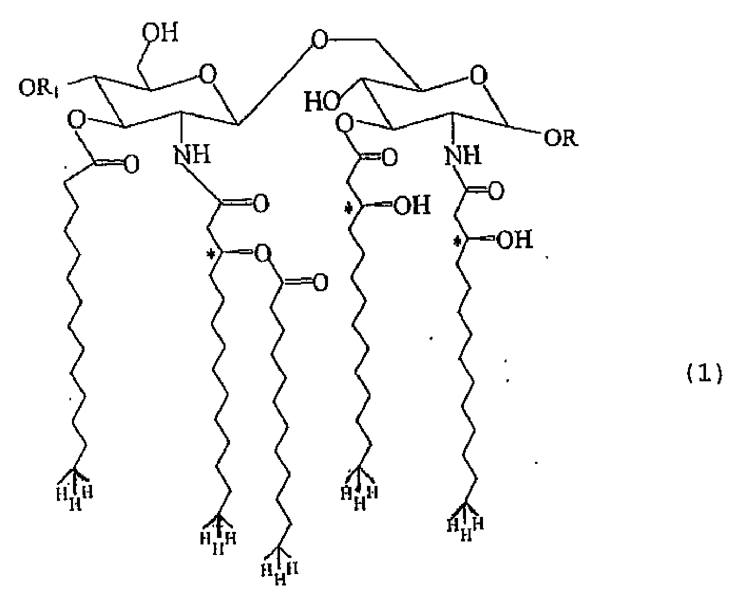 wobei R und R₁ beide 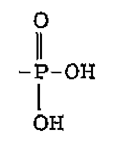 sind
oder ihrer pharmazeutisch verträglichen Salze.

30. Mischung aus einer ersten Verbindung gemäß Anspruch 1 der untenstehenden Formel (2) und einer zweiten Verbindung gemäß Anspruch 1 der untenstehenden Formel (2): 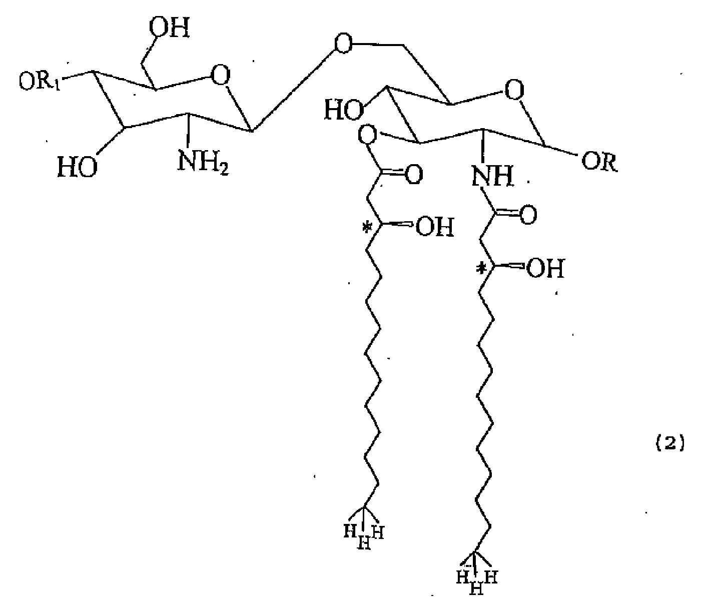 oder ihrer pharmazeutisch verträglichen Salze, wobei in der ersten Verbindung R und R₁ beide H sind,
und in der zweiten Verbindung einer von R und R₁ H und der andere 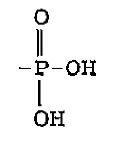 ist
oder ihrer pharmazeutisch verträglichen Salze, wobei das Gewichtsverhältnis der ersten Verbindung zur zweiten Verbindung im Bereich von ungefähr 60:40 bis 90:10 ist.

31. Mischung gemäß Anspruch 30, wobei das Gewichtsverhältnis ungefähr 80:20 ist.

32. Mischung gemäß Anspruch 30, wobei die chiralen Zentren, die darin mit einem * identifiziert sind, in der R-Konfiguration sind.

33. Mischung gemäß Anspruch 30, wobei eine dritte Verbindung gemäß Anspruch 1 vorhanden ist mit der Formel: 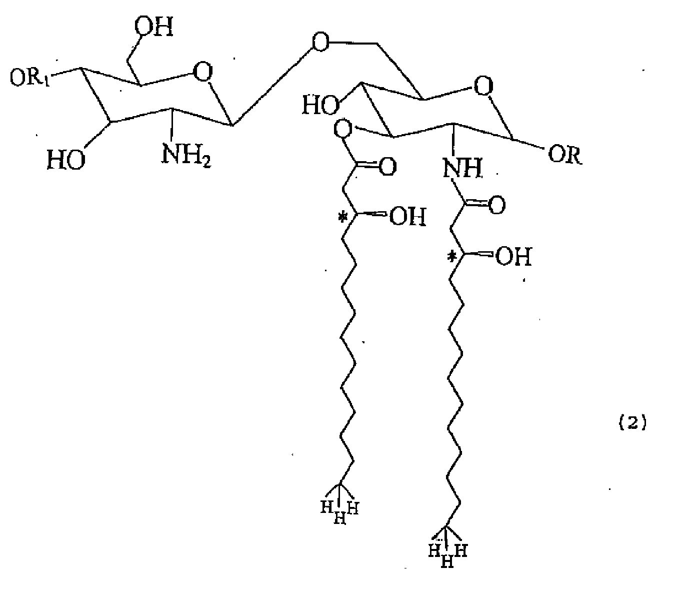 wobei R und R₁ beide 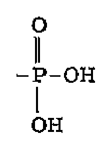 sind
oder ihrer pharmazeutisch verträglichen Salze.

34. Mischung aus einer ersten Verbindung gemäß Anspruch 1 der untenstehenden Formel (3) und einer zweiten Verbindung gemäß Anspruch 1 der untenstehenden Formel (3): 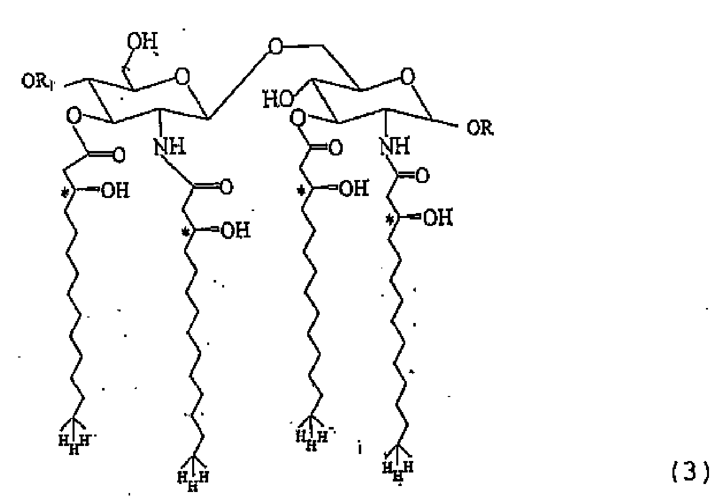 oder ihrer pharmazeutisch verträglichen Salze, wobei in der ersten Verbindung R und R₁ beide H sind,
und in der zweiten Verbindung einer von R und R₁ H und der andere 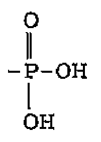 ist
oder ihrer pharmazeutisch verträglichen Salze, wobei das Gewichtsverhältnis der ersten Verbindung zur zweiten Verbindung im Bereich von ungefähr 60:40 bis 90:10 ist.

35. Mischung gemäß Anspruch 34, wobei das Gewichtsverhältnis ungefähr 80:20 ist.

36. Mischung gemäß Anspruch 34, wobei die chiralen Zentren, die darin mit einem * identifiziert sind, in der R-Konfiguration sind.

37. Mischung gemäß Anspruch 34, wobei eine dritte Verbindung gemäß Anspruch 1 vorhanden ist mit der Formel: 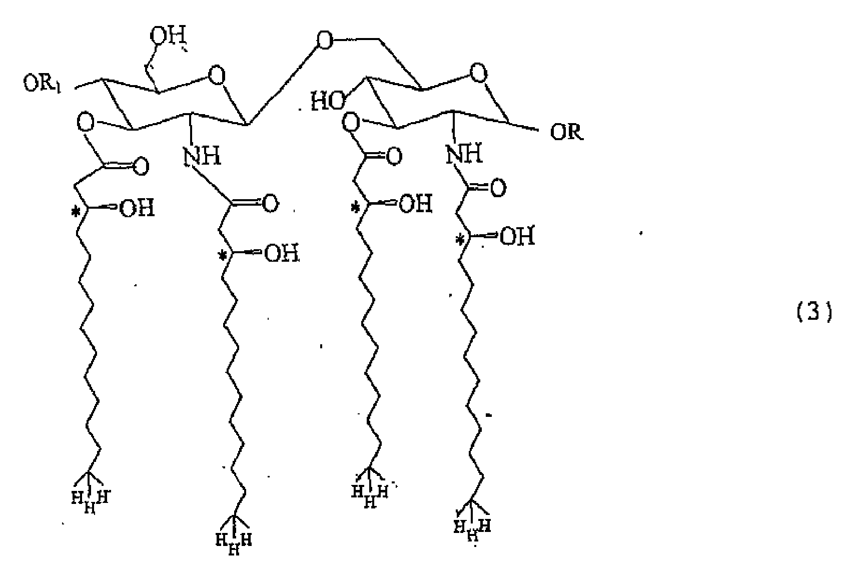 wobei R und R₁ beide 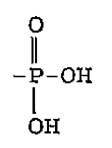 sind
oder ihrer pharmazeutisch verträglichen Salze.

38. Mischung aus einer ersten Verbindung gemäß Anspruch 1 der untenstehenden Formel (4) und einer zweiten Verbindung gemäß Anspruch 1 der untenstehenden Formel (4): 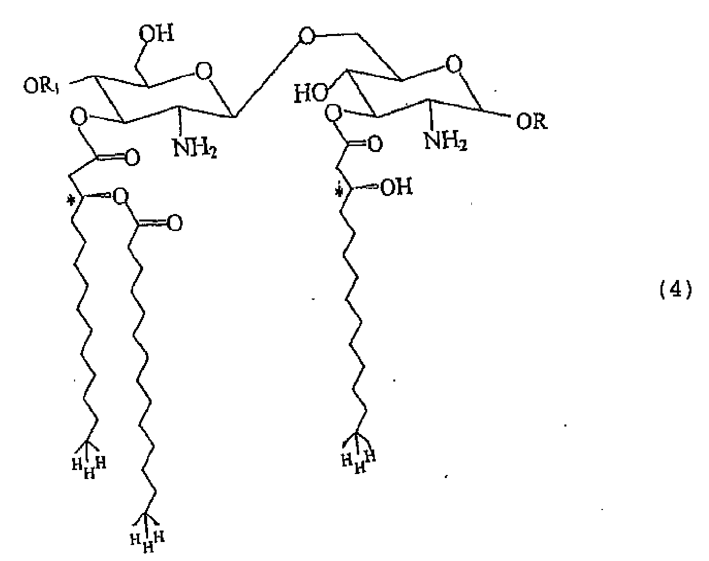 oder ihrer pharmazeutisch verträglichen Salze, wobei in der ersten Verbindung R und R₁ beide H sind,
und in der zweiten Verbindung einer von R und R₁ H und der andere 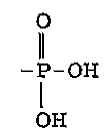 ist
oder ihrer pharmazeutisch verträglichen Salze, wobei das Gewichtsverhältnis der ersten Verbindung zur zweiten Verbindung im Bereich von ungefähr 60:40 bis 90:10 ist.

39. Mischung gemäß Anspruch 38, wobei das Gewichtsverhältnis ungefähr 80:20 ist.

40. Mischung gemäß Anspruch 38, wobei die chiralen Zentren, die darin mit einem * identifiziert sind, in der R-Konfiguration sind.

41. Mischung gemäß Anspruch 38, wobei eine dritte Verbindung gemäß Anspruch 1 vorhanden ist mit der Formel: 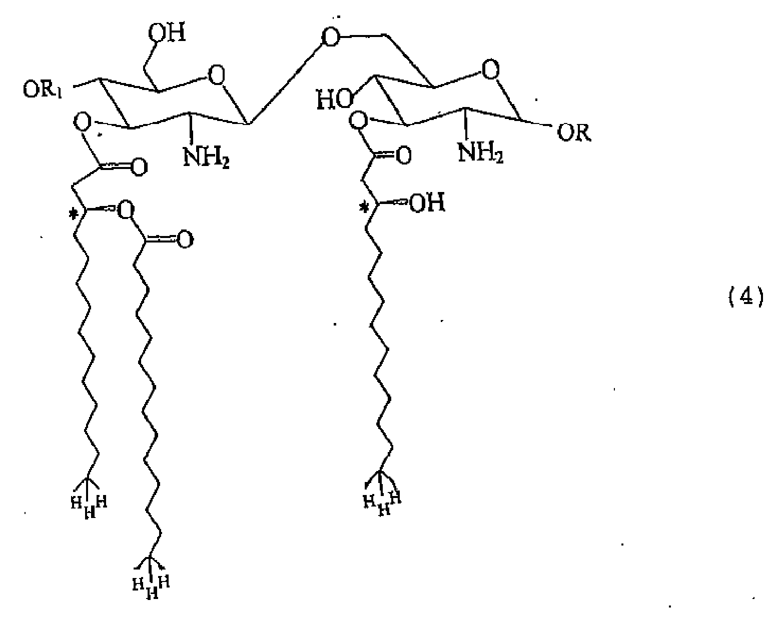 wobei R und R₁ beide 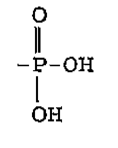 sind
oder ihrer pharmazeutisch verträglichen Salze.

## Revendications

1. Matière biologique isolée sensiblement pure associée à un sel pharmaceutiquement acceptable de ladite matière ayant les caractéristiques suivantes :
(a) a un rayon hydrodynamique constant d'environ 0,3 à 0,40 µm, ayant un faible indice de polydispersité d'environ 0,05 à environ 0,08 % ;
(b) a un agrégat d'unités monomériques en solution saline, contenant environ 68 à 75 monomères dans l'agrégat ;
(c) l'agrégat a une masse moléculaire en nombre d'environ 130.000 à 150.000 daltons ;
(d) le monomère a une masse moléculaire d'environ 1.900 à 2.000 daltons ;
(e) contient deux fragments de sucre aminé, dans lesquels le sucre est du glucose ou du galactose, à condition qu'un des sucres soit du glucose ;
(f) ne contient pas de groupes pyrophosphates ;
(g) contient une liaison β-1,6 entre les deux sucres ;
(h) le monomère ne contient pas de groupe phosphate ou peut contenir un phosphate à la position 1 ou à la position 4' du sucre ; cependant, l'agrégat contient au moins à 80 % en poids un fragment de sucre qui ne possède pas de phosphate, et au plus à 20 % en poids un fragment de sucre ayant un monophosphate ;
(i) contient une fonction amine aux positions 2 et 2' qui peut former des liaisons amides avec un acide 3-hydroxytétradécanoïque ;
(j) contient une fonction hydroxy aux positions 3 et 3' qui peut être estérifiée avec un acide 3-hydroxytétradécanoïque ;
(k) contient un nombre pair d'acides 3'-hydroxytétradécanoïques par monomère ; et
(l) a le diagramme de diffraction des rayons X selon la Fig. 8A à 25°C.

2. Procédé de préparation d'une matière biologique non toxique comprenant :
(a) la fourniture d'un extrait d'endotoxines dérivé d'entérobactéries sur le lieu de l'infection ;
(b) le dépistage et le recueil de ces entérobactéries qui produisent de la colicine mais qui ne transforment pas le tryptophane en indole et qui ne réagissent pas dans l'analyse MUG ;
(c) la récolte de ces bactéries sélectionnées ;
(d) la sélection de ces souches de l'étape (c) qui ne peuvent pas faire d'endotoxines ;
(e) la destruction des souches de l'étape (d).

3. Procédé selon la revendication 2, dans lequel l'étape (e) comprend le réchauffement des souches de (d) à des températures suffisantes pour dénaturer la protéine qu'elles contiennent.

4. Procédé selon la revendication 2, dans lequel l'isolation du produit de l'étape (e) comprend l'extraction de la matière lipide présentant des absorptions à 230 nm et 550 nm.

5. Procédé selon la revendication 4, dans lequel l'extraction comprend la précipitation de la matière lipide avec une solution de chloroforme/méthanol ou d'acétonitrile/méthanol et la purification ainsi que la lyophilisation.

6. Procédé selon la revendication 4, dans lequel l'extraction comprend le placement de la matière lipide dans une solution aqueuse saline, la purification du produit lipide brut par chromatographie, la collecte de ces fractions ayant une absorbtion d'UV à environ 230 et 550 nm, la lyophilisation des fractions recueillies et la précipitation du produit souhaité avec CHCl₃/MeOH ou CH₃CN/MeOH et la purification du produit précipité.

7. Procédé selon la revendication 4, dans lequel la matière biologique non toxique a une masse moléculaire du monomère d'environ 1900 daltons et une masse moléculaire d'agrégat en solution saline située entre environ 120.000 et 150.000 daltons.

8. Procédé selon la revendication 2, comprenant le placement de matière biologique non toxique dans une solution aqueuse.

9. Procédé selon la revendication 2, dans lequel les entérobactéries sont des entérobactéries aérobies.

10. Procédé selon la revendication 2, dans lequel les entérobactéries se présentent sous la forme R.

11. Procédé selon la revendication 2, dans lequel les entérobactéries se présentent sous la forme S.

12. Procédé selon la revendication 11, dans lequel les entérobactéries font partie de la souche sélectionnée parmi le groupe constitué de Bacillus, Bacteroides, Brucella, Carnobacterium, Caulobacter, Citrobacter, Clostridium, Corynebacterium, Enterobacter, Escherichia coli, Halobacteria, Klebsiella, Lactobacillus, Lactoeoecus, Leuconstoc, Listeria, Micrococcus, Mycobacterium, Neisseria, Pasteurella, Pediococcus, Propionibacterium, Proteus, Pseudomonas, Salmonella, Sarcina, Shigella, Serratia, Staphylococcus, Sreptococcus, et Vibrio.

13. Cristal colloïdal de la matière biologique de la revendication 1 ou composition pharmaceutique obtenue par le procédé selon la revendication 2.

14. Composition pharmaceutique comprenant une quantité pharmaceutiquement efficace de la matière biologique de la revendication 1 ou composition pharmaceutique contenant un matériel biologique non toxique obtenue par le procédé selon la revendication 2 en association avec un excipient pharmaceutique.

15. Composition pharmaceutique selon la revendication 14, comprenant en outre du calcium, du magnésium et des sels de zinc.

16. Composition pharmaceutique selon la revendication 15, dans laquelle les sels sont du ZnCl2, du D-gluconate de zinc, du méglumine de zinc, du D-citrate de zinc ou du salicylate de zinc.

17. Composition pharmaceutique selon la revendication 14, sous la forme d'un liposome.

18. Composition pharmaceutique de la revendication 14, dans laquelle la composition est sous la forme lyophilisée.

19. Composition pharmaceutique selon la revendication 14, sous la forme d'une émulsion de gouttes d'huile.

20. Matière biologique selon la revendication 1 ou matière biologique obtenue par le procédé selon la revendication 2 en quantité pharmaceutique efficace pour une utilisation dans un procédé de traitement d'une maladie d'un mammifère atteint d'une infection virale ou bactérienne.

21. Micro-organisme isolé ayant les attributs suivants :
(a) est une entérobactérie ;
(b) produit de la colicine ;
(c) ne forme pas d'endotoxines ;
(d) ne possède pas de gènes responsables de la production de vérotoxines ;
(e) ne contient pas de facteurs d'activation pour l'adénylate cyclase AMPc ;
(f) ne contient pas de facteurs d'activation pour le guanylate cyclase GMPMc ;
(g) n'a pas de molécules d'adhésion ;
(h) n'a pas de séquence de gène eae ;
(i) qui ne transforme pas le tryptophane en indole et qui ne réagit pas sans l'analyse MUG ;
(j) a une apparence de bâtonnet ; et
(k) obtenu par le procédé de
(i) fourniture d'un extrait d'endotoxines dérivé d'entérobactéries sur le lieu de l'infection ;
(ii) dépistage et recueil de ces entérobactéries qui produisent de la colicine mais qui ne transforment pas le tryptophane en indole et qui ne réagissent pas dans l'analyse MUG ;
(iii) récolte de ces bactéries sélectionnées ;
(iv) sélection de ces souches de l'étape (iii) qui ne peuvent pas faire d'endotoxines.

22. Souche sensiblement pure du micro-organisme de la revendication 21.

23. Cellule hôte isolée contenant la matière biologique de la revendication 1 ou composition pharmaceutique contenant un matériel biologique non toxique obtenue par le procédé selon la revendication 2.

24. Vaccin comprenant la matière biologique de la revendication 1 ou composition pharmaceutique contenant un matériel biologique non toxique obtenue par le procédé selon la revendication 2.

25. Quantité pharmaceutiquement efficace de la matière biologique de la revendication 1 ou matière biologique non toxique obtenue par le procédé selon la revendication 2 destinée à une utilisation en immunothérapie d'infections bactériennes et virales sur des humains nécessitant un tel traitement.

26. Mélange d'un premier composé selon la revendication 1 de la formule (1) énoncée ci-dessous et d'un second composé selon la revendication 1 de la formule (1) énoncée ci-dessous : 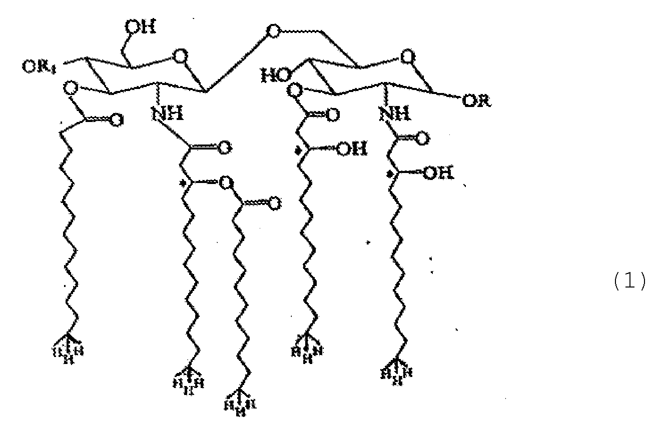 ou leurs sels pharmaceutiquement acceptables, dans lequel dans le premier composé, R et R₁ sont tous les deux H et dans le second composé, l'un de R et R₁ est H et
l'autre est
ou les sels pharmac 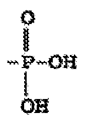 t acceptables dudit mélange, dans lequel le rapport massique entre le premier composé et le second composé est situé entre environ 60:40 et 90:10.

27. Mélange selon la revendication 26, dans lequel le rapport massique est environ 80:20.

28. Mélange selon la revendication 26, dans lequel les centres chiraux y étant identifiés avec un * sont dans la configuration R.

29. Mélange selon la revendication 26, dans lequel un troisième composé selon la revendication 1 est présent, dont la formule est : 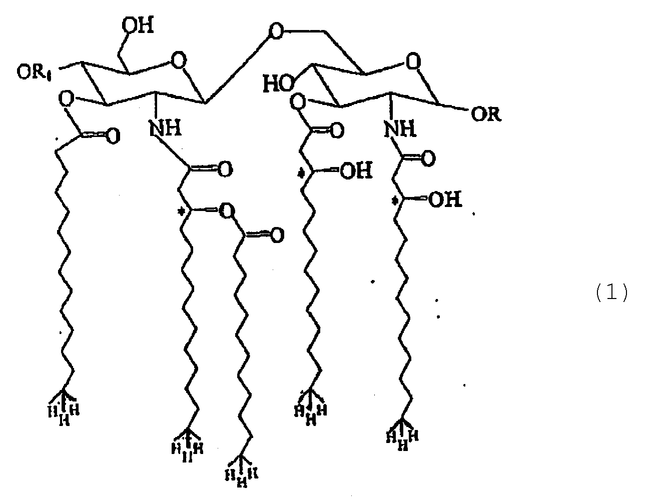 dans lequel R et R₁ sont tous les deux 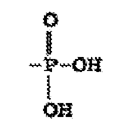 ou les sels pharmaceutiquement acceptables dudit mélange.

30. Mélange d'un premier composé selon la revendication 1 de la formule (2) énoncée ci-dessous et d'un second composé selon la revendication 1 de la formule (2) énoncée ci-dessous : 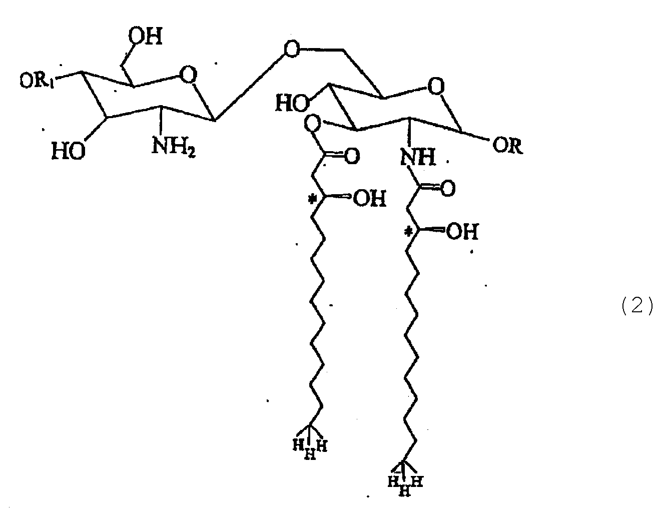 ou leurs sels pharmaceutiquement acceptables, dans lequel dans le premier composé R et R₁ sont tous les deux H et dans le second composé, l'un de R et R₁ est H et l'autre
est
ou les 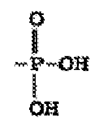 aceutiquement acceptables dudit mélange, dans lequel le rapport massique entre le premier composé et le second composé est situé entre environ 60:40 et 90:10.

31. Mélange selon la revendication 30, dans lequel le rapport massique est 80:20.

32. Mélange selon la revendication 30, dans lequel les centres chiraux y étant identifiés avec un * sont dans la configuration R.

33. Mélange selon la revendication 30, dans lequel un troisième composé selon la revendication 1 est présent, dont la formule est : 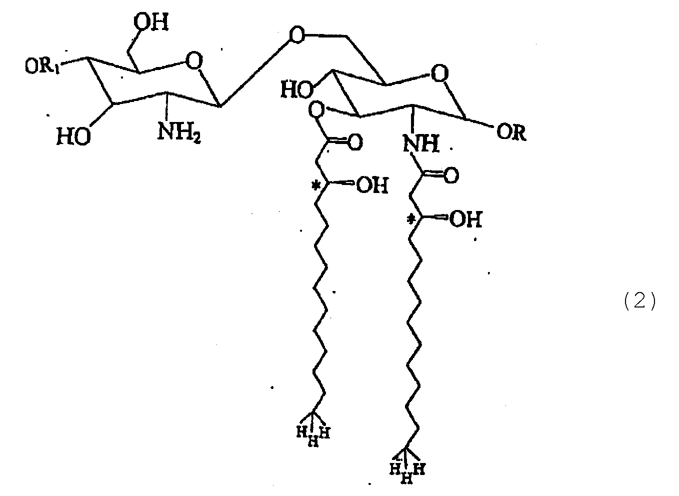 dans lequel R et R₁ sont tous les deux 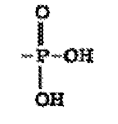 ou les sels pharmaceutiquement acceptables dudit mélange.

34. Mélange d'un premier composé selon la revendication 1 de la formule (3) énoncée ci-dessous et d'un second composé selon la revendication 1 de la formule (3) énoncée ci-dessous : 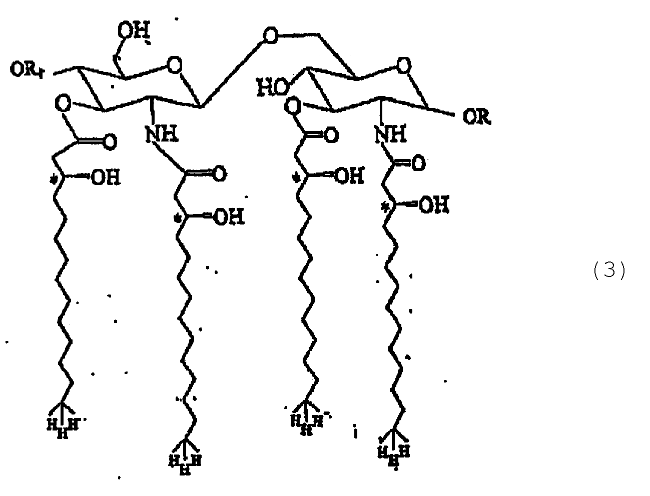 ou leurs sels pharmaceutiquement acceptables, dans lequel dans le premier composé, R et R₁ sont tous les deux H
et dans le second composé, l'un de R et R₁, est H et
l'autre est 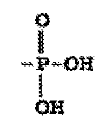 ou les sels pharmaceutiquement acceptables dudit mélange, dans lequel le rapport massique entre le premier composé et le second composé est situé entre environ 60:40 et 90:10.

35. Mélange selon la revendication 34, dans lequel le rapport massique est environ 80:20.

36. Mélange selon la revendication 34, dans lequel les centres chiraux y étant identifiés avec un * sont dans la configuration R.

37. Mélange selon la revendication 34, dans lequel un troisième composé selon la revendication 1 est présent, dont la formule est : 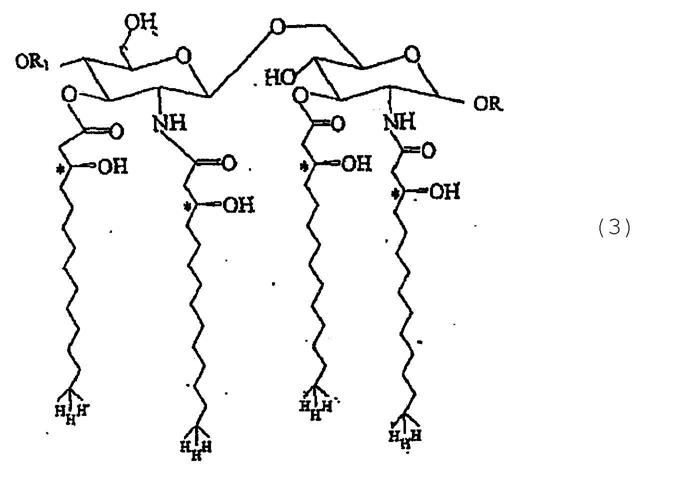 dans lequel R et R₁ sont tous les deux 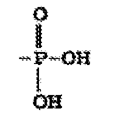 ou les sels pharmaceutiquement acceptables dudit mélange.

38. Mélange d'un premier composé selon la revendication 1 de la formule (4) énoncée ci-dessous et d'un second composé selon la revendication 1 de la formule (4) énoncée ci-dessous : 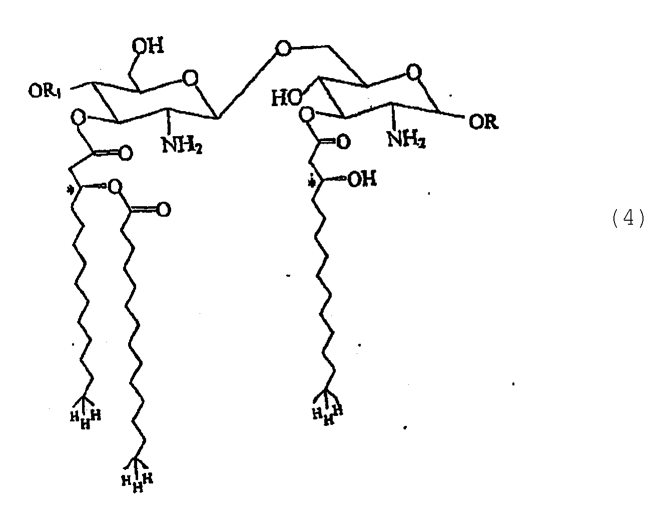 ou leurs sels pharmaceutiquement acceptables, dans lequel dans le premier composé, R et R₁ sont tous les deux H
et dans le second composé, l'un de R et R₁ est H et
l'autre est 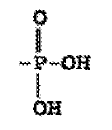 ou les sels pharmaceutiquement acceptables dudit mélange, dans lequel le rapport massique entre le premier composé et le second composé est situé entre environ 60:40 et 90:10.

39. Mélange selon la revendication 38, dans lequel le rapport massique est environ 80:20.

40. Mélange selon la revendication 38, dans lequel les centres chiraux y étant identifiés avec un * sont dans la configuration R.

41. Mélange selon la revendication 38, dans lequel un troisième composé selon la revendication 1 est présent, dont la formule est : 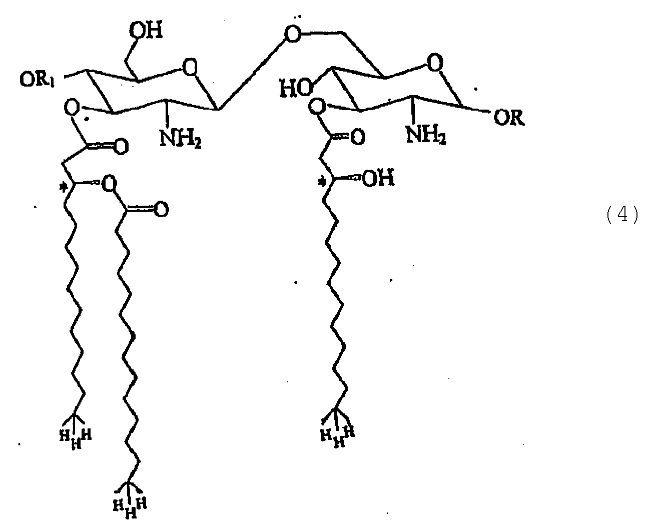 dans lequel R et R₁ sont tous les deux 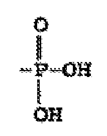 ou les sels pharmaceutiquement acceptables dudit mélange.
